# EUROPÄISCHE PATENTSCHRIFT

(11) **EP 3 131 901 B1**
(45) Veröffentlichungstag und Bekanntmachung des Hinweises auf die Patenterteilung: **07.02.2018**
(21) Anmeldenummer: 15711049.5
(22) Anmeldetag: 19.03.2015
(51) Int. Cl.: C07D 471/10, C09K 11/06, H01L 51/50

(54) **MATERIALIEN FÜR ELEKTRONISCHE VORRICHTUNGEN**
MATERIALS FOR ELECTRONIC DEVICES
MATIÈRES POUR DISPOSITIFS ÉLECTRONIQUES

(30) Priorität: 14.04.2014 EP 14001344
(43) Veröffentlichungstag der Anmeldung: 22.02.2017
(73) Patentinhaber: Merck Patent GmbH, 64293 Darmstadt (DE)
(72) Erfinder: PFISTER, Jochen, 64665 Alsbach-Haehnlein (DE); VOGES, Frank, 67098 Bad Duerkheim (DE); MONTENEGRO, Elvira, 69469 Weinheim (DE); MUJICA-FERNAUD, Teresa, 64283 Darmstadt (DE)
(86) Internationale Anmeldenummer: PCT/EP2015/000603
(87) Internationale Veröffentlichungsnummer: WO 2015/158411

(56) Entgegenhaltungen:
- WO-A1-2012/150001
- JP-A- 2002 265 938
- GILMAN; ZUECH: "Synthesis of some 5,10-dihydrophenazasiline derivatives", J. ORG. CHEM., Bd. 26, Nr. 6, 1. Juni 1961 (1961-06-01), Seiten 2013-2017, XP009158163, ISSN: 0022-3263, DOI: 10.1021/JO01065A081

## Beschreibung

Die vorliegende Anmeldung betrifft eine Hetero-Spirobifluorenverbindung einer unten näher definierten Formel (I). Die Verbindung wird bevorzugt in einer elektronischen Vorrichtung verwendet, besonders bevorzugt in einer organischen Elektrolumineszenzvorrichtung (OLED).

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter OLEDs verstanden.

Der Aufbau von OLEDs, in denen organische Verbindungen als funktionelle Materialien eingesetzt werden, ist beispielsweise in US 4539507, US 5151629, EP 0676461 und WO 98/27136 beschrieben. Allgemein werden unter der Bezeichnung OLEDs elektronische Vorrichtungen verstanden, welche eine oder mehrere Schichten enthaltend organische Verbindungen aufweisen und unter Anlegen von elektrischer Spannung Licht emittieren.

Einen großen Einfluss auf die Leistungsdaten von elektronischen Vorrichtungen haben Schichten mit lochtransportierender Funktion (lochtransportierende Schichten), wie beispielsweise Lochinjektionsschichten, Lochtransportschichten, Elektronenblockierschichten und emittierende Schichten.

Es ist im Stand der Technik bekannt, Triarylamine als Materialien mit lochtransportierenden Eigenschaften in den oben genannten Schichten einzusetzen. Diese können Mono-Triarylamine darstellen, wie beispielsweise in JP 1995/053955, WO 2006/123667 und JP 2010/222268 beschrieben, oder Bis- oder andere Oligoamine darstellen, wie beispielsweise in US 7504163 oder US 2005/0184657 beschrieben. Bekannte Beispiele für Triarylamin-Verbindungen als Materialien mit lochtransportierenden Eigenschaften für OLEDs sind unter anderem Tris-p-biphenyl-amin, N,N'-Di-1-naphthyl-N,N'-diphenyl-1,1'-biphenyl-4,4'-diamin (NPB) und 4,4',4"-Tris-(3-methylphenylphenylamino)triphenylamin (MTDATA).

Es ist im Stand der Technik bekannt, Acridin-Derivate in OLEDs zu verwenden, beispielsweise aus WO 2012/150001. Spiro-Bisacridin-Derivate werden in dieser Offenlegungsschrift jedoch nicht vorgeschlagen.

Weiterhin im Stand der Technik bekannt ist die Verwendung von Spiro-Bisacridin-Verbindungen in OLEDs, beispielsweise aus JP 2002-265938. Die in dieser Offenlegungsschrift beschriebenen Verbindungen weisen entweder keine Substituenten an den Benzolringen des Spiro-Bisacridin-Grundgerüsts auf, oder sie weisen Phenylgruppen an den Stickstoffatomen des Spiro-Bisacridin-Grundgerüsts auf.

Eine weitere Offenlegungsschrift, die die Verwendung von Spiro-Bisacridin-Verbindungen in OLEDs beschreibt, ist KR 2011-0120075. Die in dieser Offenlegungsschrift beschriebenen Verbindungen weisen keine Substituenten an den Benzolringen des Spiro-Bisacridin-Grundgerüsts auf.

Auch wenn die in den oben genannten Dokumenten offenbarten Verbindungen gut geeignet zur Verwendung in elektronischen Vorrichtungen sind, besteht doch weiterhin Bedarf an neuen Verbindungen für diese Verwendung. Insbesondere besteht Bedarf an Verbindungen, die zu einer Verbesserung der Leistungsdaten der elektronischen Vorrichtung, insbesondere zu einer Verbesserung von Lebensdauer, Effizienz und Betriebsspannung, führen. Insbesondere zur Verwendung in lochtransportierenden Schichten der elektronischen Vorrichtungen werden kontinuierlich neue Materialien mit entsprechenden Eigenschaften gesucht.

Im Rahmen von Untersuchungen zu neuartigen Materialien für diese Verwendung wurde nun überraschend gefunden, dass sich Spiro-Bisacridinverbindungen, welche der unten definierten Formel (I) entsprechen und insbesondere dadurch gekennzeichnet sind, dass sie ein ausgedehntes aromatisches oder heteroaromatisches System an mindestens einem der Stickstoffatome aufweisen und ein aromatisches oder heteroaromatisches Ringsystem an mindestens einem der Benzolringe der Spiro-Bisacridin-Grundgerüsts aufweisen, hervorragend zur Verwendung in OLEDs eignen. Sie eignen sich insbesondere zur Verwendung in einer lochtransportierenden Schicht.

Die gefundenen Verbindungen weisen eine oder mehrere Eigenschaften gewählt aus sehr guten lochleitenden Eigenschaften, sehr guten elektronenblockierenden Eigenschaften, hoher Oxidationsstabilität, guter Löslichkeit, und hoher Temperaturstabilität auf. Bei Verwendung in OLEDs führen sie zu einer oder mehreren vorteilhaften Eigenschaften der OLEDs, gewählt aus langer Lebensdauer, hoher Quanteneffizienz und niedriger Betriebsspannung.

Gegenstand der vorliegenden Erfindung ist eine Verbindung der Formel (I) 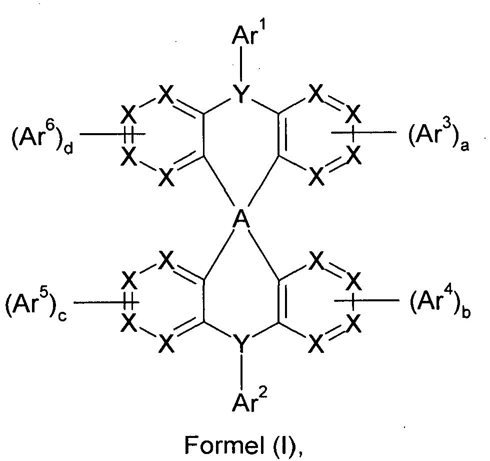 wobei für die auftretenden Symbole und Indices gilt:
- A: ist C oder Si;
- Y: ist bei jedem Auftreten gleich oder verschieden N oder P;
- X: ist gleich CR¹;
- Ar¹, Ar²: ist bei jedem Auftreten gleich oder verschieden ein aromatisches Ringsystem mit 6 bis 40 aromatischen Ringatomen, das mit einem oder mehreren Resten R² substituiert sein kann, oder ein heteroaromatisches Ringsystem mit 5 bis 40 aromatischen Ringatomen, das mit einem oder mehreren Resten R² substituiert sein kann;
- Ar³, Ar⁴, Ar⁵, Ar⁶: ist bei jedem Auftreten gleich oder verschieden ein aromatisches Ringsystem mit 6 bis 40 aromatischen Ringatomen, das mit einem oder mehreren Resten R² substituiert sein kann, oder ein heteroaromatisches Ringsystem mit 5 bis 40 aromatischen Ringatomen, das mit einem oder mehreren Resten R² substituiert sein kann;
- R¹, R²: ist bei jedem Auftreten gleich oder verschieden gewählt aus H, D, F, C(=O)R³, CF₃, OCF₃, CN, Si(R³)₃, N(R³)₂, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxy-gruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ bzw. R² miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C=C-, Si(R³)₂, C=O, C=NR³, -C(=O)O-, -C(=O)NR³-, NR³, P(=O)(R³), -O-, -S-, SO oder SO₂ ersetzt sein können;
- R³: ist bei jedem Auftreten gleich oder verschieden gewählt aus H, D, F, C(=O)R⁴, CF₃, OCF₃, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch - R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
- R⁴: ist bei jedem Auftreten gleich oder verschieden gewählt aus H, D, F, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁴ miteinander verknüpft sein können und einen Ring bilden können; und wobei die genannten Alkylgruppen, aromatischen Ringsysteme und heteroaromatischen Ringsysteme mit F oder CN substituiert sein können;
a, b, c, d ist bei jedem Auftreten gleich oder verschieden 0 oder 1;
wobei mindestens eine der beiden Gruppen Ar¹ und Ar² ein aromatisches Ringsystem mit 12 bis 40 aromatischen Ringatomen ist, das mit einem oder mehreren Resten R² substituiert sein kann, oder ein heteroaromatisches Ringsystem mit 12 bis 40 aromatischen Ringatomen ist, das mit einem oder mehreren Resten R² substituiert sein kann; und
wobei mindestens einer der Indices a, b, c und d gleich 1 ist.

Wenn ein Index a, b, c oder d gleich 0 ist, entfällt die entsprechende Gruppe Ar³, Ar⁴, Ar⁵ oder Ar⁶.

Wenn ein Index a, b, c oder d gleich 1 ist, ist die entsprechende Gruppe Ar³, Ar⁴, Ar⁵ oder Ar⁶ an eine der Gruppen X des Rings gebunden. Diese Gruppe X ist dann entsprechend der Vierbindigkeit des Kohlenstoffs gleich C.

Eine Arylgruppe im Sinne dieser Erfindung enthält 6 bis 60 aromatische Ringatome, von denen keines ein Heteroatom darstellt. Unter einer Arylgruppe im Sinne dieser Erfindung wird entweder ein einfacher aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einfachen aromatischen Cyclen. Unter Kondensation zwischen Cyclen ist dabei zu verstehen, dass die Cyclen mindestens eine Kante miteinander teilen.

Eine Heteroarylgruppe im Sinne dieser Erfindung enthält 5 bis 60 aromatische Ringatome, von denen mindestens eines ein Heteroatom darstellt. Die Heteroatome der Heteroarylgruppe sind bevorzugt ausgewählt aus N, O und S. Unter einer Heteroarylgruppe im Sinne dieser Erfindung wird entweder ein einfacher heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einfachen heteroaromatischen Cyclen. Unter Kondensation zwischen Cyclen ist dabei zu verstehen, dass die Cyclen mindestens eine Kante miteinander teilen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 60 C-Atome im Ringsystem und umfasst keine Heteroatome als aromatische Ringatome. Ein aromatisches Ringsystem im Sinne dieser Erfindung enthält daher keine Heteroarylgruppen. Unter einem aromatischen Ringsystem im Sinne dieser Erfindung soll ein System verstanden werden, das nicht notwendigerweise nur Arylgruppen enthält, sondern in dem auch mehrere Arylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome, bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehr Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Arylgruppen über Einfachbindungen miteinander verknüpft sind, als aromatische Ringsysteme im Sinne dieser Erfindung verstanden, wie beispielsweise Systeme wie Biphenyl und Terphenyl.

Ein heteroaromatisches Ringsystem im Sinne dieser Erfindung enthält 5 bis 60 aromatische Ringatome, von denen mindestens eines ein Heteroatom darstellt. Die Heteroatome des heteroaromatischen Ringsystems sind bevorzugt ausgewählt aus N, O und/oder S. Ein heteroaromatisches Ringsystem entspricht der oben genannten Definition eines aromatischen Ringsystems, weist jedoch mindestens ein Heteroatom als eines der aromatischen Ringatome auf. Es unterscheidet sich dadurch von einem aromatischen Ringsystem im Sinne der Definition der vorliegenden Anmeldung, welches gemäß dieser Definition kein Heteroatom als aromatisches Ringatom enthalten kann.

Unter einem aromatischen Ringsystem mit 6 bis 60 aromatischen Ringatomen oder einem heteroaromatischen Ringsystem mit 5 bis 60 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 40 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Gemäß einer bevorzugten Ausführungsform umfasst die Verbindung der Formel (I) keine Arylaminogruppe als Substituent. Unter einer Arylaminogruppe wird im Sinne der vorliegenden Anmeldung eine Gruppe verstanden, in der ein oder mehrere Aryl- oder Heteroarylgruppen, bevorzugt drei Aryl- oder Heteroarylgruppen, an ein Stickstoffatom binden.

Gemäß einer weiteren bevorzugten Ausführungsform der Erfindung umfasst die Verbindung der Formel (I) keine kondensierte Arylgruppe mit mehr als 10 aromatischen Ringatomen und keine kondensierte Heteroarylgruppe mit mehr als 14 aromatischen Ringatomen.

Bevorzugt sind genau 1, 2 oder 3 Indices gewählt aus den Indices a, b, c und d gleich 1, besonders bevorzugt sind genau 1 oder 2 Indices gewählt aus den Indices a, b, c und d gleich 1.

Gemäß einer bevorzugten Ausführungsform der Erfindung ist der Index a gleich 1, und die Indices b, c und d sind gleich 0.

Gemäß einer alternativen bevorzugten Ausführungsform sind die Indices a und b gleich 1, und die Indices c und d sind gleich 0.

Gemäß einer alternativen bevorzugten Ausführungsform sind die Indices a und c gleich 1, und die Indices b und d sind gleich 0.

Bevorzugt ist A ein Kohlenstoffatom.

Bevorzugt ist Y ein Stickstoffatom.

Bevorzugt sind Ar¹ und Ar² bei jedem Auftreten gleich oder verschieden gewählt aus aromatischen Ringsystemen mit 6 bis 24 aromatischen Ringatomen, die mit einem oder mehreren Resten R² substituiert sein können, oder aus heteroaromatischen Ringsystemen mit 5 bis 24 aromatischen Ringatomen, die mit einem oder mehreren Resten R² substituiert sein können. Es ist in Kombination damit bevorzugt, dass mindestens eine der beiden Gruppen Ar¹ und Ar² ein aromatisches Ringsystem mit 12 bis 24 aromatischen Ringatomen ist, das mit einem oder mehreren Resten R² substituiert sein kann, oder ein heteroaromatisches Ringsystem mit 12 bis 24 aromatischen Ringatomen ist, das mit einem oder mehreren Resten R² substituiert sein kann. Besonders bevorzugt sind beide der Gruppen Ar¹ und Ar² bei jedem Auftreten gleich oder verschieden gewählt aus aromatischen Ringsystemen mit 12 bis 24 aromatischen Ringatomen, die mit einem oder mehreren Resten R² substituiert sein können, und heteroaromatischen Ringsystemen mit 12 bis 24 aromatischen Ringatomen, die mit einem oder mehreren Resten R² substituiert sein können.

Bevorzugt enthalten Ar¹, Ar², Ar³, Ar⁴, Ar⁵ und Ar⁶ jeweils mindestens eine Gruppe gewählt aus Benzol, Naphthalin, Phenanthren, Fluoranthen, Biphenyl, Terphenyl, Quaterphenyl, Fluoren, Indenofluoren, Spirobifluoren, Furan, Benzofuran, Isobenzofuran, Dibenzofuran, Thiophen, Benzothiophen, Isobenzothiophen, Dibenzothiophen, Indol, Isoindol, Carbazol, Indolocarbazol, Indenocarbazol, Pyridin, Chinolin, Isochinolin, Acridin, Phenanthridin, Benzimidazol, Pyrimidin, Pyrazin, und Triazin, wobei die genannten Gruppen jeweils mit einem oder mehreren Resten R² substituiert sein können.

Es ist bevorzugt, dass Ar³, Ar⁴, Ar⁵, Ar⁶ bei jedem Auftreten gleich oder verschieden gewählt sind aus aromatischen Ringsystemen mit 6 bis 24 aromatischen Ringatomen, die mit einem oder mehreren Resten R² substituiert sein können, oder aus heteroaromatischen Ringsystemen mit 5 bis 24 aromatischen Ringatomen, die mit einem oder mehreren Resten R² substituiert sein können.

Es ist bevorzugt, dass die auftretenden Gruppen Ar³, Ar⁴, Ar⁵, Ar⁶ in der Verbindung der Formel (I) gleich gewählt sind.

Bevorzugte Ausführungsformen der Gruppen Ar¹, Ar², Ar³, Ar⁴, Ar⁵ und Ar⁶ sind die im Folgenden genannten Gruppen: 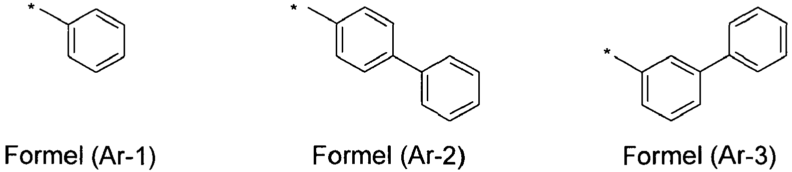 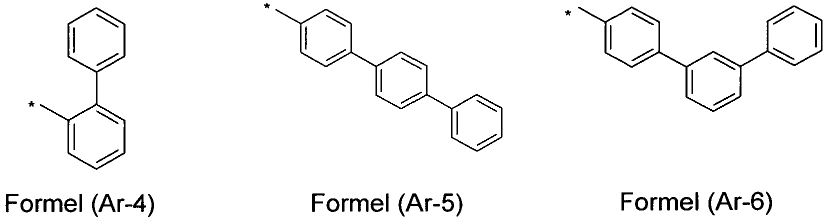 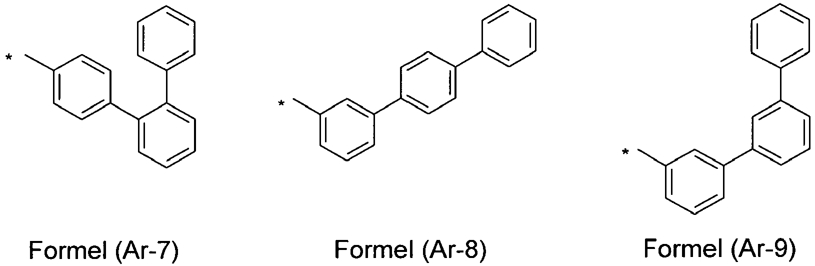 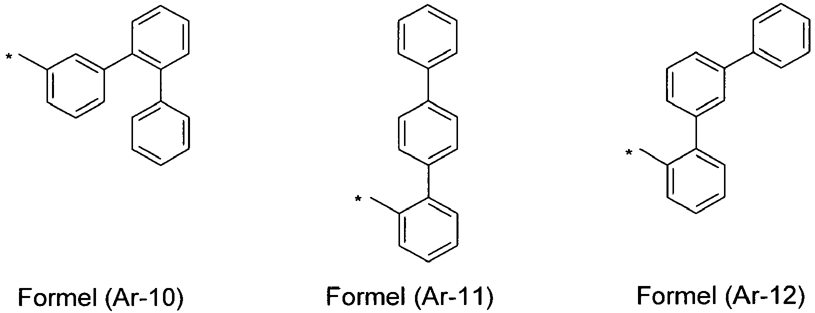 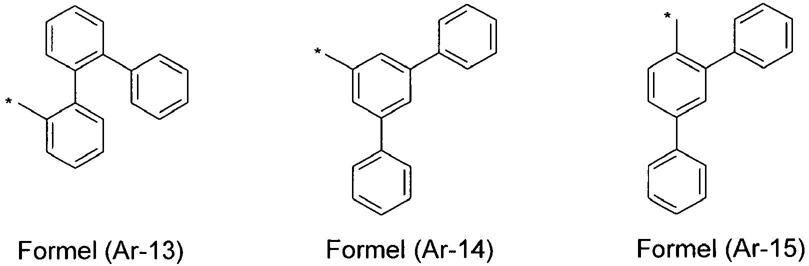 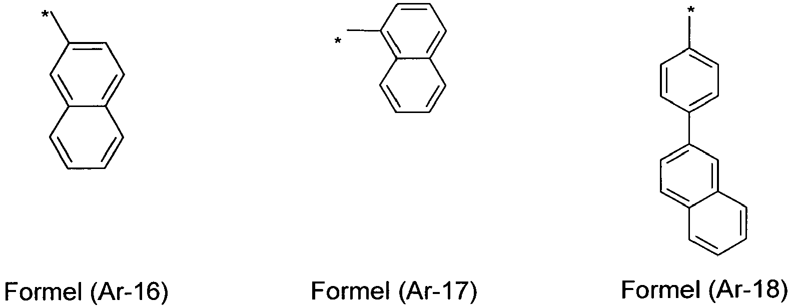 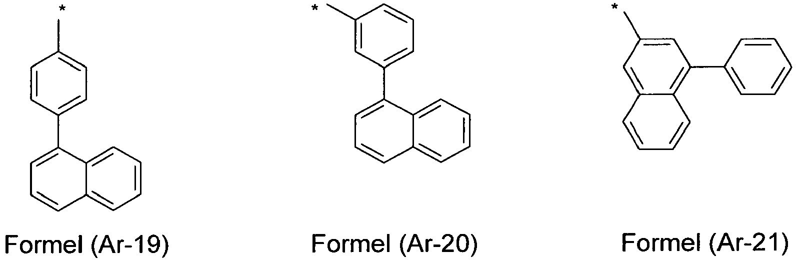 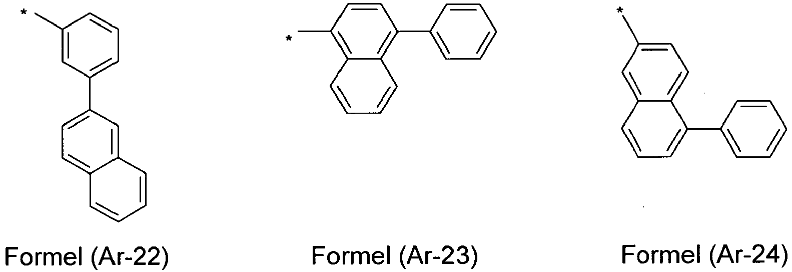 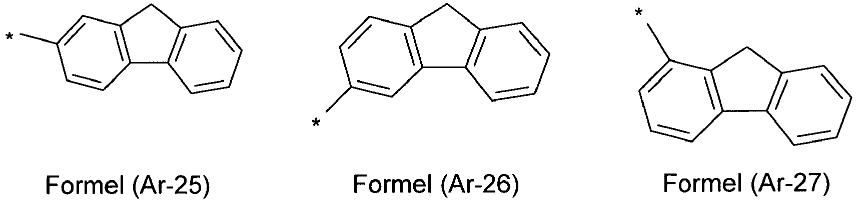 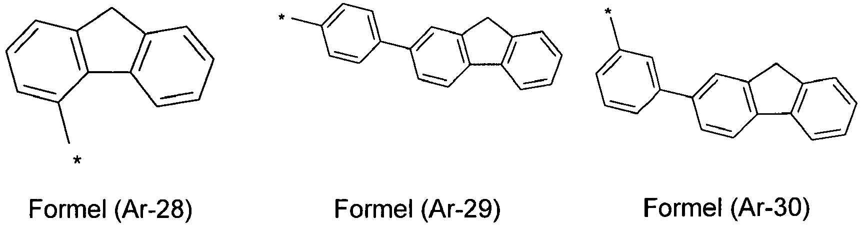 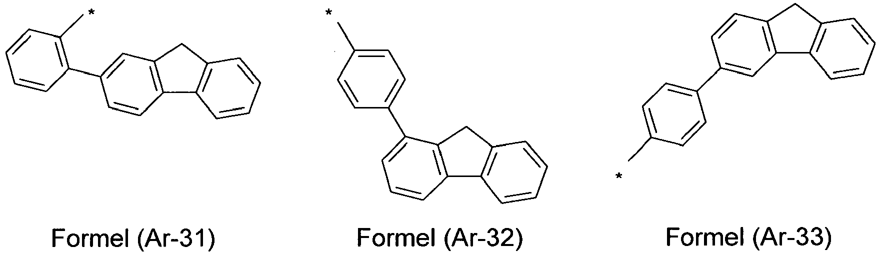 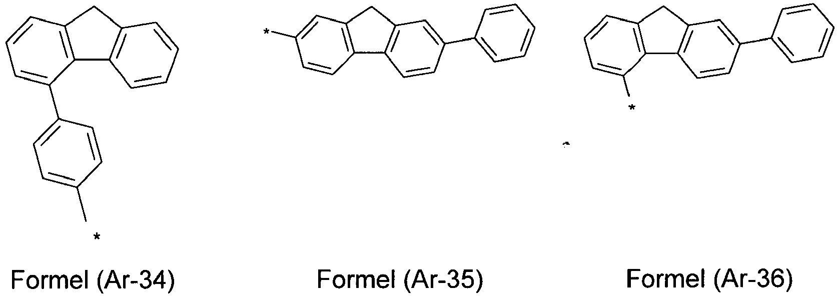 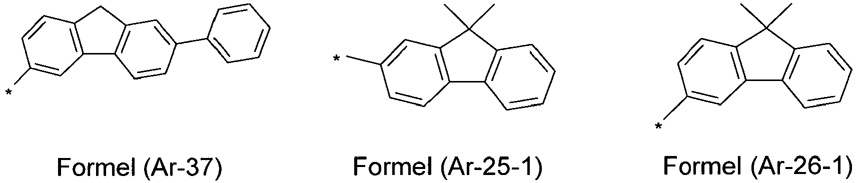 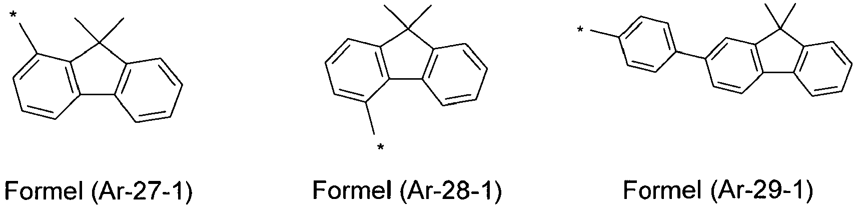 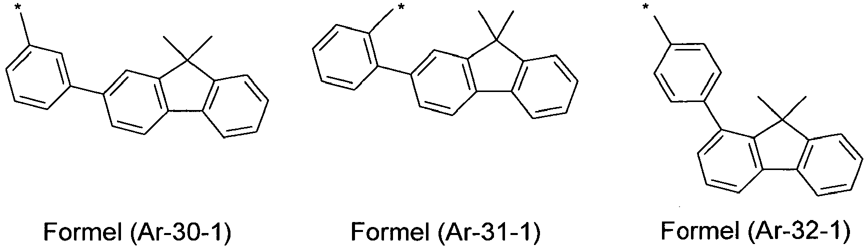 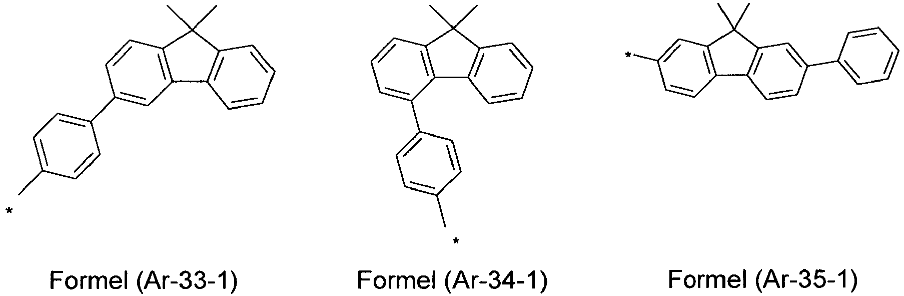 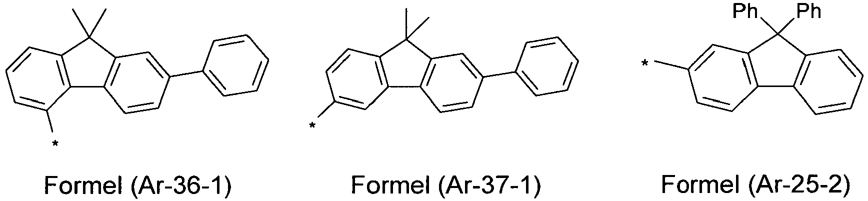 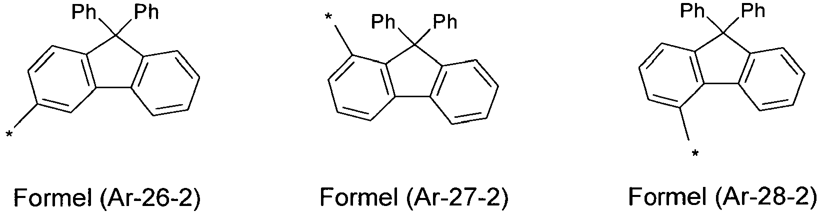 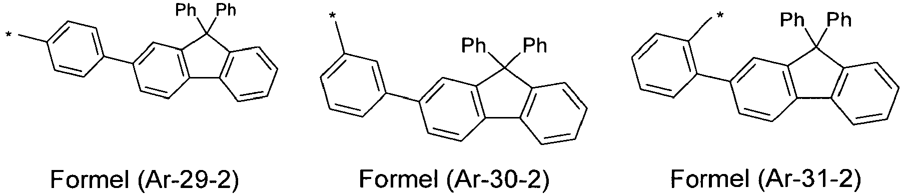 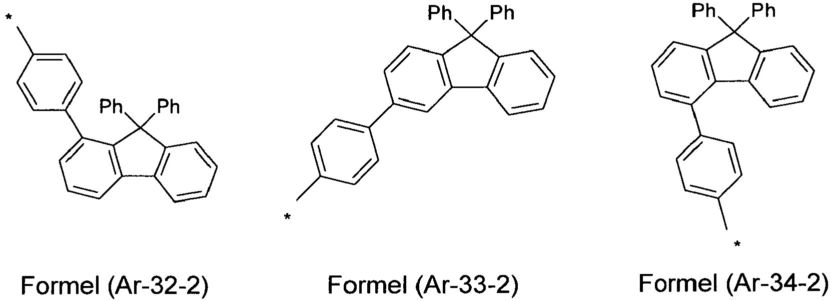 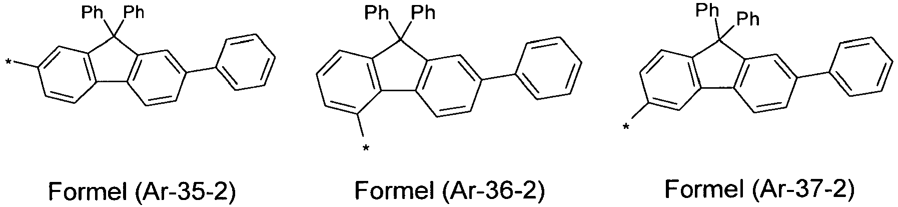 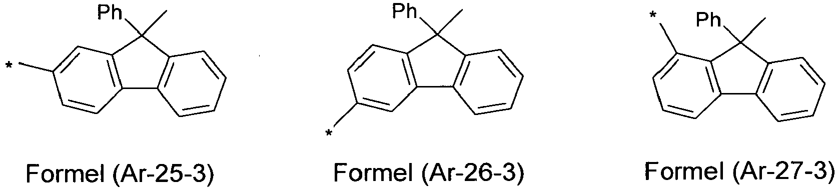 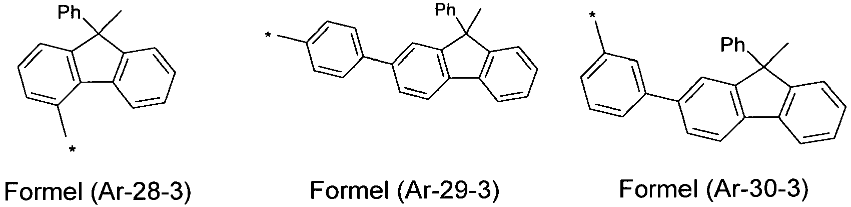 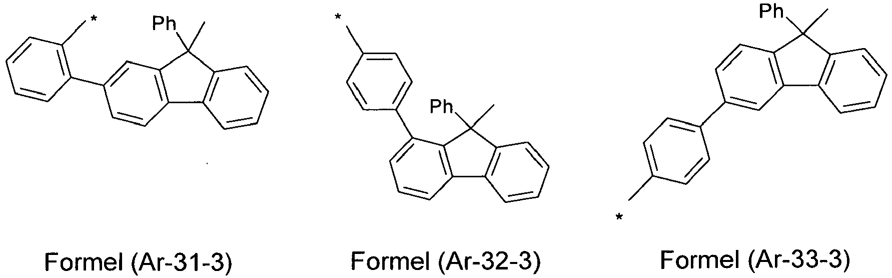 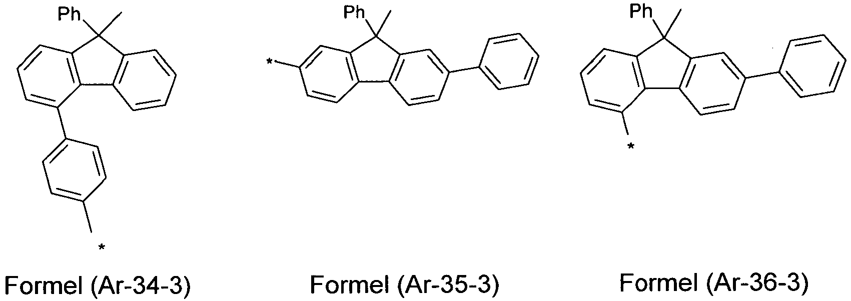 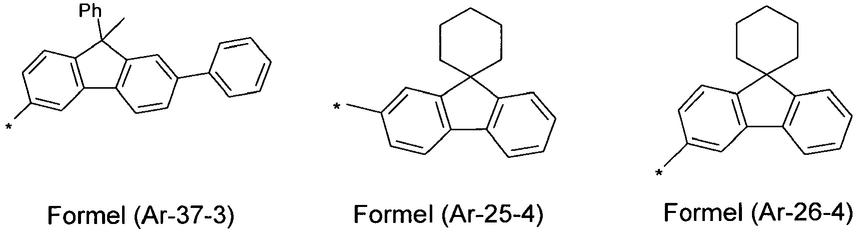 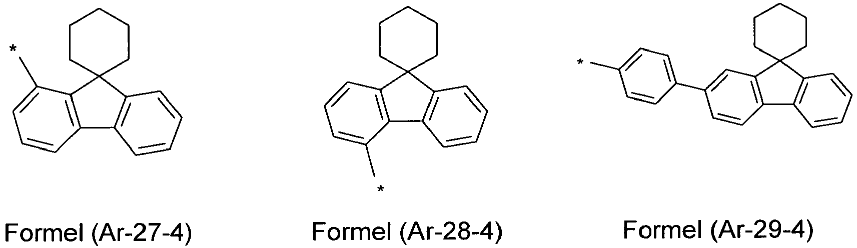 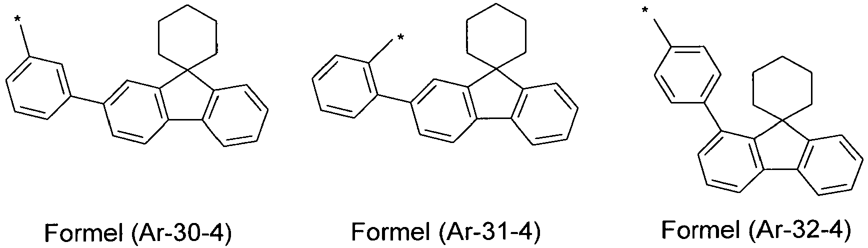 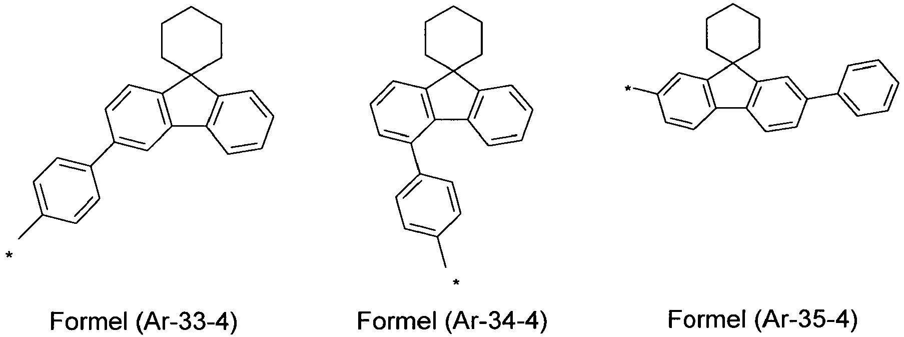 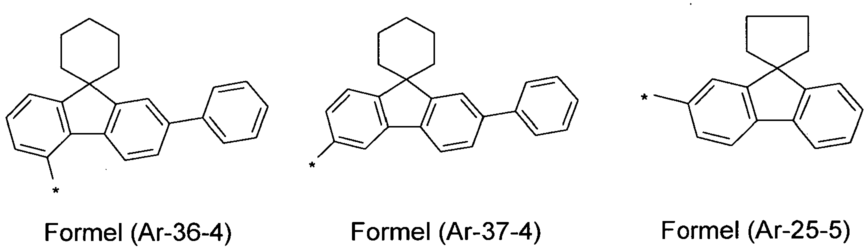 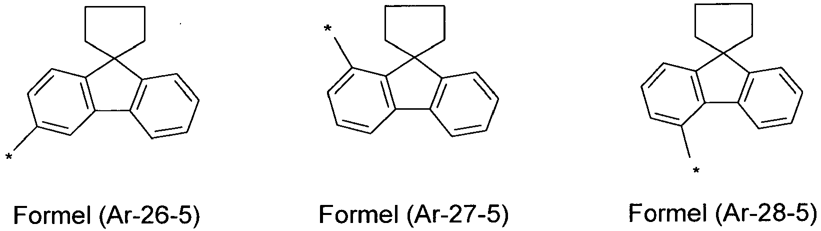 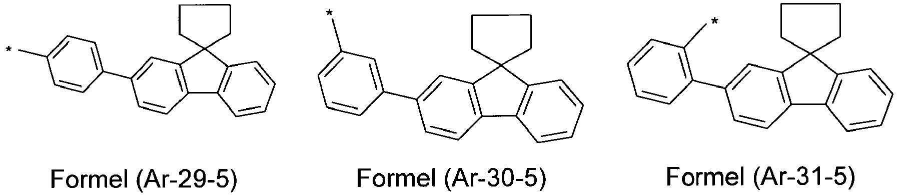 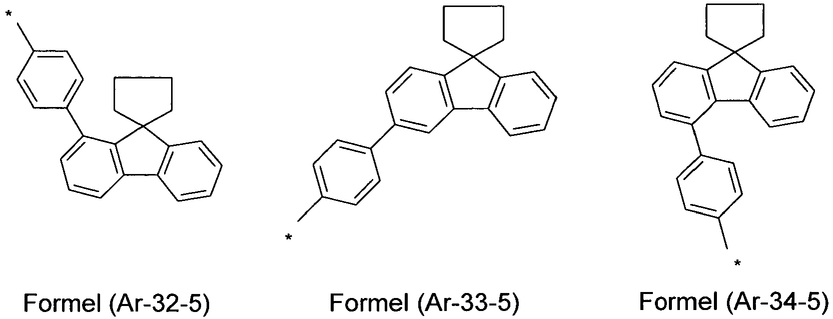 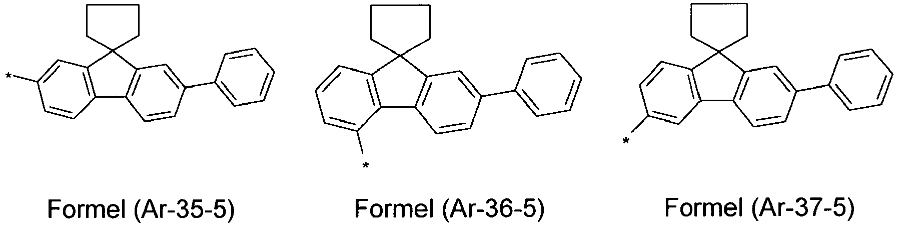 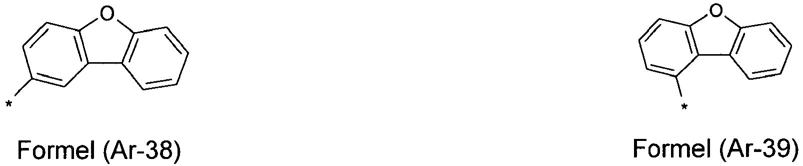 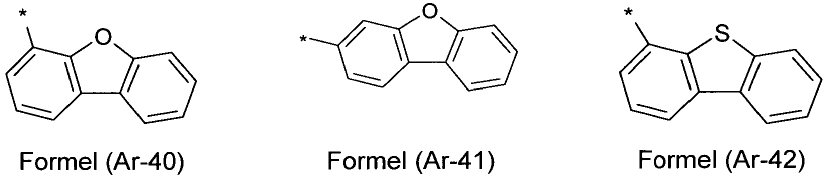 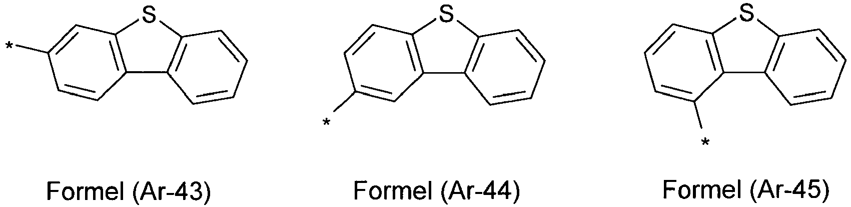 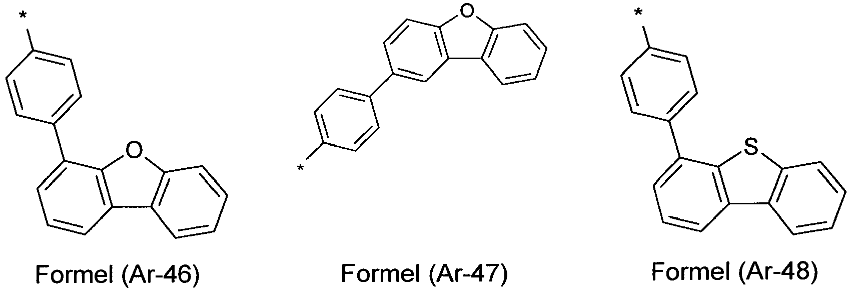 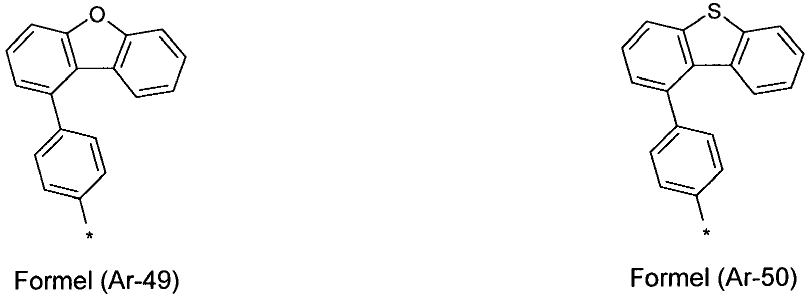 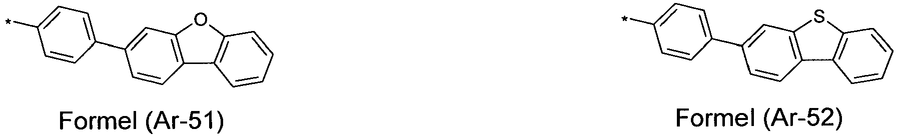 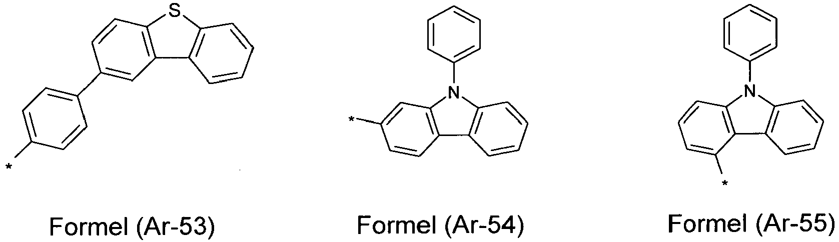 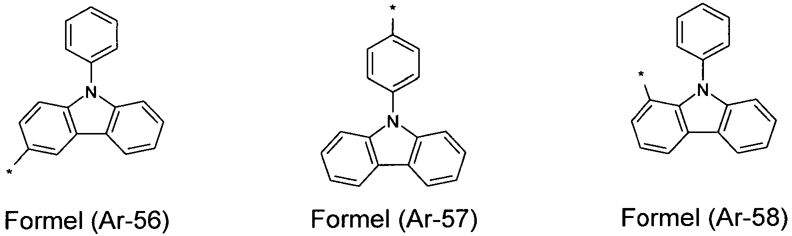 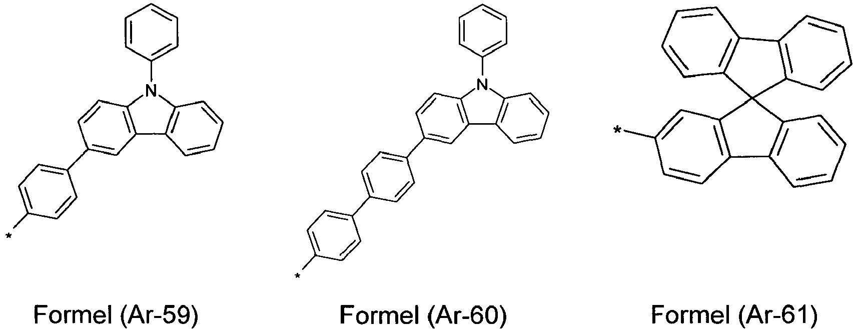 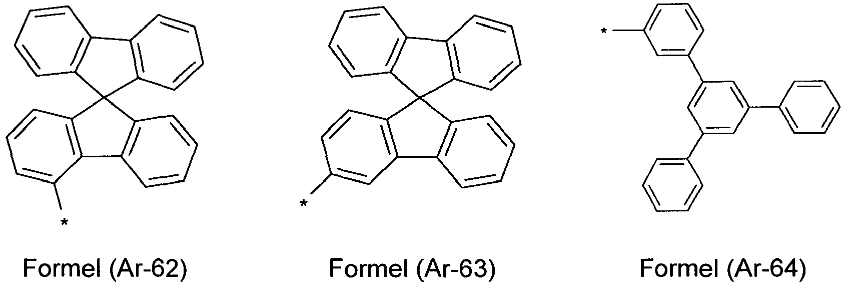 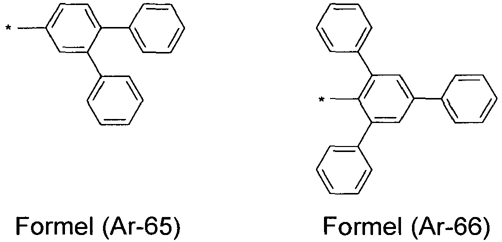 wobei die genannten Gruppen an einer oder mehreren der freien Positionen jeweils mit Resten R² substituiert sein können, und wobei die mit * gekennzeichnete Bindung die Anbindungsposition der jeweiligen Gruppe darstellt.

Bevorzugt ist mindestens eine der Gruppen Ar¹ und Ar² gewählt aus einer der oben genannten Gruppen der Formeln (Ar-2) bis (Ar-15) und (Ar-18) bis (Ar-66). Besonders bevorzugt sind beide Gruppen Ar¹ und Ar² gewählt aus einer der oben genannten Gruppen (Ar-2) bis (Ar-15) und (Ar-18) bis (Ar-66).

Bevorzugt umfasst mindestens eine der Gruppen Ar¹, Ar², Ar³, Ar⁴, Ar⁵ und Ar⁶ mindestens eine Heteroarylgruppe, wie oben definiert. Bevorzugt weist die mindestens eine Heteroarylgruppe 5 bis 20 aromatische Ringatome auf, besonders bevorzugt 6 bis 14 aromatische Ringatome.

Bevorzugt ist der Rest R¹ bei jedem Auftreten gleich oder verschieden gewählt aus H, D, F, CN, Si(R³)₃, geradkettigen Alkyl- oder Alkoxygruppen mit 1 bis 10 C-Atomen, verzweigten oder cyclischen Alkyl- oder Alkoxygruppen mit 3 bis 10 C-Atomen, aromatischen Ringsystemen mit 6 bis 24 aromatischen Ringatomen, und heteroaromatischen Ringsystemen mit 5 bis 24 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R³ substituiert sein können; und wobei in den genannten Alkyl- oder Alkoxygruppen eine oder mehrere CH₂-Gruppen durch -C=C-, - R³C=CR³-, Si(R³)₂, C=O, C=NR³, -NR³-, -O-, -S-, -C(=O)O- oder - C(=O)NR³- ersetzt sein können. Besonders bevorzugt ist der Rest R¹ bei jedem Auftreten gleich oder verschieden gewählt aus H, F, CN, Si(R³)₃, aromatischen Ringsystemen mit 6 bis 24 aromatischen Ringatomen, und heteroaromatischen Ringsystemen mit 5 bis 24 aromatischen Ringatomen; wobei die genannten aromatischen Ringsysteme und die genannten heteroaromatischen Ringsysteme jeweils mit einem oder mehreren Resten R³ substituiert sein können. Ganz besonders bevorzugt ist der Rest R¹ gleich H.

Bevorzugt ist der Rest R² bei jedem Auftreten gleich oder verschieden gewählt aus H, D, F, CN, Si(R³)₃, geradkettigen Alkyl- oder Alkoxygruppen mit 1 bis 10 C-Atomen, verzweigten oder cyclischen Alkyl- oder Alkoxygruppen mit 3 bis 10 C-Atomen, aromatischen Ringsystemen mit 6 bis 24 aromatischen Ringatomen, und heteroaromatischen Ringsystemen mit 5 bis 24 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R³ substituiert sein können; und wobei in den genannten Alkyl- oder Alkoxygruppen eine oder mehrere CH₂-Gruppen durch -C=C-, - R³C=CR³-, Si(R³)₂, C=O, C=NR³, -NR³-, -O-, -S-, -C(=O)O- oder - C(=O)NR³- ersetzt sein können. Besonders bevorzugt ist der Rest R² bei jedem Auftreten gleich oder verschieden gewählt aus H, F, CN, Si(R³)₃, geradkettigen Alkylgruppen mit 1 bis 10 C-Atomen, verzweigten oder cyclischen Alkylgruppen mit 3 bis 10 C-Atomen, aromatischen Ringsystemen mit 6 bis 24 aromatischen Ringatomen, und heteroaromatischen Ringsystemen mit 5 bis 24 aromatischen Ringatomen; wobei die genannten Alkylgruppen, die genannten aromatischen Ringsysteme und die genannten heteroaromatischen Ringsysteme jeweils mit einem oder mehreren Resten R³ substituiert sein können.

Bevorzugt ist der Rest R³ bei jedem Auftreten gleich oder verschieden gewählt aus H, D, F, CN, Si(R⁴)₃, geradkettigen Alkyl- oder Alkoxygruppen mit 1 bis 10 C-Atomen, verzweigten oder cyclischen Alkyl- oder Alkoxygruppen mit 3 bis 10 C-Atomen, aromatischen Ringsystemen mit 6 bis 24 aromatischen Ringatomen, und heteroaromatischen Ringsystemen mit 5 bis 24 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R⁴ substituiert sein können; und wobei in den genannten Alkyl- oder Alkoxygruppen eine oder mehrere CH₂-Gruppen durch -C=C-, - R⁴C=CR⁴-, Si(R⁴)₂, C=O, C=NR⁴, -NR⁴-, -O-, -S-, -C(=O)O- oder-C(=O)NR⁴- ersetzt sein können. Besonders bevorzugt ist der Rest R³ bei jedem Auftreten gleich oder verschieden gewählt aus H, F, CN, Si(R⁴)₃, geradkettigen Alkylgruppen mit 1 bis 10 C-Atomen, verzweigten oder cyclischen Alkylgruppen mit 3 bis 10 C-Atomen, aromatischen Ringsystemen mit 6 bis 24 aromatischen Ringatomen, und heteroaromatischen Ringsystemen mit 5 bis 24 aromatischen Ringatomen; wobei die genannten Alkylgruppen, die genannten aromatischen Ringsysteme und die genannten heteroaromatischen Ringsysteme jeweils mit einem oder mehreren Resten R⁴ substituiert sein können.

Bevorzugt entspricht die Verbindung der Formel (I) einer der Formeln (I-1) bis (I-3) 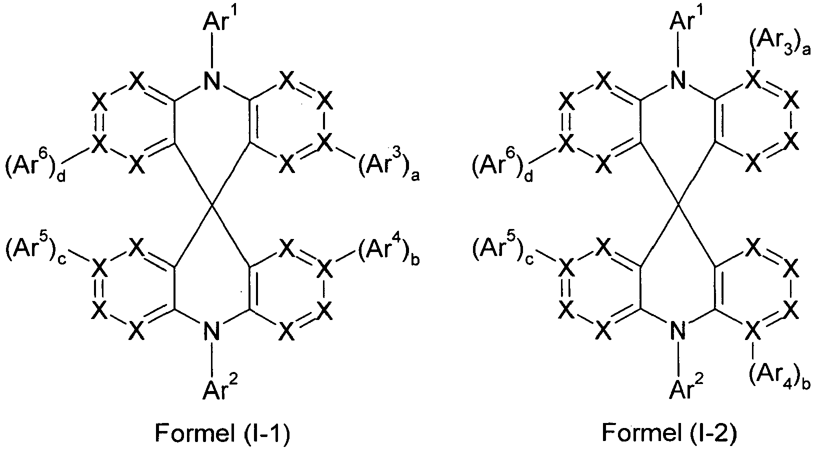 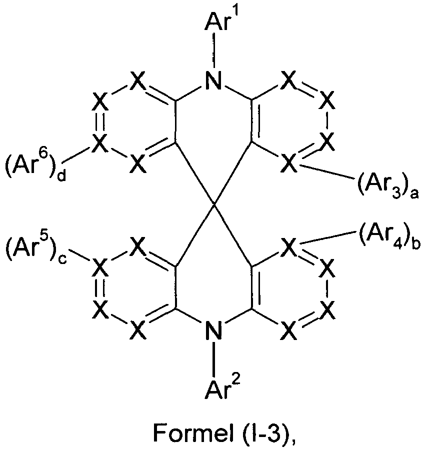 wobei die auftretenden Symbole und Indices definiert sind wie oben.

Insbesondere ist es für die Verbindungen der Formeln (I-1) bis (I-3) bevorzugt, dass genau 1, genau 2 oder genau 3 Indices gewählt aus den Indices a, b, c und d gleich 1 sind.

Bevorzugt unter den Formeln (I-1) bis (I-3) ist Formel (I-1).

Besonders bevorzugt entspricht die Verbindung der Formel (I) einer der Formeln (I-1-1) bis (I-1-4) 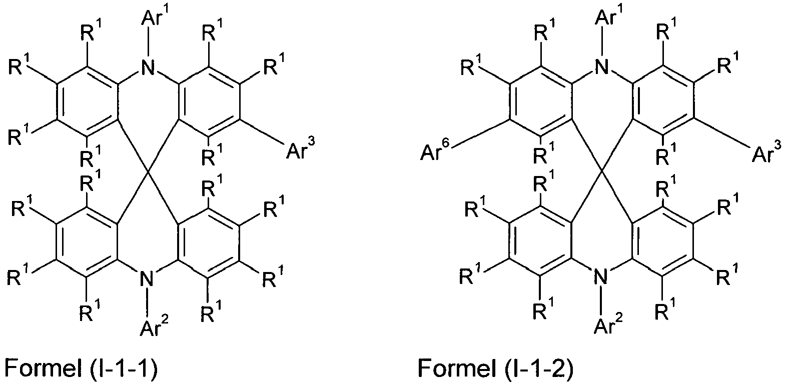 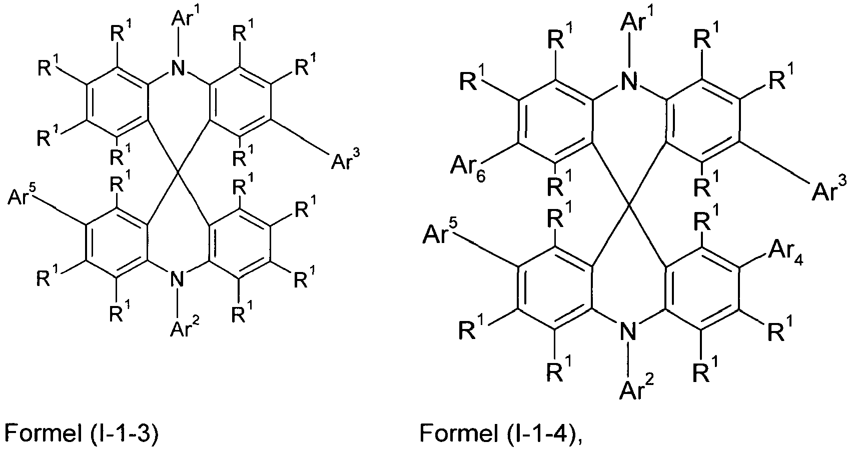 wobei die auftretenden Symbole definiert sind wie oben.

Insbesondere bevorzugt ist die Kombination der bevorzugten Ausführungsformen der Formeln (I-1-1) bis (I-1-4) mit den oben angegebenen bevorzugten Ausführungsformen der Gruppen R¹, Ar¹, Ar², Ar³, Ar⁴, Ar⁵ und Ar⁶ .

Beispiele für Verbindungen gemäß Formel (I) sind in der folgenden Tabelle abgebildet:

| | |
|---|---|
| 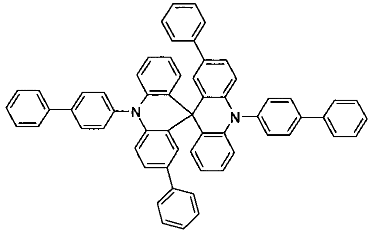 | 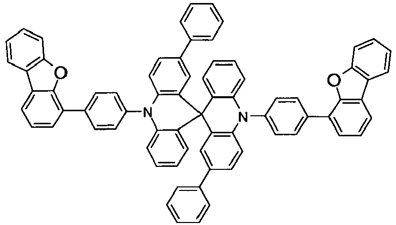 |
| 1 | 2 |
| 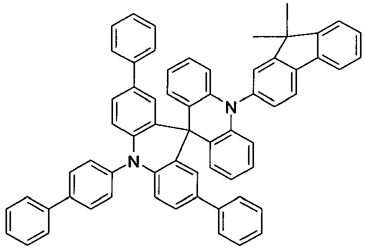 | 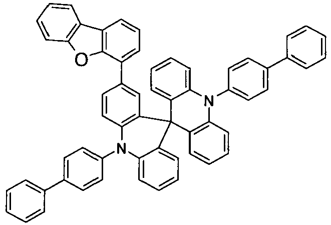 |
| 3 | 4 |
| 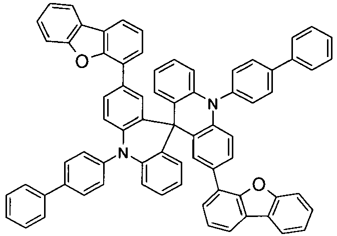 | 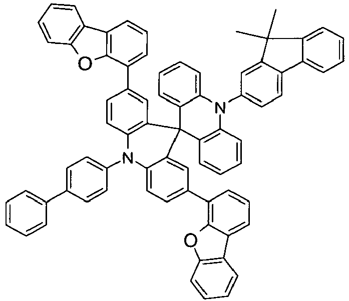 |
| 5 | 6 |
| 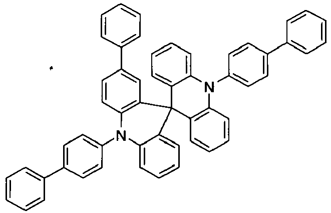 | 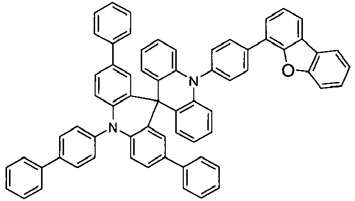 |
| 7 | 8 |
| 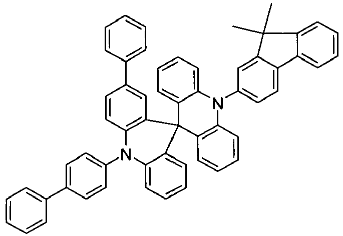 | 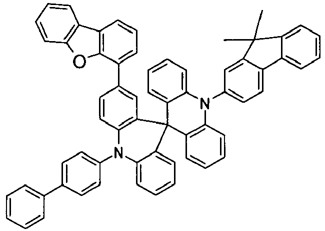 |
| 9 | 10 |
| 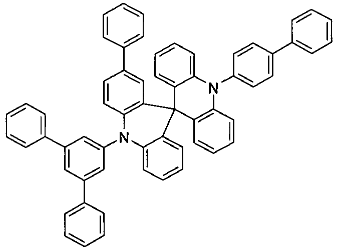 | 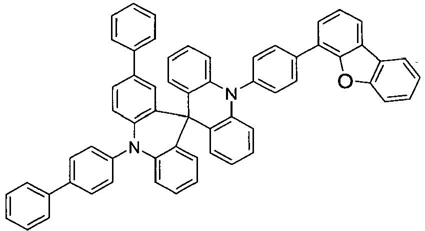 |
| 11 | 12 |
| 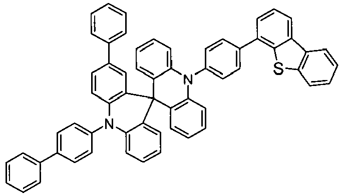 | 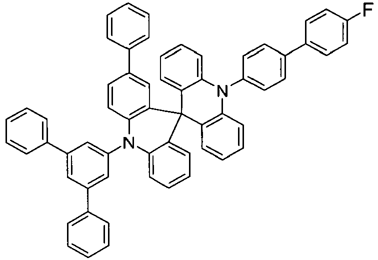 |
| 13 | 14 |
| 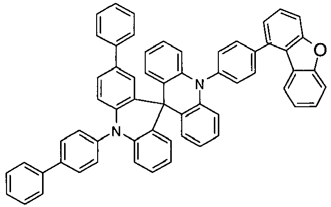 | 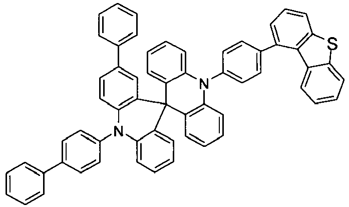 |
| 15 | 16 |
| 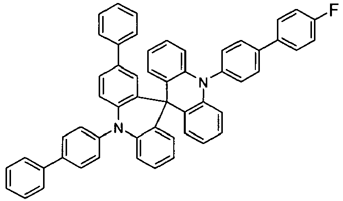 | 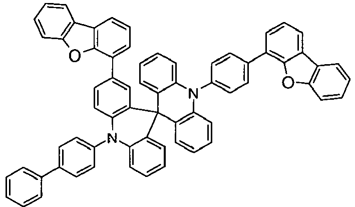 |
| 17 | 18 |
| 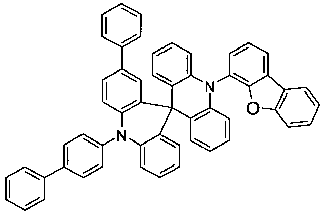 | 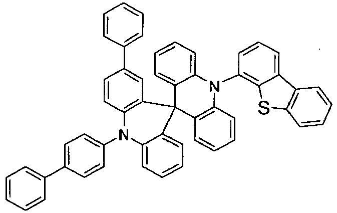 |
| 19 | 20 |
| 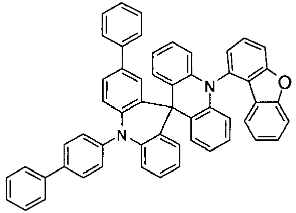 | 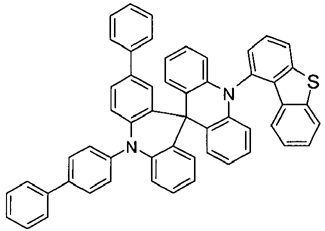 |
| 21 | 22 |
| 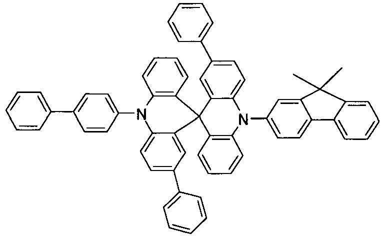 | 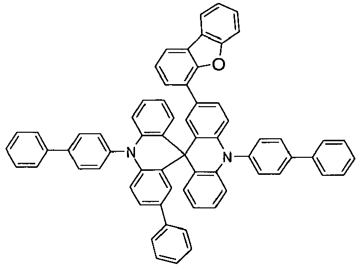 |
| 23 | 24 |
| 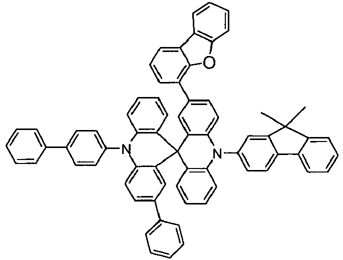 | 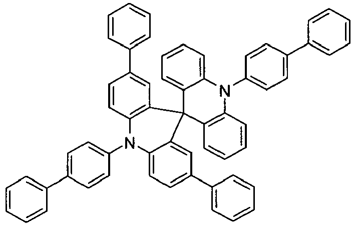 |
| 25 | 26 |
| 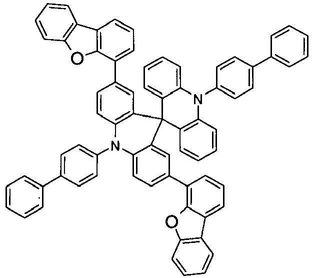 | 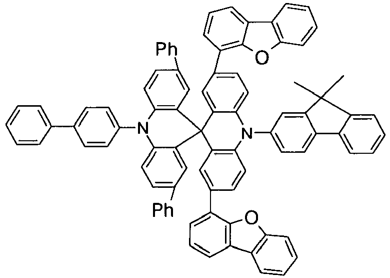 |
| 27 | 28 |
| 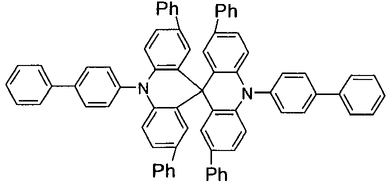 | 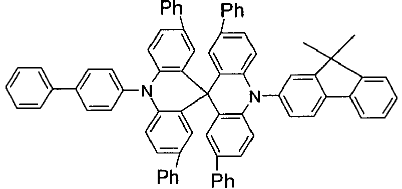 |
| 29 | 30 |
| 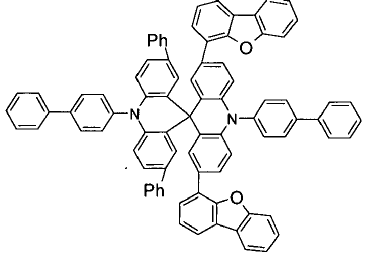 | 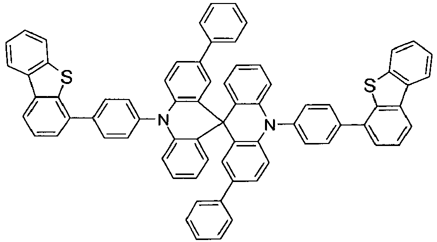 |
| 31 | 32 |
| 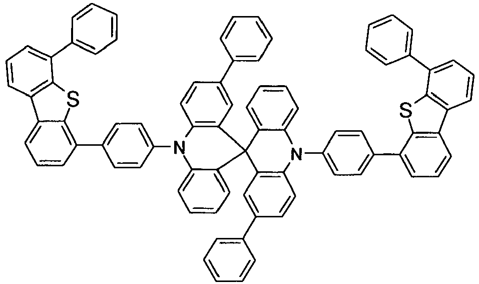 | 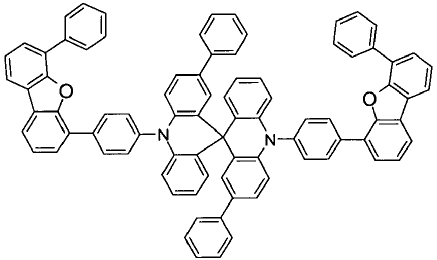 |
| 33 | 34 |
| 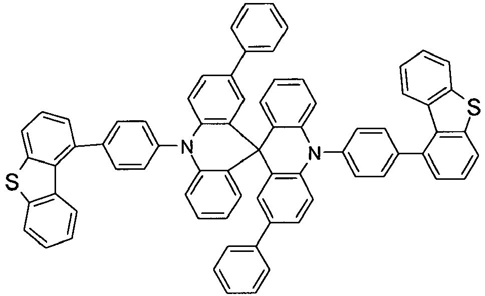 | 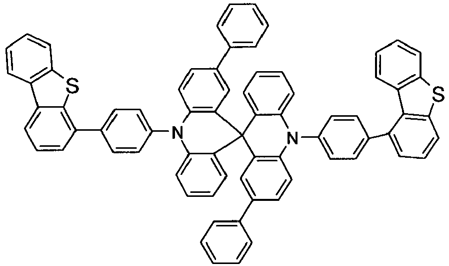 |
| 35 | 36 |
| 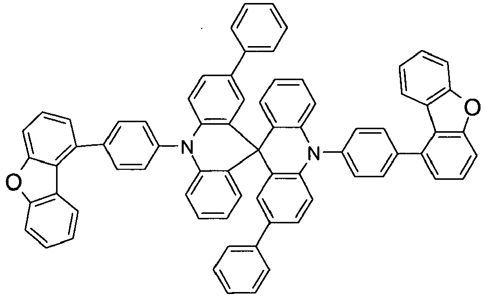 | 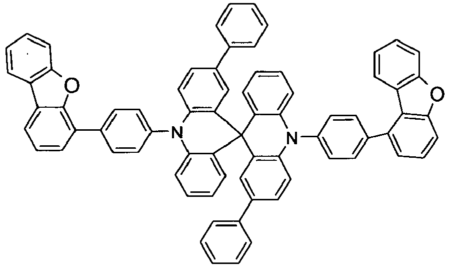 |
| 37 | 38 |
| 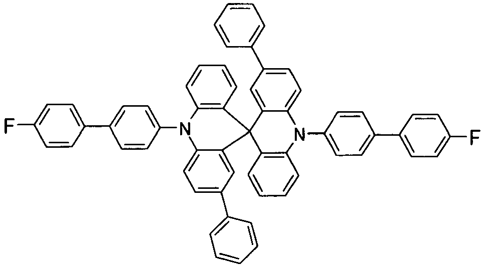 | 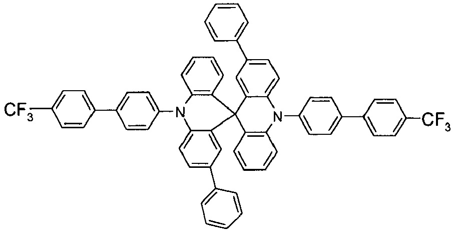 |
| 39 | 40 |
| 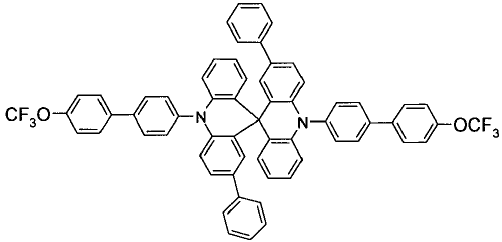 | 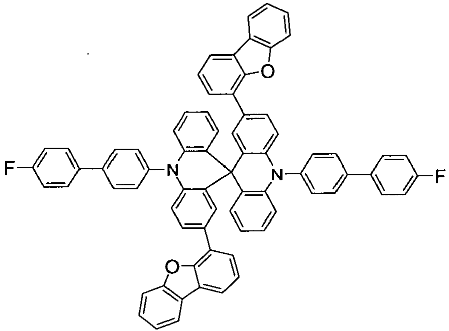 |
| 41 | 42 |
| 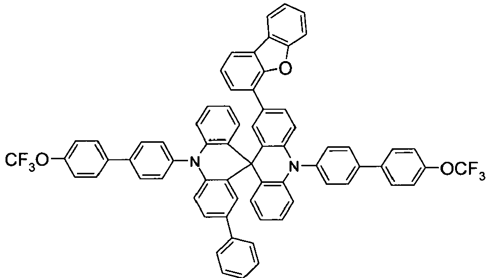 | 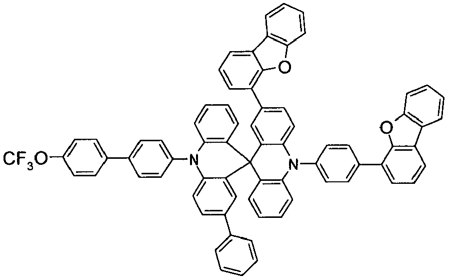 |
| 43 | 44 |
| 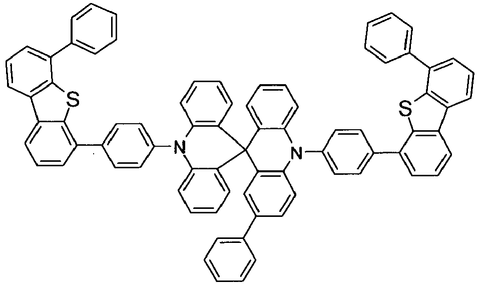 | 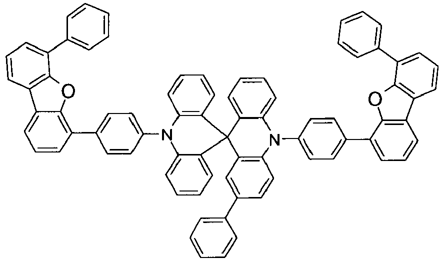 |
| 45 | 46 |
| 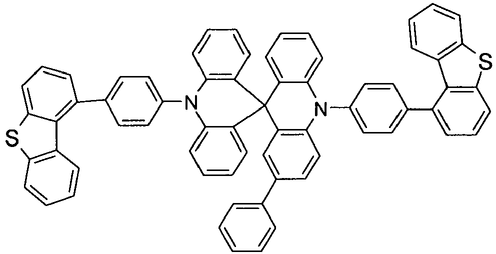 | 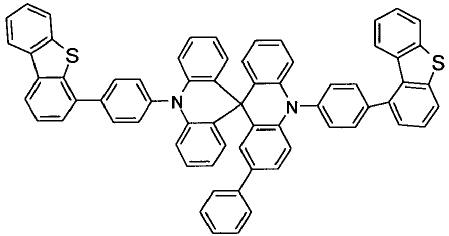 |
| 47 | 48 |
| 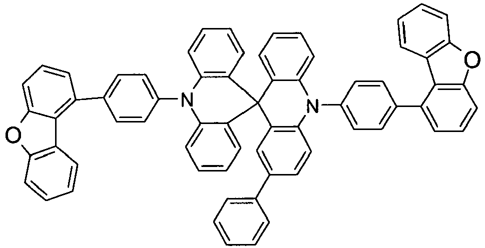 | 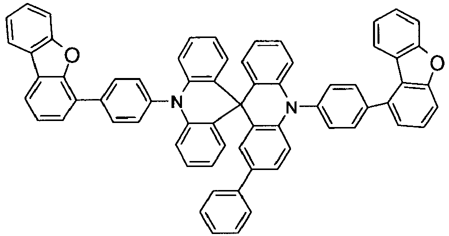 |
| 49 | 50 |
| 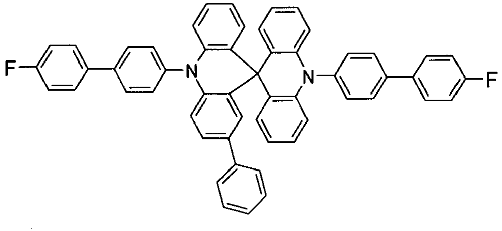 | 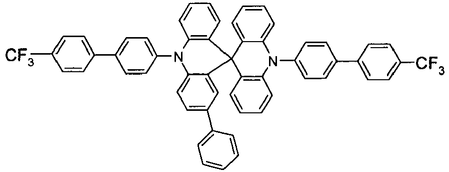 |
| 51 | 52 |
| 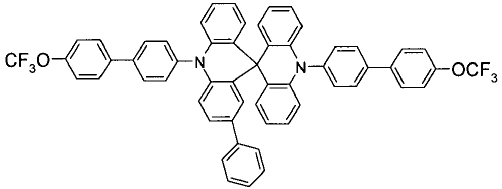 | 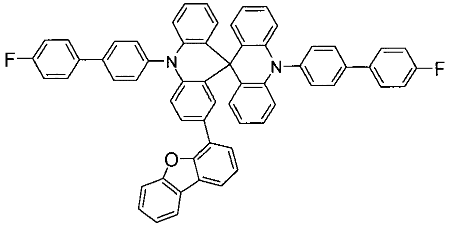 |
| 53 | 54 |
| 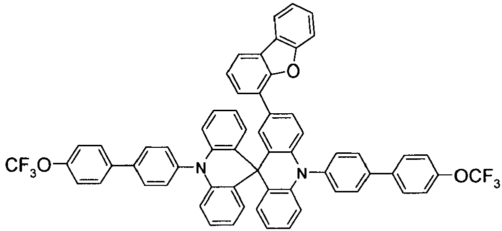 | 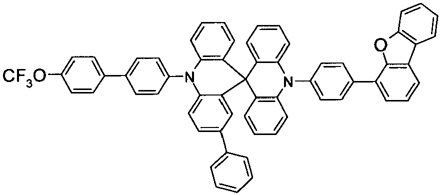 |
| 55 | 56 |
| 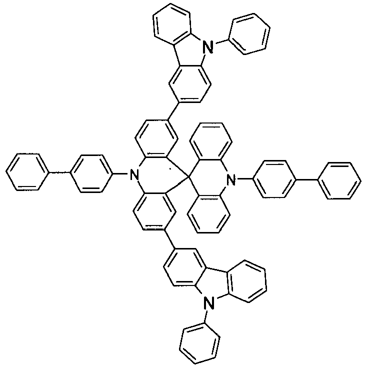 | 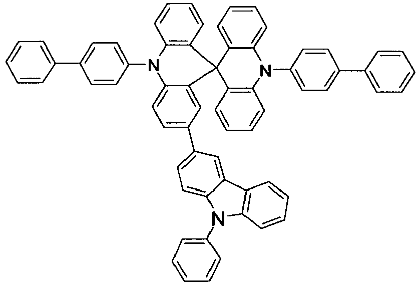 |
| 57 | 58 |
| 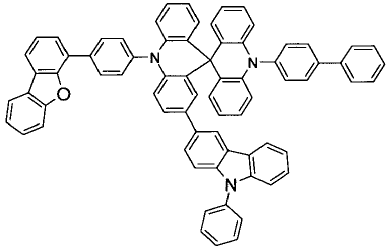 | 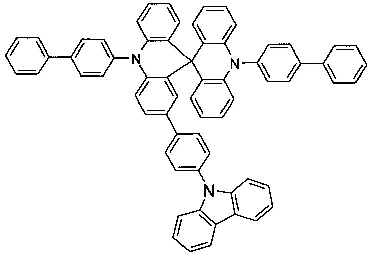 |
| 59 | 60 |
| 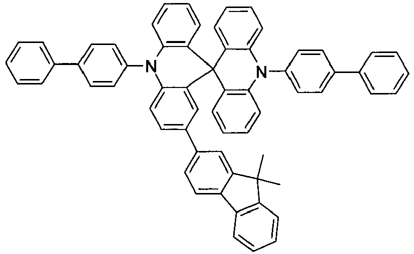 | 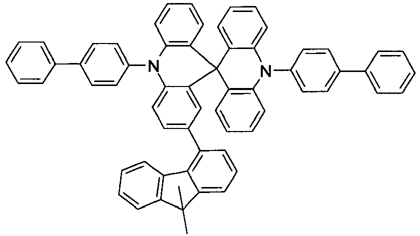 |
| 61 | 62 |
| 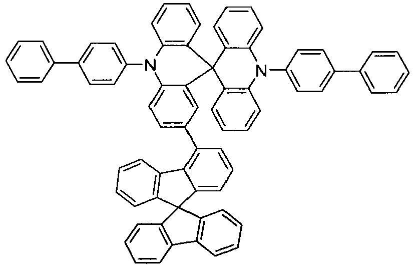 | 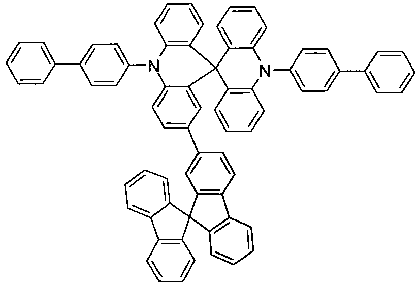 |
| 63 | 64 |
| 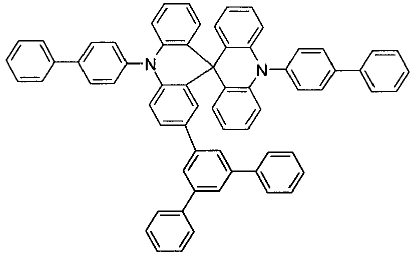 | 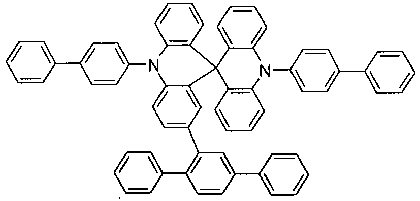 |
| 65 | 66 |
| 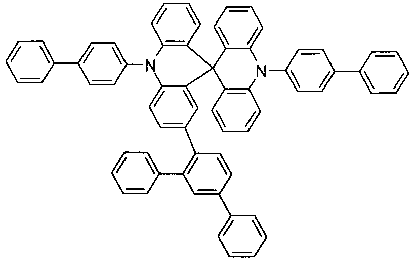 | 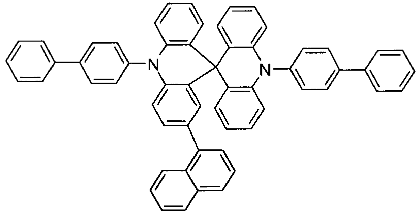 |
| 67 | 68 |
| 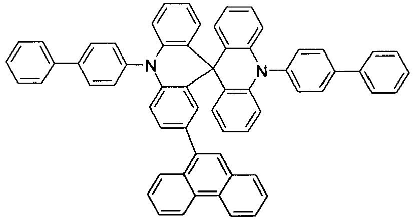 | 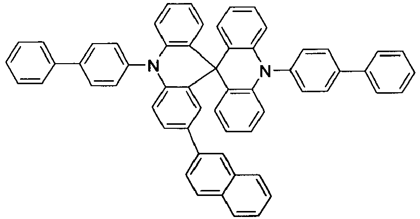 |
| 69 | 70 |
| 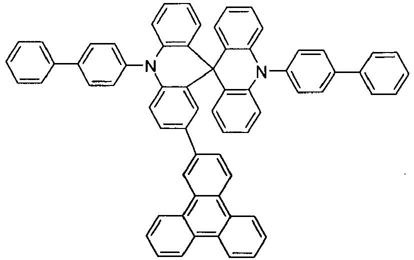 | 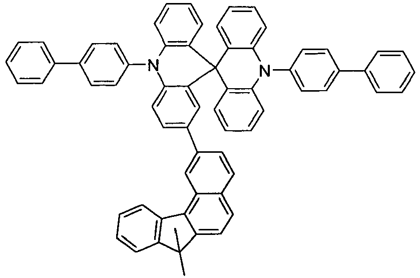 |
| 71 | 72 |
| 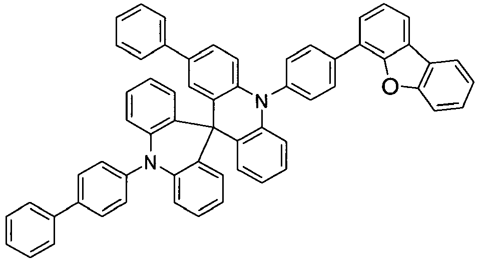 | 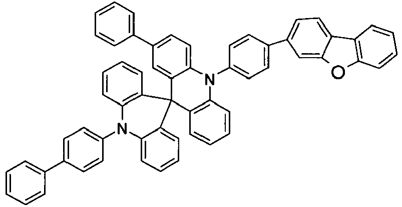 |
| 73 | 74 |
| 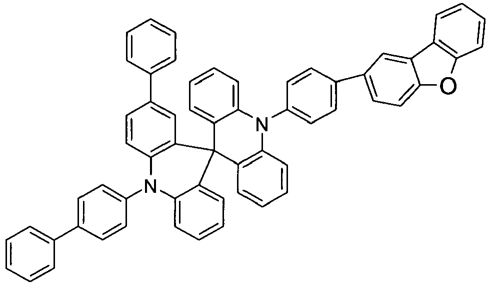 | 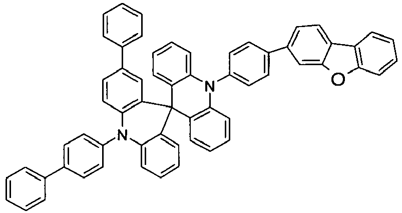 |
| 75 | 76 |
| 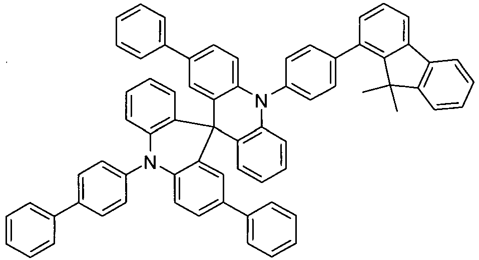 | 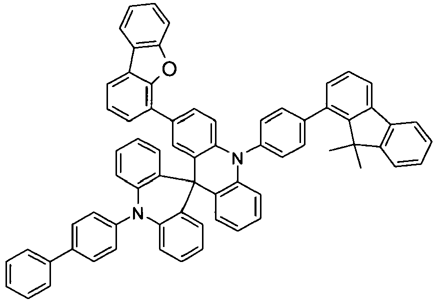 |
| 77 | 78 |
| 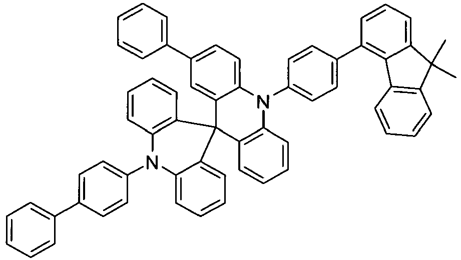 | 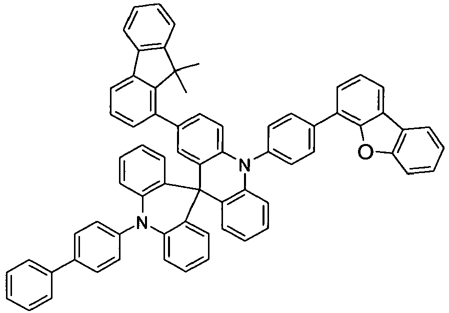 |
| 79 | 80 |
| 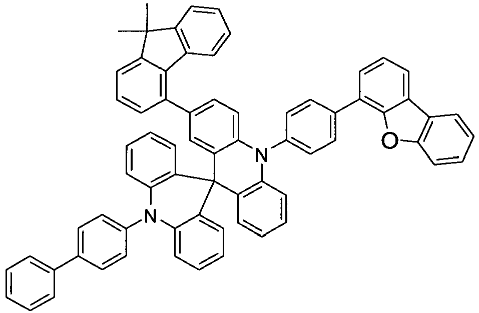 | 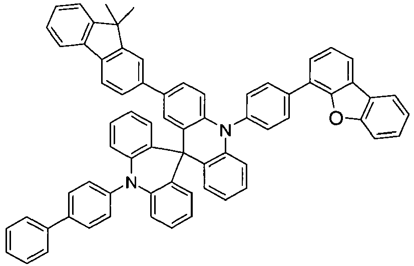 |
| 81 | 82 |

Die Verbindungen gemäß Formel (I) können unter Verwendung bekannter Reaktionen der organischen Chemie hergestellt werden, beispielsweise unter Verwendung von Bromierungsreaktionen, Buchwald-Kupplungsreaktionen und Suzuki-Kupplungsreaktionen.

Bevorzugte Verfahren zur Herstellung der Verbindungen gemäß Formel (I) werden im Folgenden vorgestellt und allgemein erläutert. In den Schemata können die gezeigten Verbindungen wahlweise substituiert sein. Explizite Beispiele für die genannten Verfahren werden in den Ausführungsbeispielen gezeigt.

Ein bevorzugtes Verfahren zur Herstellung von Verbindungen gemäß Formel (I) beginnt mit Acridinon oder einem Derivat von Acridinon (Schema 1). Dieses wird in einer Buchwald-Kupplung umgesetzt, wobei ein mit einer Arylgruppe am N substituiertes Acridinon erhalten wird. Dieses wird in einem weiteren Schritt bromiert, und es werden in einer Suzuki-Reaktion an den bromierten Positionen Arylgruppen eingeführt. Auf diesem Weg werden bevorzugt symmetrische Acridinon-Zwischenstufen erhalten.

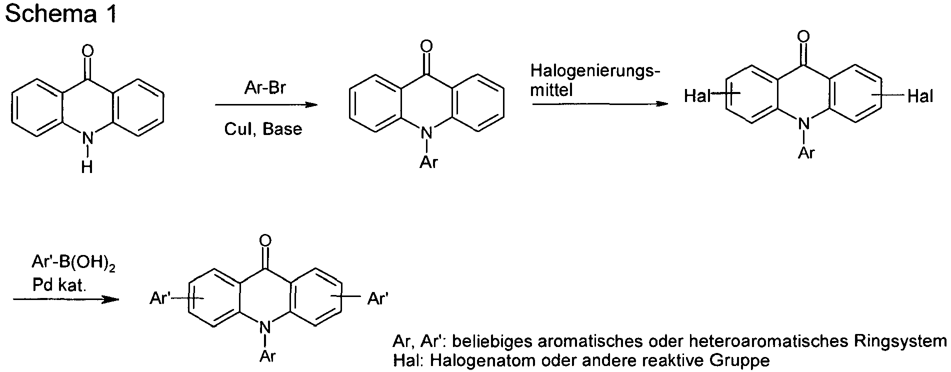

Asymmetrische Acridinon-Grundkörper können gemäß Schema 2 hergestellt werden. Hierzu wird von einem Diphenylamin-Derivat ausgegangen, das zu einem Carbonsäure-substituierten Triphenylderivat umgesetzt wird. Dieses kann in einer Ringschlussreaktion zu einem asymmetrisch substituierten Acridinon-Derivat umgesetzt werden.

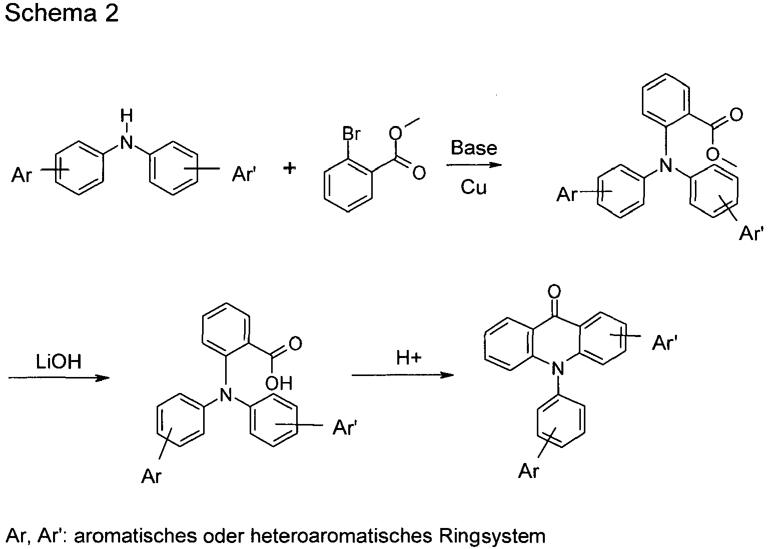

Die symmetrisch substituierten oder asymmetrisch substituierten Acridinon-Derivate werden mit entsprechend substituierten Diphenylaminen zu Spiro-Bisacridinen umgesetzt (Schema 3). An die Reaktion, die zur Bildung der Spiro-Einheit führt, können sich Suzuki-Kupplungsreaktionen zur Einführung von Substituenten an den aromatischen Ringen und/oder Buchwald-Kupplungsreaktionen zur Einführung von Substituenten am Stickstoffatom des Spiro-Bisacridins anschließen.

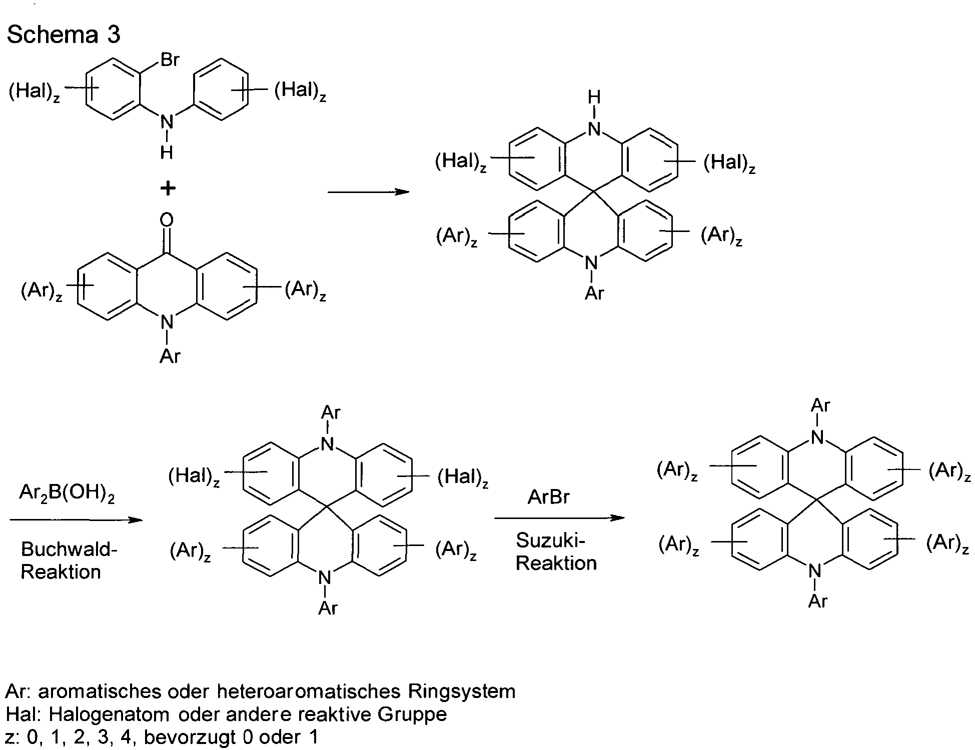

Die beschriebenen Syntheseverfahren sind lediglich bevorzugte Verfahren. Es sind alternative Verfahren möglich, auf die der Fachmann im Rahmen seines allgemeinen Fachwissens zurückgreifen kann, sofern dies nötig ist.

Gegenstand der vorliegenden Erfindung ist ein Verfahren zur Herstellung von Verbindungen der Formel (I), dadurch gekennzeichnet, dass es die folgenden Schritte umfasst:
1) Umsetzung eines halogensubstituierten Diphenylamin-Derivats mit einem Acridinon-Derivat in Gegenwart eines Metallorganyls;
2) Ringschlussreaktion der in 1) entstandenen Zwischenstufe zu einem Spiro-Bisacridin-Derivat;
3) Einführung einer Aryl- oder Heteroarylgruppe mittels Buchwald-Kupplung an einem freien Stickstoffatom des Spiro-Bisacridin-Derivats.

Bevorzugt findet Schritt 2) nach Schritt 1) statt, und Schritt 3) findet nach Schritt 2) statt. Weiterhin bevorzugt schließen sich Derivatisierungs- und Kupplungsreaktionen an Schritt 3) an, beispielsweise Suzuki-Kupplungen. Nochmals weiterhin ist es bevorzugt, dass das Metallorganyl in Schritt 1) eine lithiumorganische Base ist, besonders bevorzugt n-Butyllithium. Weiterhin bevorzugt ist es, dass in Schritt 1) eine nukleophile Additionsreaktion an die Carbonylgruppe stattfindet, bei der ein tertiärer Alkohol entsteht.

Die oben beschriebenen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere, enthaltend eine oder mehrere Verbindungen gemäß Formel (I), wobei die Bindung(en) zum Polymer, Oligomer oder Dendrimer an beliebigen, in Formel (I) mit R¹ oder R² substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) oder mindestens ein Polymer, Oligomer oder Dendrimer enthaltend mindestens eine Einheit gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung der Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist, wie bereits oben ausgeführt, eine elektronische Vorrichtung, enthaltend mindestens eine Verbindung gemäß Formel (I). Dabei ist die elektronische Vorrichtung bevorzugt ausgewählt aus den oben genannten Vorrichtungen.

Besonders bevorzugt ist sie eine organische Elektrolumineszenzvorrichtung (OLED), enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, *Multiphoton Organic EL Device Having Charge Generation Layer*) und/oder organischen oder anorganischen p/n-Übergängen.

Die Abfolge der Schichten der organischen Elektrolumineszenzvorrichtung enthaltend die Verbindung der Formel (I) ist bevorzugt die folgende: Anode-Lochinjektionsschicht-Lochtransportschicht-wahlweise weitere Lochtransportschicht-wahlweise Elektronenblockierschicht-emittierende Schicht-Elektronentransportschicht-Elektroneninjektionsschicht-Kathode. Es müssen jedoch nicht alle der genannten Schichten vorhanden sein, und es können zusätzlich weitere Schichten vorhanden sein.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Die erfindungsgemäßen Verbindungen sind dabei bevorzugt in der Lochtransportschicht, Lochinjektionsschicht oder der Elektronenblockierschicht vorhanden.

Es ist erfindungsgemäß bevorzugt, wenn die Verbindung gemäß Formel (I) in einer elektronischen Vorrichtung enthaltend einen oder mehrere phosphoreszierende emittierende Verbindungen eingesetzt wird. Dabei kann die Verbindung in unterschiedlichen Schichten, bevorzugt in einer Lochtransportschicht, einer Elektronenblockierschicht, einer Lochinjektionsschicht oder in einer emittierenden Schicht, enthalten sein.

Vom Begriff phosphoreszierende emittierende Verbindungen sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende emittierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platin- oder Kupferkomplexe als phosphoreszierende emittierende Verbindungen angesehen.

Beispiele für die oben beschriebenen emittierenden Verbindungen können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I) in organischen Elektrolumineszenzvorrichtungen einsetzen. Weitere Beispiele sind in einer folgenden Tabelle aufgeführt.

Die Verbindung gemäß Formel (I) kann aber auch erfindungsgemäß in einer elektronischen Vorrichtung enthaltend eine oder mehrere fluoreszierende emittierende Verbindungen eingesetzt werden.

In einer bevorzugten Ausführungsform der Erfindung werden die Verbindungen gemäß Formel (I) als Lochtransportmaterial eingesetzt. Die Verbindungen liegen dann bevorzugt in einer Lochtransportschicht, einer Elektronenblockierschicht oder einer Lochinjektionsschicht vor.

Eine Lochtransportschicht gemäß der vorliegenden Anmeldung ist eine Schicht mit lochtransportierender Funktion, welche sich zwischen Anode und emittierender Schicht befindet.

Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt.

Wird die Verbindung gemäß Formel (I) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 und WO 2012/095143 offenbarten Verbindungen.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇ MoO₃, WO₃ und ReO₃.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden.

Bevorzugt sind als p-Dotanden inbesondere die folgenden Verbindungen:

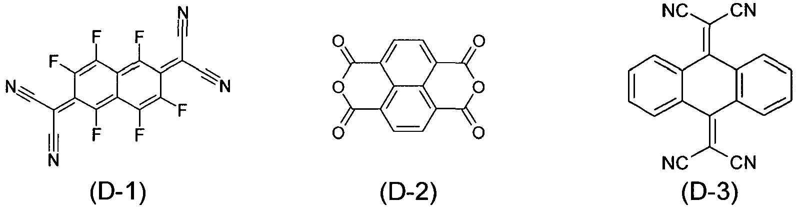

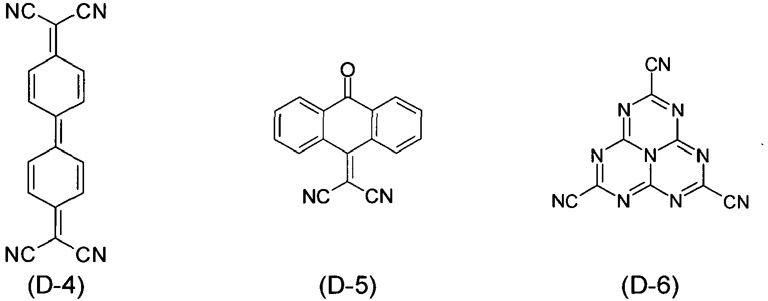

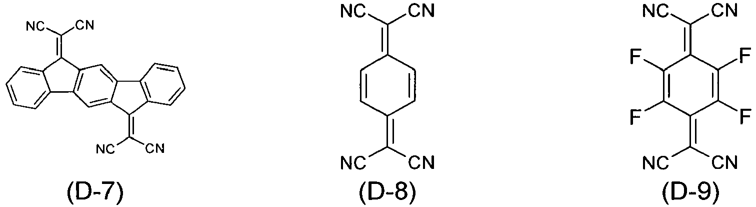

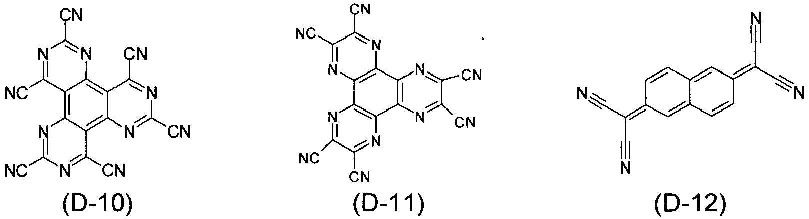

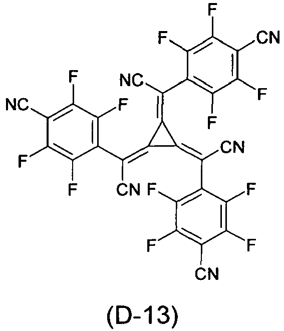

In einer weiteren bevorzugten Ausführungsform der Erfindung wird die Verbindung gemäß Formel (I) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

In einer weiteren Ausführungsform der vorliegenden Erfindung wird die Verbindung der Formel (I) in einer emittierenden Schicht als Matrixmaterial in Kombination mit einer oder mehreren emittierenden Verbindungen, vorzugsweise phosphoreszierenden emittierenden Verbindungen, eingesetzt.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil der emittierenden Verbindung zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere emittierende Verbindungen enthalten. Auch in diesem Fall sind die emittierenden Verbindungen im Allgemeinen diejenigen Verbindungen, deren Anteil im System der kleinere ist und die Matrixmaterialien sind diejenigen Verbindungen, deren Anteil im System der größere ist. In Einzelfällen kann jedoch der Anteil eines einzelnen Matrixmaterials im System kleiner sein als der Anteil einer einzelnen emittierenden Verbindung.

Es ist bevorzugt, dass die Verbindungen gemäß Formel (I) als eine Komponente von Mixed-Matrix-Systemen verwendet werden. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die Verbindung der Formel (I) stellt dabei bevorzugt das Matrixmaterial mit lochtransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere emittierende Verbindungen umfassen, bevorzugt eine oder mehrere phosphoreszierende emittierende Verbindungen. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende emittierende Verbindungen oder den bevorzugten Matrixmaterialien für fluoreszierende emittierende Verbindungen, je nachdem welche Art von emittierender Verbindung im mixed-Matrix-System eingesetzt wird.

Bevorzugte phosphoreszierende emittierende Verbindungen zur Verwendung in Mixed-Matrix-Systemen sind in einer folgenden Tabelle aufgeführt.

Im Folgenden werden bevorzugte Ausführungsformen für die verschiedenen Funktionsmaterialien der elektronischen Vorrichtung aufgeführt.

Bevorzugte phosphoreszierende emittierende Verbindungen sind die oben genannten Verbindungen und die in der folgenden Tabelle gezeigten Verbindungen:

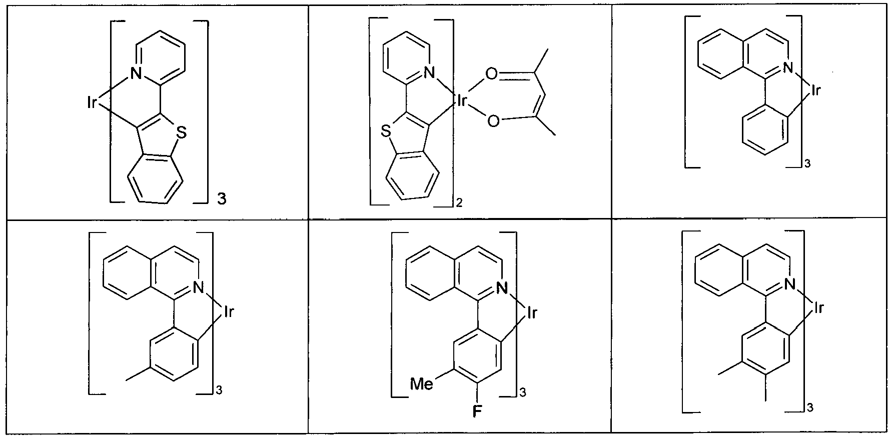

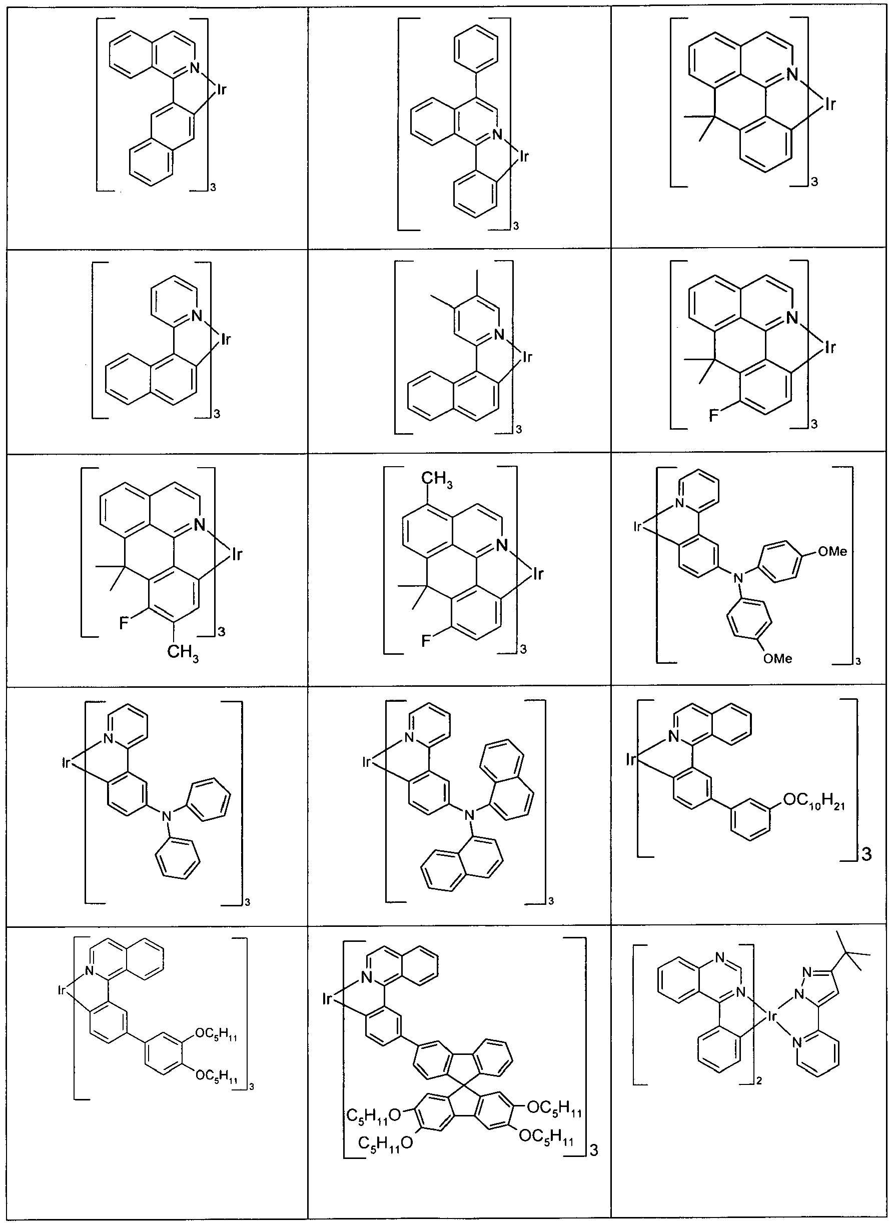

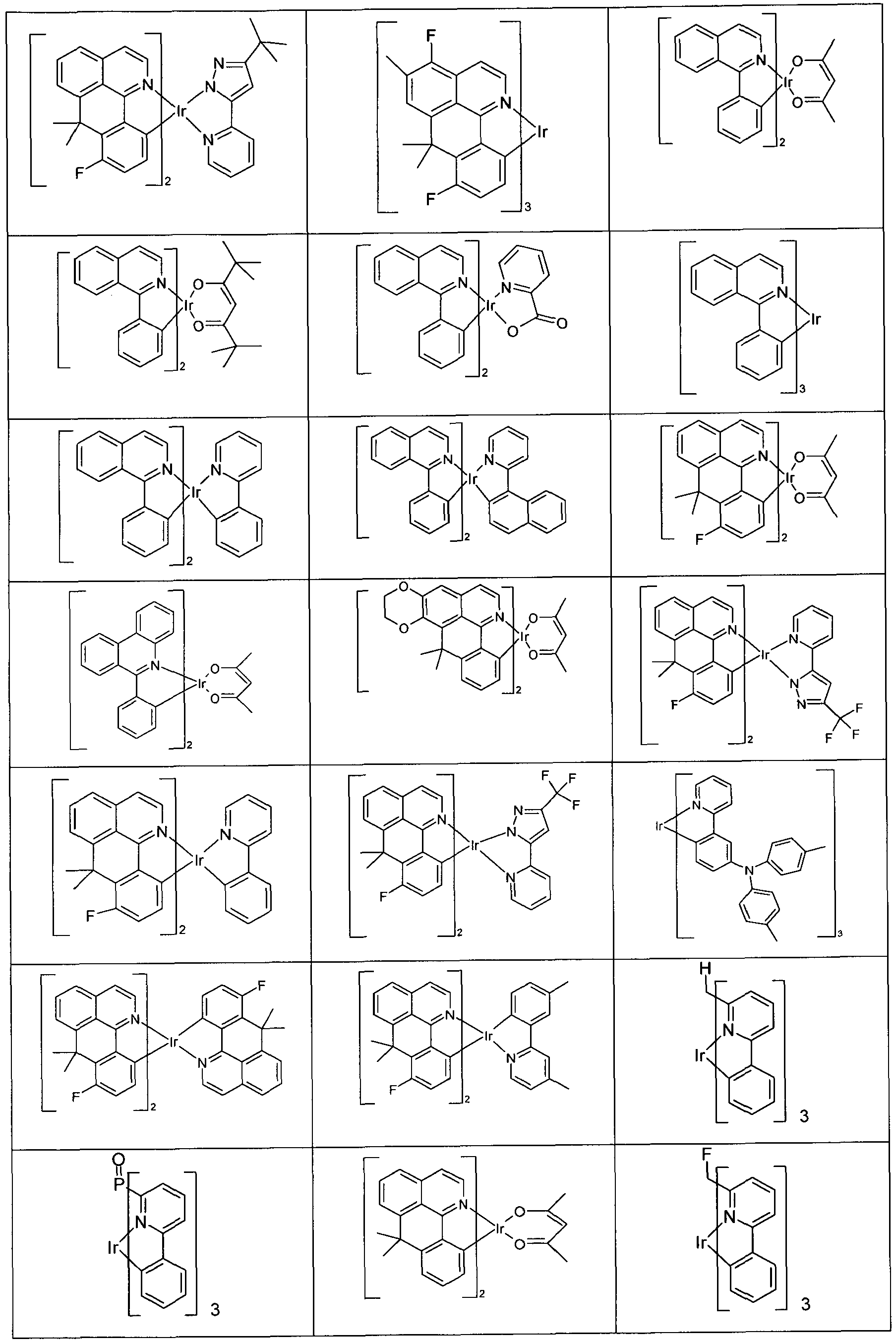

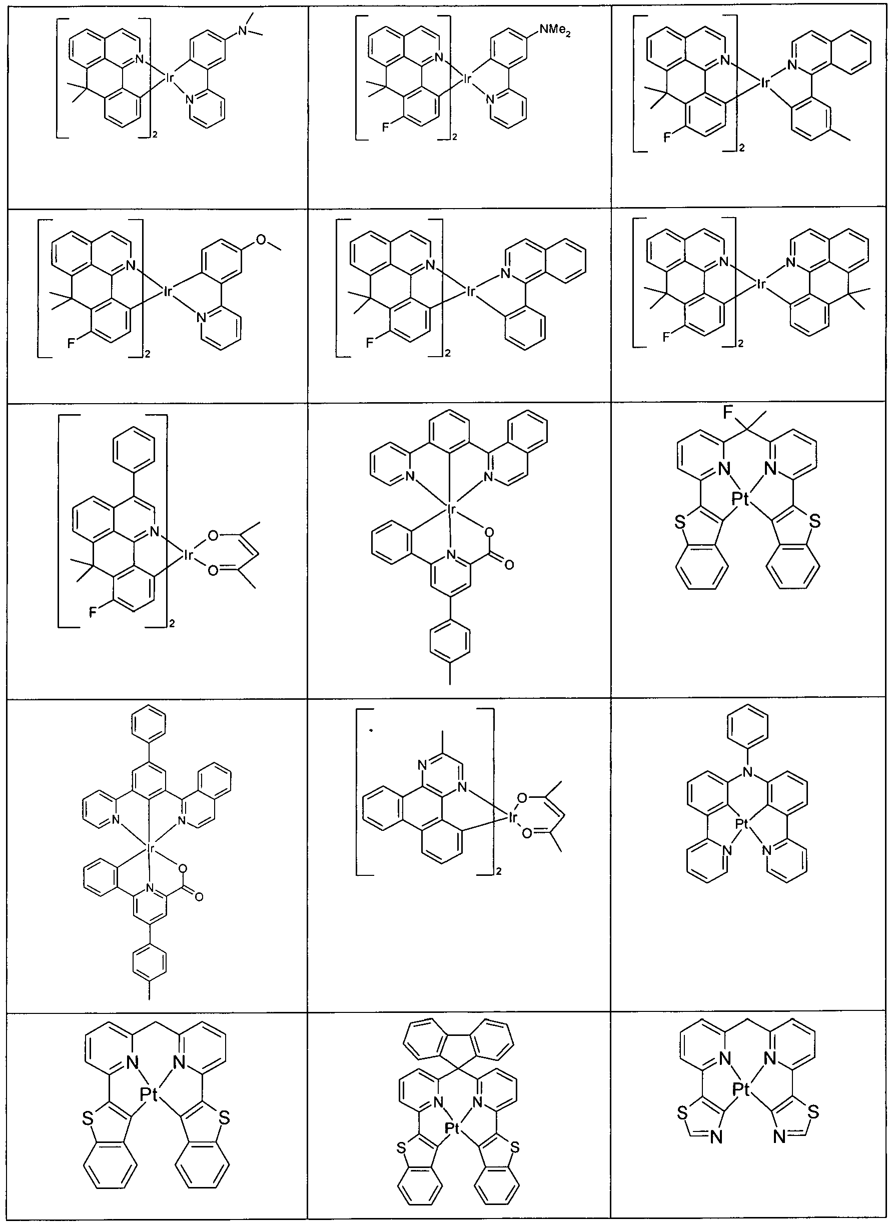

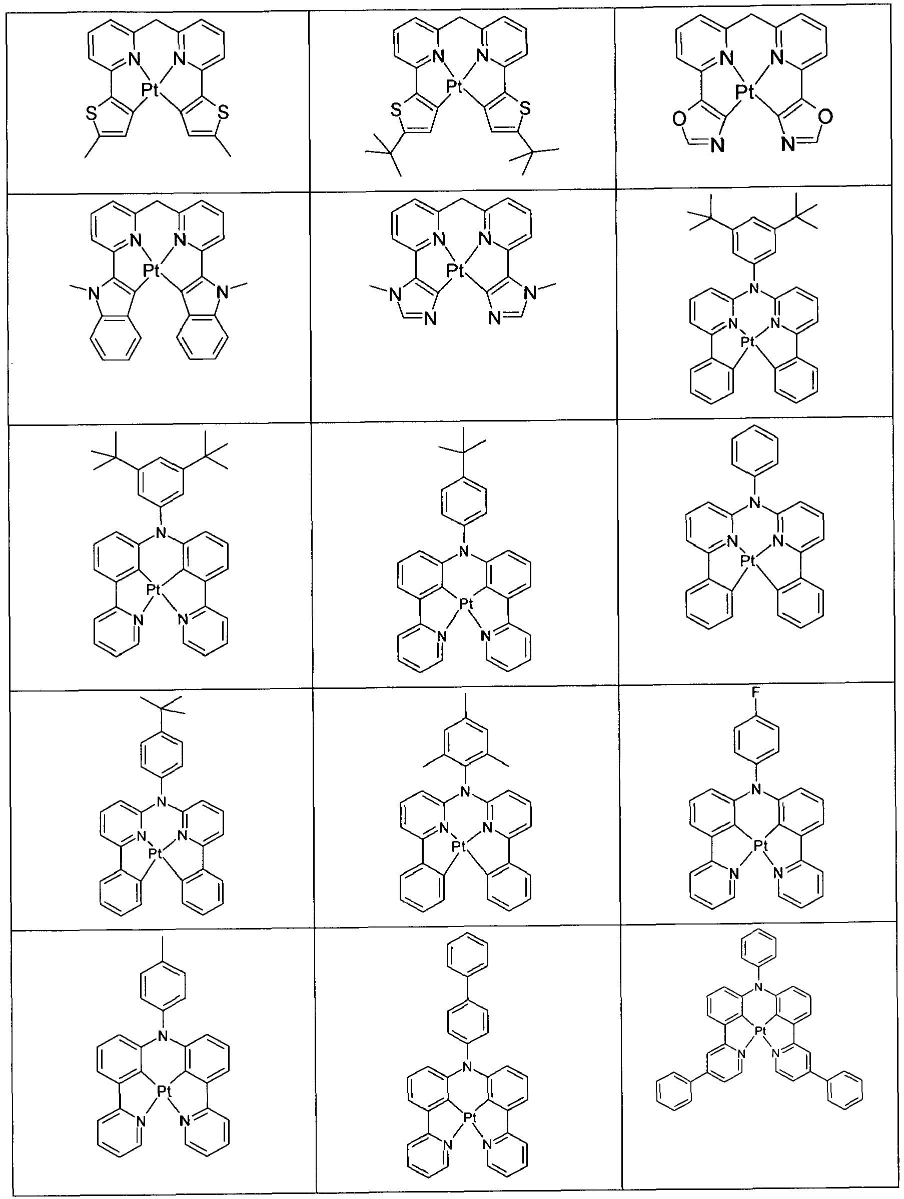

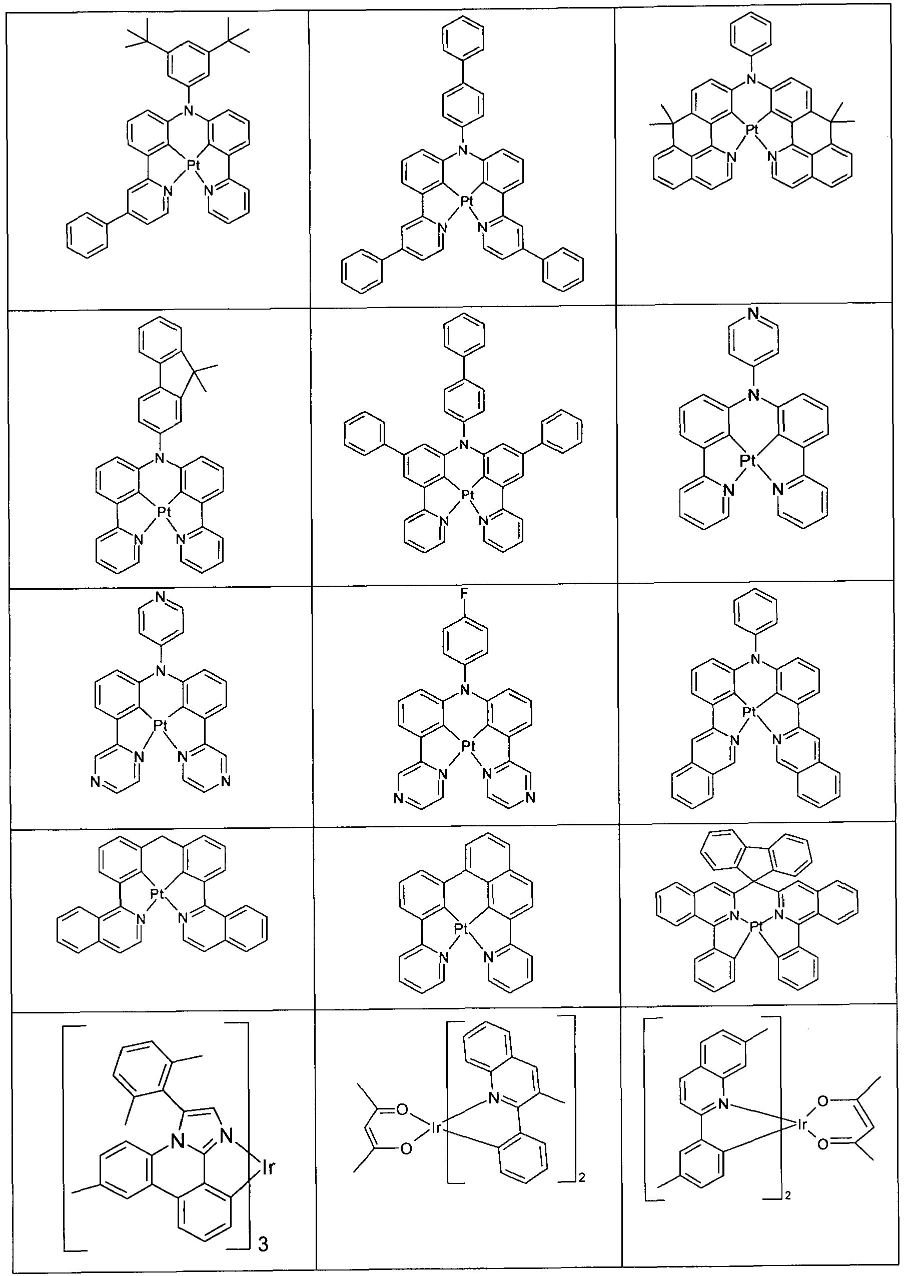

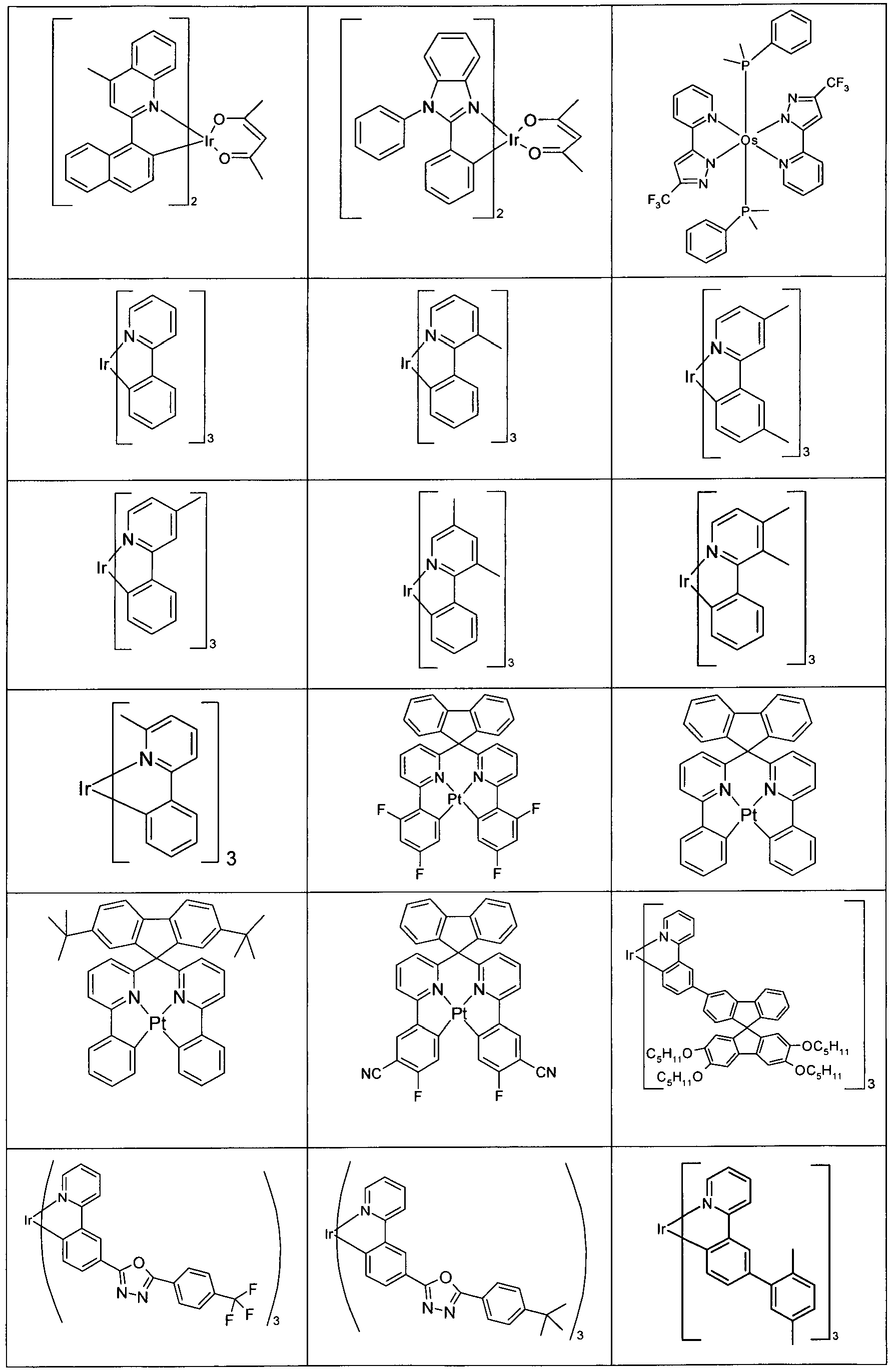

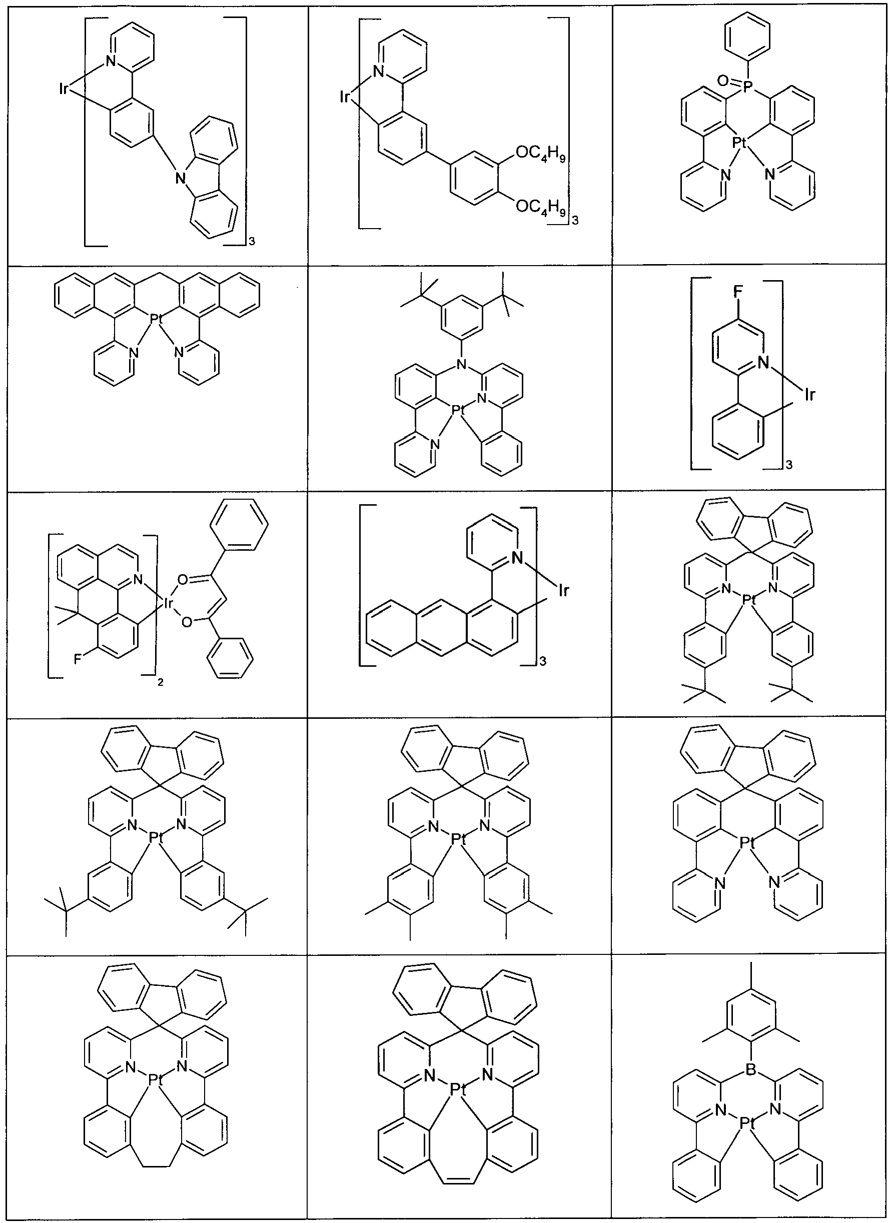

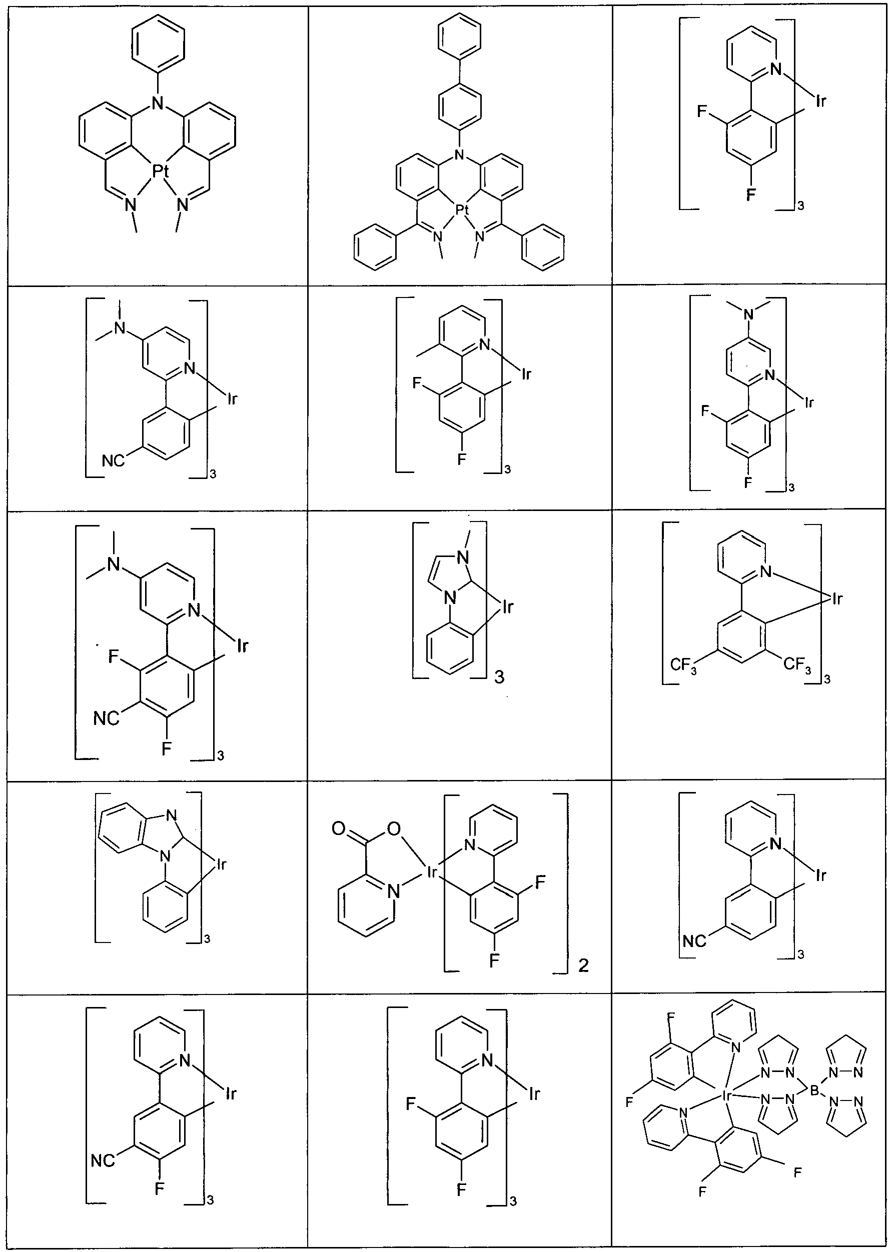

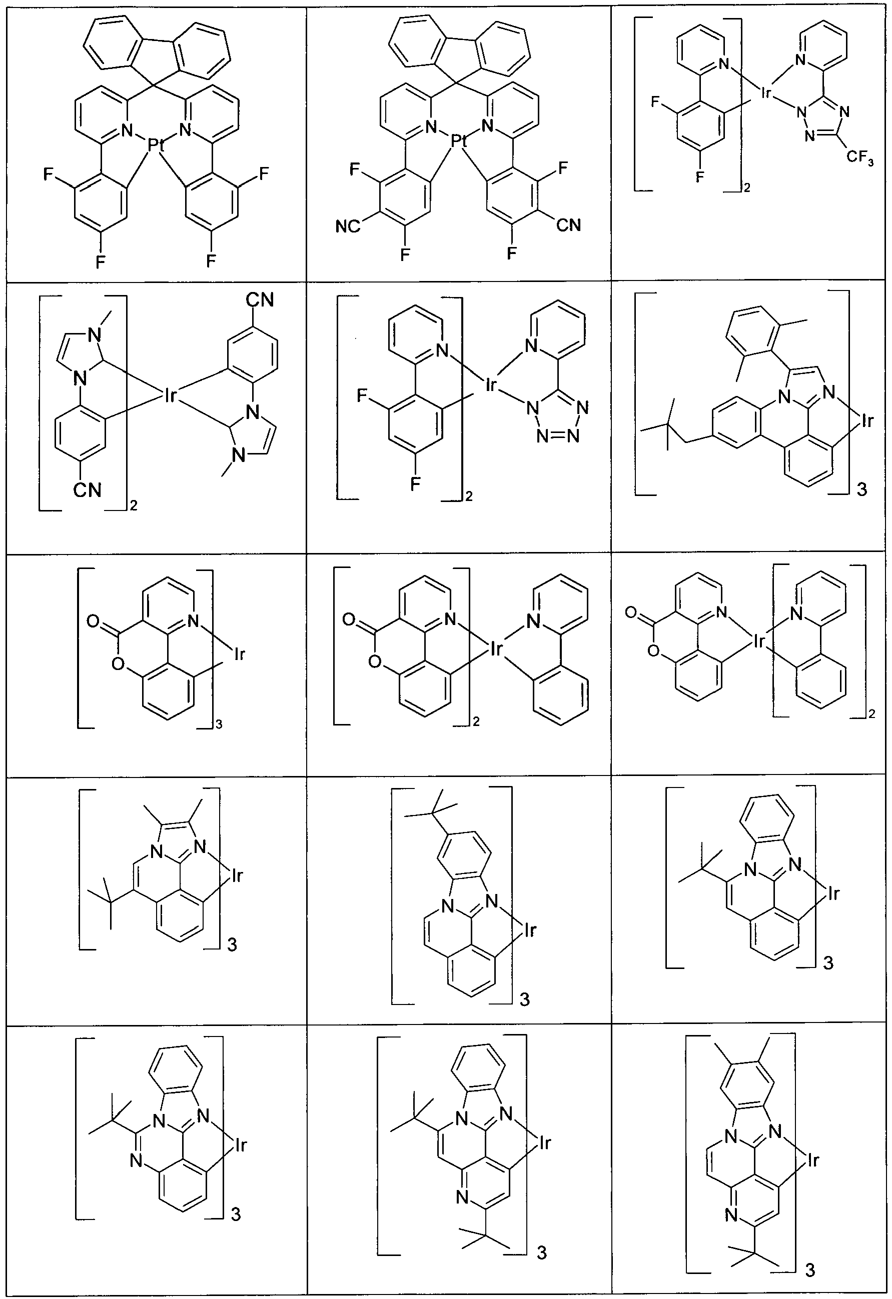

Bevorzugte fluoreszierende emittierende Verbindungen sind neben den Verbindungen der Formel (I) ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und die in WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine und die in WO 2014/111269 offenbarten erweiterten Benzoindenofluorene.

Als Matrixmaterialien, bevorzugt für fluoreszierende emittierende Verbindungen, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, sowie die in EP 1749809, EP 1905754 und US 2012/0187826 offenbarten Pyren-Verbindungen.

Bevorzugte Matrixmaterialien für phosphoreszierende emittierende Verbindungen sind neben den Verbindungen der Formel (I) aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol-bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den Verbindungen der Formel (I) beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Bevorzugt umfasst die erfindungsgemäße OLED zwei oder mehr unterschiedliche lochtransportierende Schichten. Die Verbindung der Formel (I) kann dabei in einer oder in mehreren oder in allen lochtransportierende Schichten eingesetzt werden. Gemäß einer bevorzugten Ausführungsform wird die Verbindung der Formel (I) in genau einer lochtransportierenden Schicht eingesetzt, und in den weiteren vorhandenen loch transportierenden Schichten werden andere Verbindungen eingesetzt, bevorzugt aromatische Aminverbindungen.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, AI, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder AI, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, C_{S2}CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

### A) Synthesebeispiele

### A-1) Synthese von symmetrischen Grundkörpern

### Schritt 1:

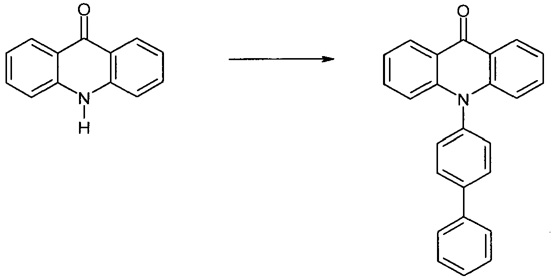

100 g (0,5 mol) 10H-Acridin-9-on, 140 g (0,6 mol) 4-Brom-Biphenyl, 9,6 g (0,05 mol) Cul, 104,0 g (0,75 mol) Kaliumcarbonat und 22,0 mL (0,1 mol) 2,2,6,6-Tetramethyl-heptan-3,5-dion werden in 600 mL Dimethylformamid unter Schutzatmosphäre gelöst. Die Reaktionsmischung wird 48 h unter Schutzatmosphäre zum Sieden erhitzt. Zu dem Gemisch wird im Anschluss Wasser zugegeben. Der Feststoff wird abgesaugt, mit Wasser und Ethanol gewaschen und aus Toluol umkristallisiert.

Ausbeute: 167 g (0,48 mol), 96% d. Th.

Analog können folgende Verbindungen erhalten werden:

| **Edukt 1** | **Edukt 2** | **Produkt** | **Ausbeute** |
|---|---|---|---|
| 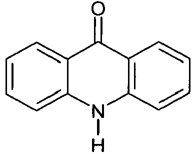 | 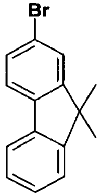 | 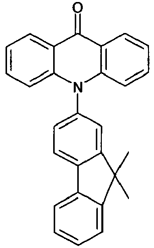 | 85% |
| 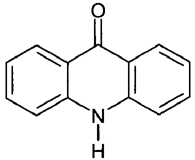 | 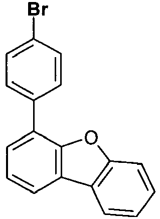 | 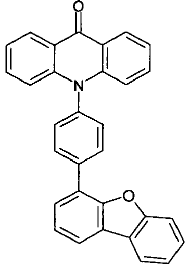 | 89 % |
| 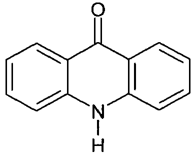 | 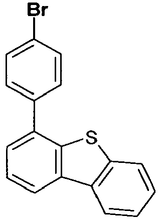 | 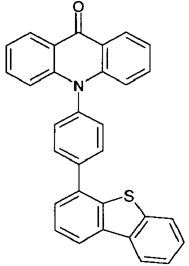 | 91% |
| 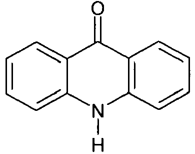 | 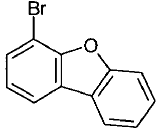 | 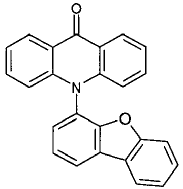 | 81% |
| 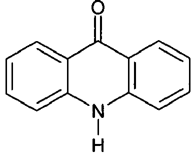 | 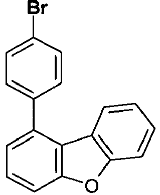 | 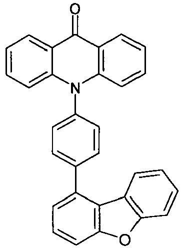 | 95% |
| 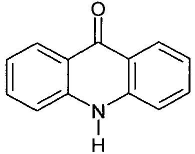 | 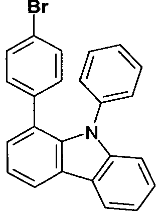 | 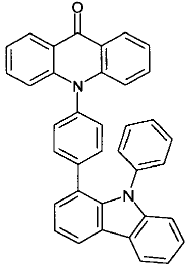 | 90% |
| 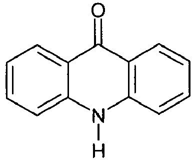 | 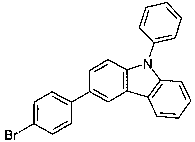 | 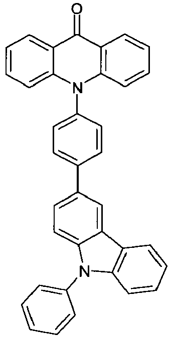 | 85% |
| 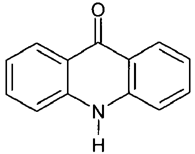 | 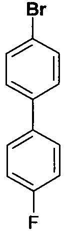 | 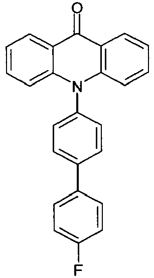 | 97% |

### Schritt 2:

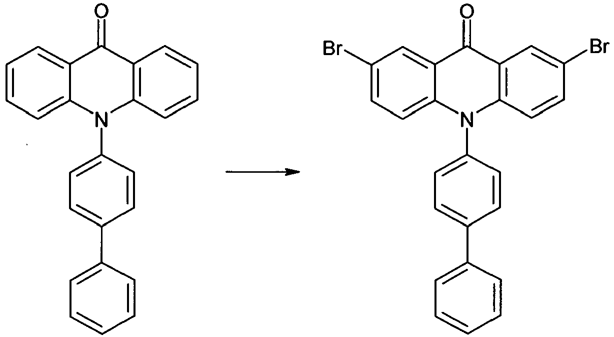

Eine Lösung des Dihydroacridons (30.0 g, 86.3 mmol) in DMF (1400 mL) wird bei 0 °C unter Lichtausschluss portionsweise mit *N*-Bromsuccinimid (31.05 g, 172.7 mmol) versetzt und über Nacht bei Raumtemperatur gerührt. Das Lösungsmittel wird zur Hälfte abrotiert und 300 mL Ethanol werden zum Gemisch zugegeben. Der Feststoff wird abgesaugt und aus DMF umkristallisiert. Ausbeute: 42 g, 97 % d. Th., gelber Feststoff.

| **Edukt 1** | **Produkt** | **Ausbeute** |
|---|---|---|
| 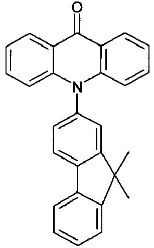 | 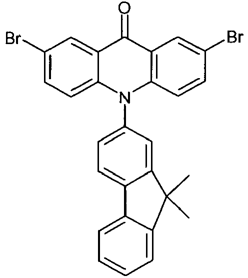 | 85% |
| 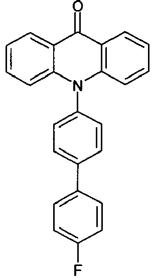 | 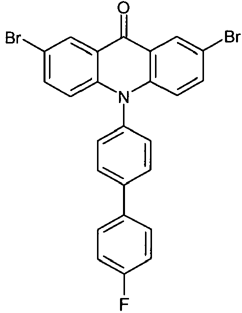 | 93 % |
| 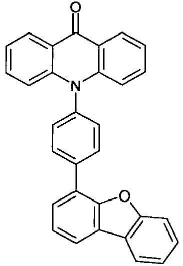 | 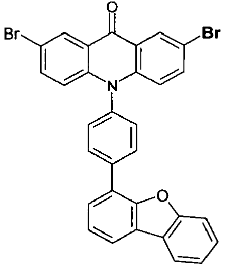 | 91% |

### Schritt 3:

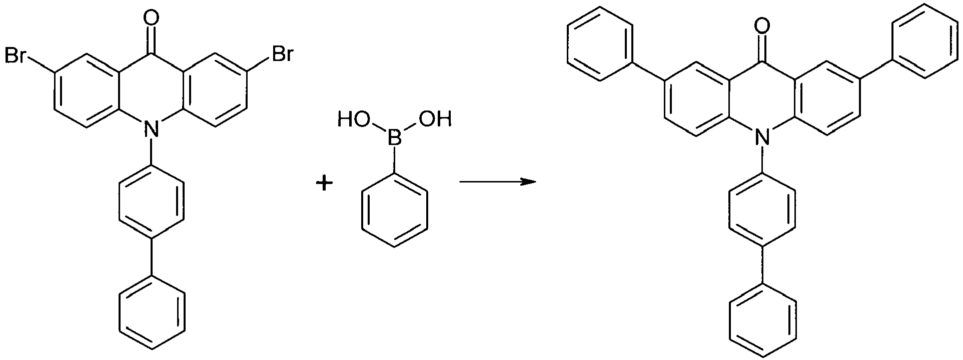

21,4 g (171,7 mmol) Phenylboronsäure, 46,3 g (85,8 mmol) Dibrom-10H-acridin-9-on und 1,4 g (1,9 mmol) Pd(PPh₃)₄ werden in 930 mL Dioxan suspendiert. Zu dieser Suspension werden 85,8 mL 2 M Kaliumcarbonat-Lösung langsam zugefügt, und die Reaktionsmischung wird 16 h unter Rückfluss erhitzt. Nach Erkalten wird die organische Phase abgetrennt, über Kieselgel filtriert und anschließend zur Trockene eingeengt. Der Rückstand wird aus Toluol/Heptan umkristallisiert.

Ausbeute: 43 g (81 mmol), 94% d. Th., Reinheit nach GC >94%.

| **Edukt 1** | **Edukt 2** | **Produkt** | **Ausbeute** |
|---|---|---|---|
| 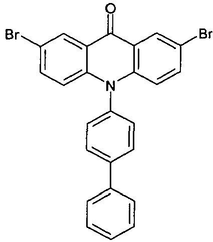 | 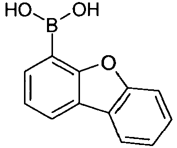 | 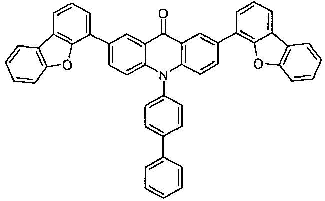 | 87% |
| 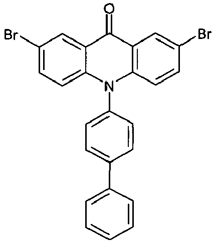 | 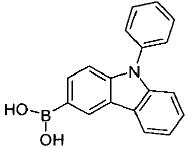 | 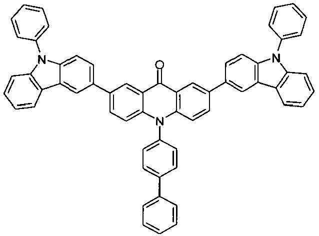 | 82% |
| 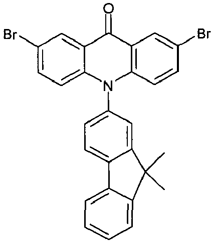 | 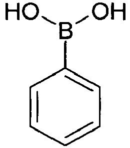 | 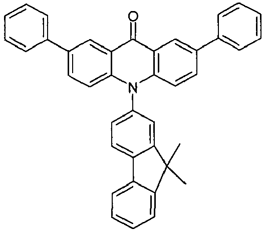 | 91% |
| 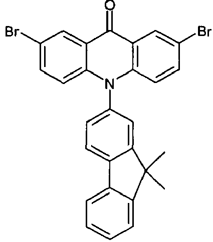 | 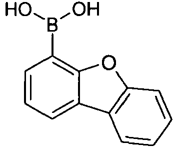 | 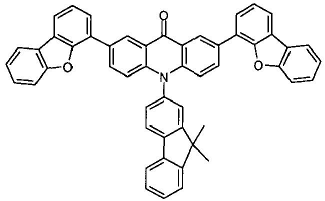 | 87% |
| 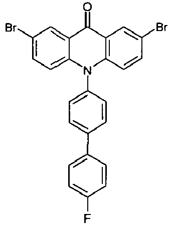 | 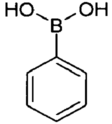 | 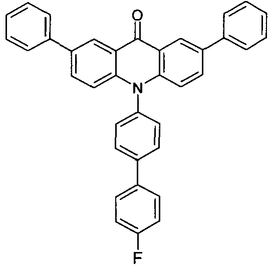 | 95% |
| 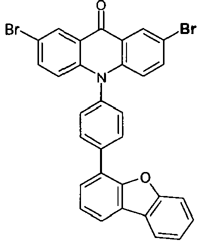 | 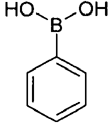 | 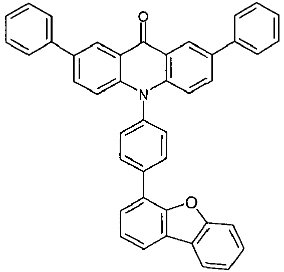 | 92% |

### A-2) Synthese von asymmetrischen Acridinonen

### Schritt 1:

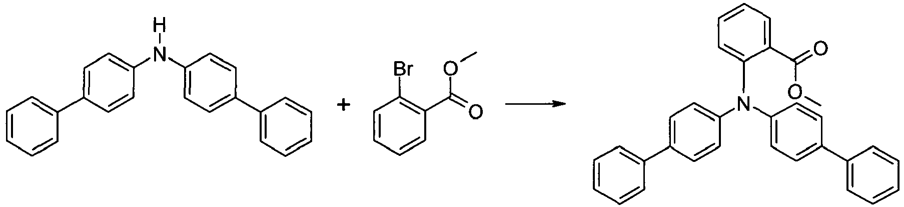

50 g (155,5 mmol) Bis-biphenyl-4-yl-amin, 66,9 g (311,1 mmol) 2-Brombenzoesäuremethylester, 21,5 g (155,5 mmol) Kaliumcarbonat, 22,1 g (155,5 mmol), Natriumsulfat und 0,9 g (15,5 mmol) Kupferpulver werden in 210 mL Nitrobenzol suspendiert. Die Reaktionsmischung wird 6 h auf 220°c erhitzt. Nach Erkalten wird über Celite filtriert und das Nitrobenzol abdestilliert. Der Rückstand wird über Kieselgel filtriert (Heptan/Dichloromethan 1:1). Das Produkt wird in Form eines Feststoffs erhalten. Die Ausbeute beträgt 64 g (91 % d. Th).

### Schritt 2:

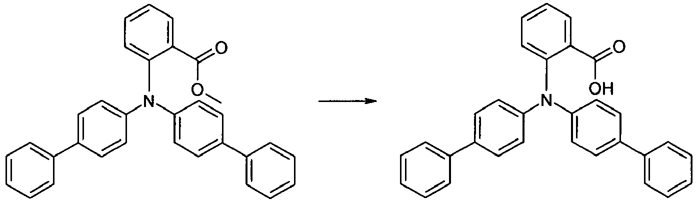

Zu einer Lösung von 62 g (136,1 mmol) Benzoesäuremethylester in 294 mL Dioxan und 294 mL Wasser werden 114,2 g (2722 mmol) LiOH*H₂O zugegeben. Die Reaktionsmischung wird 16 h auf 105°C erhitzt. Nach Erkalten wird mit Essigsäureester versetzt, auf 1500 mL 10% Zitronensäurelösung gegeben und mit Essigsäureester extrahiert. Die vereinigten organischen Phasen werden getrocknet und im Vakuum eingeengt. Der Rückstand wird ohne weitere Aufreinigung im nächsten Schritt verwendet.

### Schritt 3:

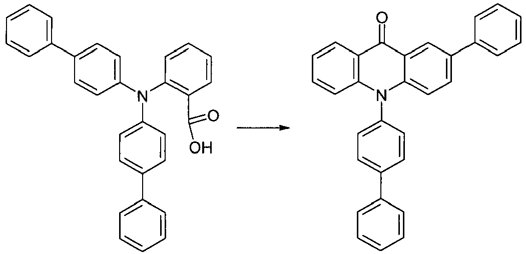

62 g (140 mmol) Benzoesäure werden in 364 mL Methansulfonsäure gelöst und über Nacht bei 60°C gerührt. Nach Erkalten wird das Gemisch langsam auf Eis/Wasser gegeben und der ausgefallene Feststoff wird abgesaugt. Der Feststoff wird in Essigsäureester gelöst und mit einer 20%igen Natriumhydrogencarbonat-Lösung gewaschen. Die vereinigten organischen Phasen werden getrocknet und im Vakuum eingeengt. Der Rückstand wird aus MeOH umkristallisiert. Die Ausbeute beträgt 56 g (94 % d. Th).

### A-3) Synthese einer Diarylamino-Zwischenstufe

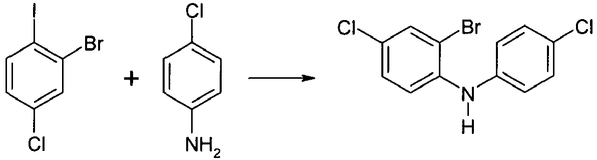

Eine Lösung von 2-Brom-4-Chlor-1-iod-benzol (50 g, 154 mmol) und 4-Chlor-phenylamin (19,7 g, 154 mmol) in entgastem Toluol (590 mL) wird mit DPE-Phos (3,3 g, 6,2 mmol), Palladiumacetat (0,7 g, 3,1 mmol) und Natrium-tert-butanolat (41,5 g, 432 mmol) versetzt und 20 h unter Rückfluss erhitzt. Das Reaktionsgemisch wird auf Raumtemperatur abgekühlt, mit Toluol erweitert und über Celite filtriert. Das Filtrat wird mit Wasser erweitert, mit Toluol extrahiert und die vereinigten organischen Phasen getrocknet und im Vakuum eingeengt. Der Rückstand wird über Kieselgel filtriert (Heptan). Das Produkt wird in Form eines hellroten Feststoffs erhalten. Die Ausbeute beträgt 31 g (63 % d. Th).

### A-4) Bildung der Spiro-Einheit

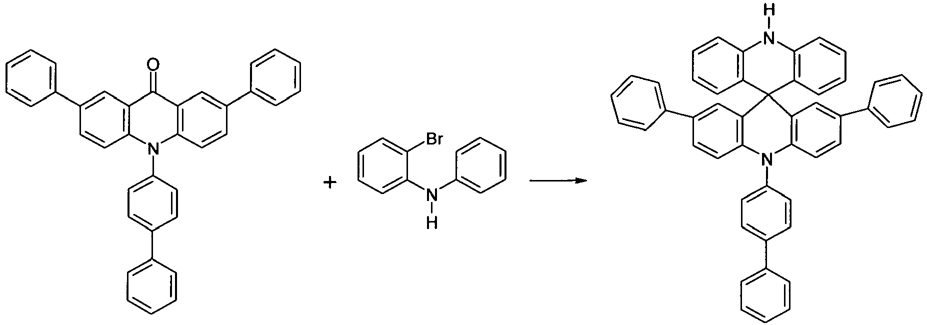

34,7 g (140 mmol) 2-Bromodiphenylamin werden in 350 ml absolutem THF vorgelegt, auf -78°C gekühlt und mit 112 ml (280 mmol) 2,5 M n-BuLi in THF versetzt. Anschließend wird auf -10°C aufgetaut und für 1h bei dieser Temperatur weitergerührt. 30 g (86 mmol) 10-Biphenyl-4-yl-2,7-diphenyl-10H-acridin-9-on gelöst in 600 ml THF wird langsam zugegeben. Dann wird 24 h bei Raumtemperatur nachgerührt. Man gibt 100 ml Ammoniumchloridlösung zu, rührt kurz nach, trennt die organische Phase ab und entfernt das Lösungsmittel im Vakuum. Der Rückstand wird in der Wärme bei 40 °C in 750 ml Eisessig suspendiert, die Suspension wird mit 60 ml konz. Salzsäure versetzt, und anschließend 8 h bei Raumtemperatur nachgerührt. Nach Erkalten saugt man vom ausgefallenen Feststoff ab, wäscht diesen einmal mit 100 ml Wasser, dreimal mit je 100 ml Ethanol und kristallisiert abschließend aus Heptan um. Ausbeute: 35,3 g (54 mmol), 77 % d. Th.

| **Edukt 1** | **Edukt 2** | **Produkt** | **Ausbeute** |
|---|---|---|---|
| 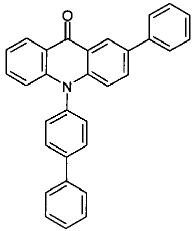 | 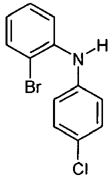 | 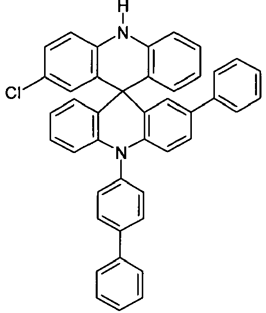 | 85% |
| 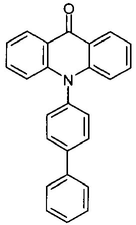 | 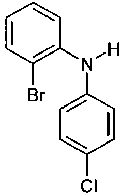 | 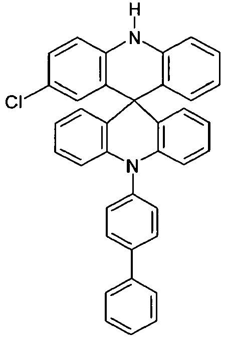 | 89 % |
| 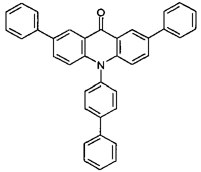 | 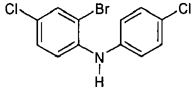 | 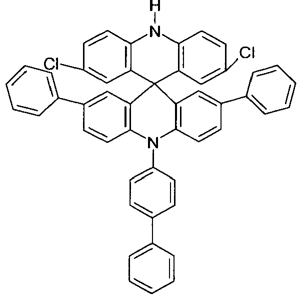 | 81% |

### A-5) Suzuki-Reaktion

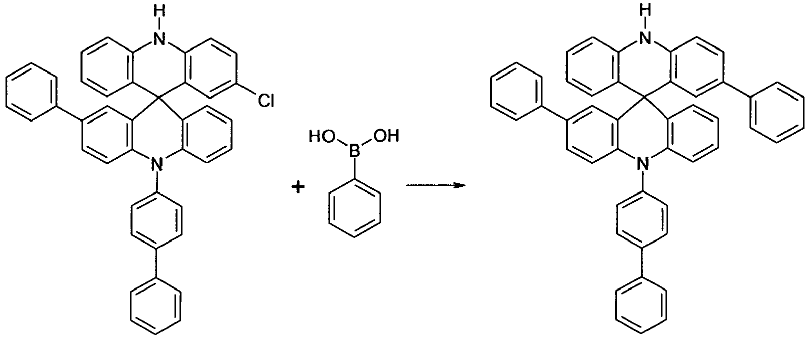

5,4 g (44,3 mmol) Benzolboronsäure, 18 g (29,5 mmol) 10-Biphenyl-4-yl-2-chlor-9,9-dimethyl-9,10-dihydro-acridin und 8,9 g (59,1 mmol) CsF werden in 250 mL Dioxan suspendiert. Zu dieser Suspension werden 1,1 g (1,5 mmol) PdCl₂(PCy₃)₂ gegeben, und die Reaktionsmischung wird 16 h unter Rückfluss erhitzt. Nach Erkalten wird über Kieselgel filtriert, dreimal mit 200 mL Wasser gewaschen und anschließend zur Trockne eingeengt. Der Rückstand wird über Kieselgel filtriert (Heptan/Essigsäureester). Das Produkt wird in Form eines Feststoffs erhalten. Die Ausbeute beträgt 19 g (98 % d. Th).

| **Bsp.** | **Edukt 1** | **Edukt 2** | **Produkt** | **Ausbeute** |
|---|---|---|---|---|
| V1 | 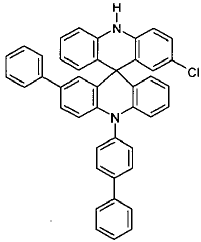 | 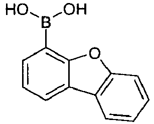 | 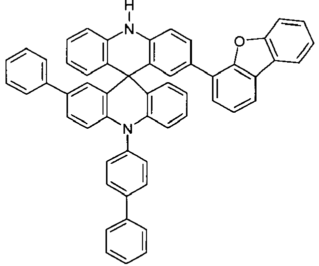 | 85% |
| 1 | 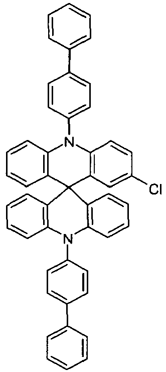 | 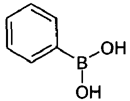 | 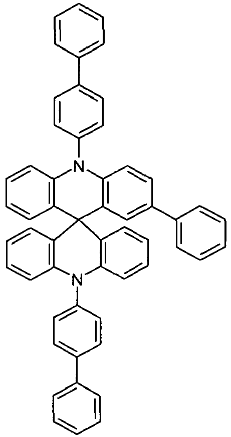 | 89 % |
| 2 | 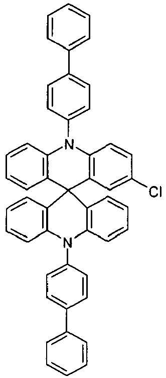 | 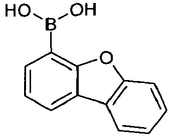 | 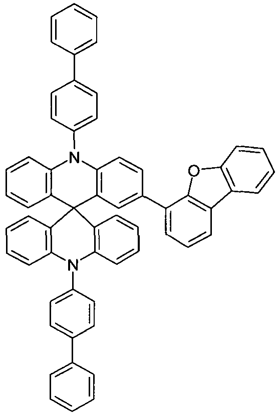 | 91% |
| V2 | 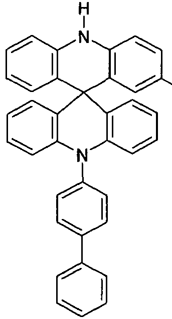 | 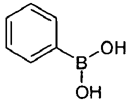 | 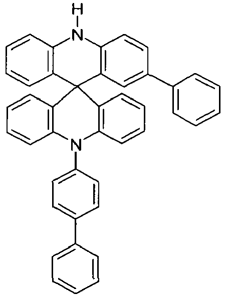 | 90% |
| V3 | 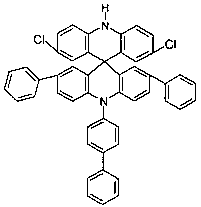 | 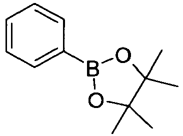 | 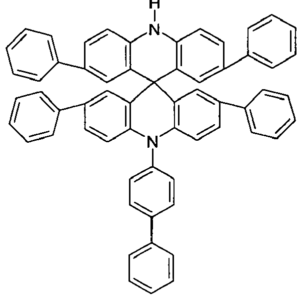 | 98% |
| 2b | 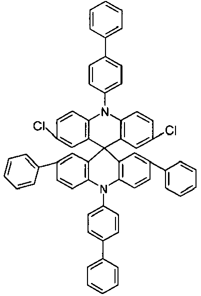 | 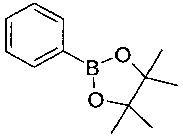 | 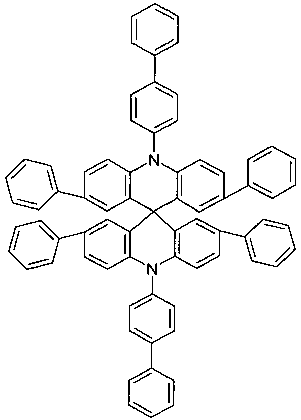 | |

### A-6) Buchwald-Reaktion

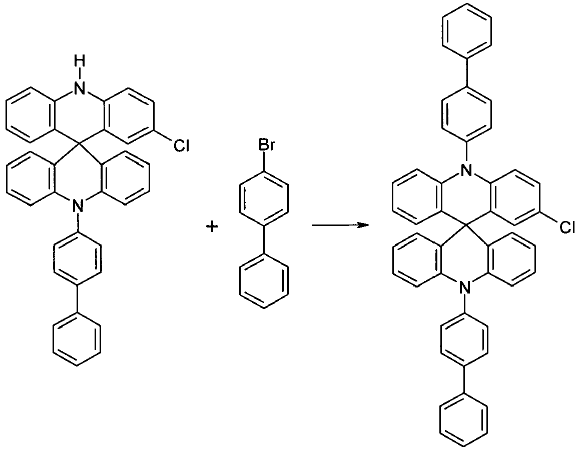

Eine entgaste Suspension von 11,1 g (46,7 mmol) 4-Brom-biphenyl, 24,9 g (46,7 mmol) des Spiro-Bisacridins in 480 mL Toluol und 11,9 g (121,3 mmol) NaOtBu wird 1 h mit N₂ gesättigt. Danach werden 1,07 g (1,9 mmol) DPPF und 1.38 g (1,9 mmol) Palladium(II)acetat zugegeben. Die Reaktionsmischung wird über Nacht unter Rückfluss erhitzt. Nach Erkalten wird die organische Phase über Kieselgel filtriert und anschließend zur Trockene eingeengt. Der Rückstand wird aus Toluol/Heptan umkristallisiert. Ausbeute: 15,7 g (47 % d. Th).

| **Bsp.** | **Edukt 1** | **Edukt 2** | **Produkt** | **Ausbeute** |
|---|---|---|---|---|
| 3 | 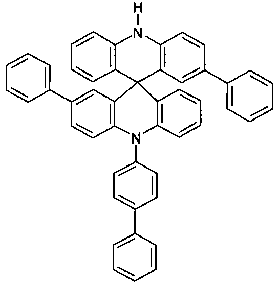 | 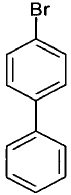 | 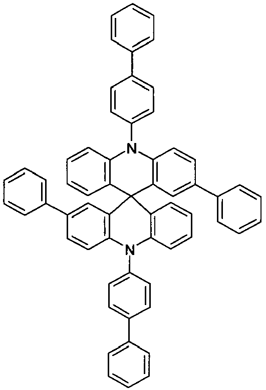 | 65% |
| 4 | 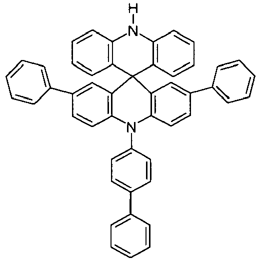 | 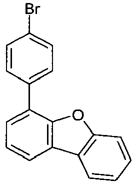 | 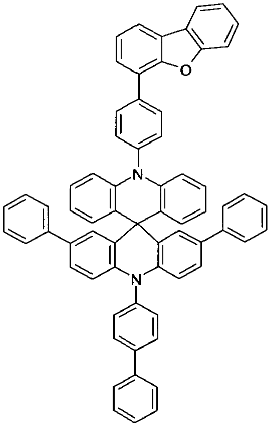 | 69 % |
| 5 | 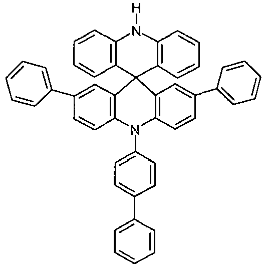 | 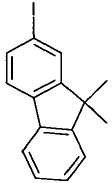 | 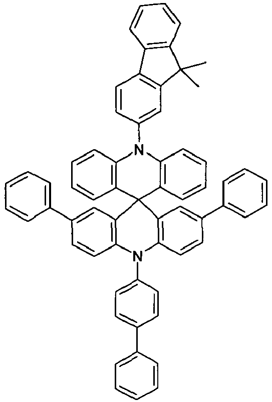 | 71% |
| 6 | 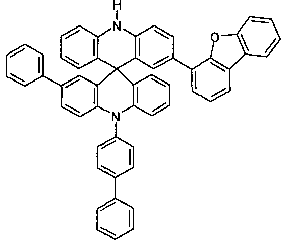 | 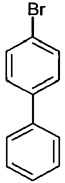 | 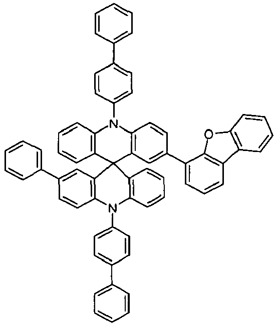 | 75% |
| V4 | 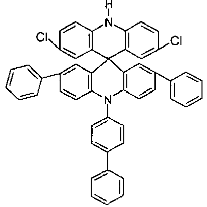 | 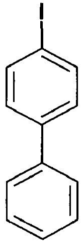 | 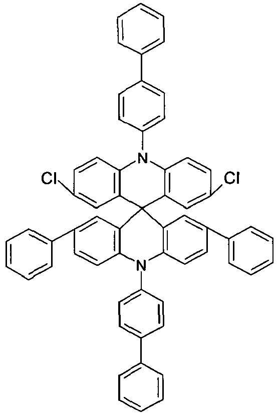 | 81% |
| 7 | 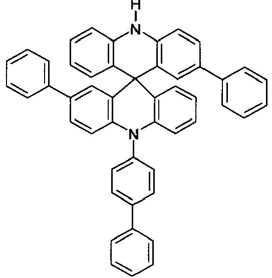 | 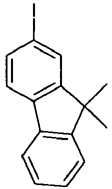 | 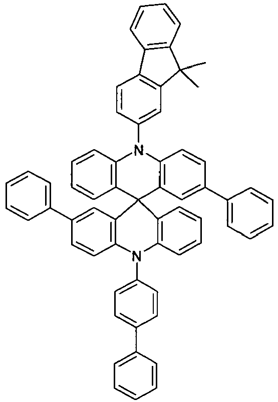 | 79% |
| 8 | 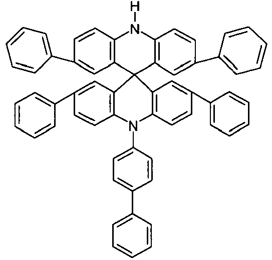 | 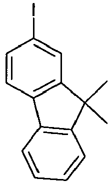 | 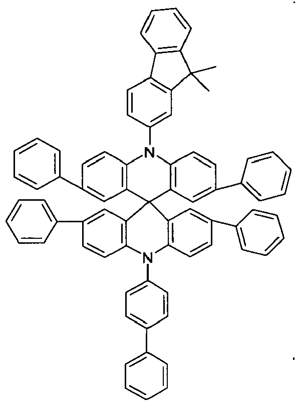 | 70% |
| 9 | 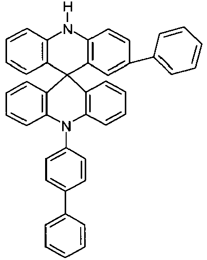 | 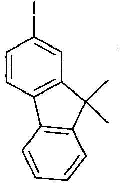 | 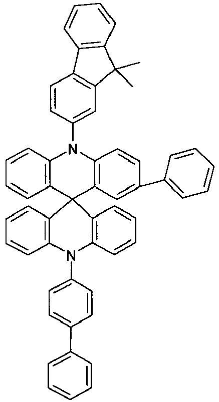 | 60% |
| 10 | 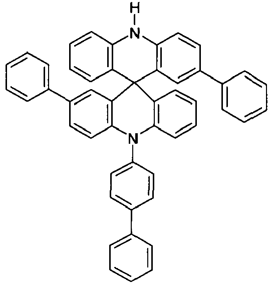 | 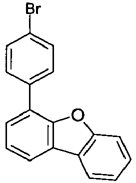 | 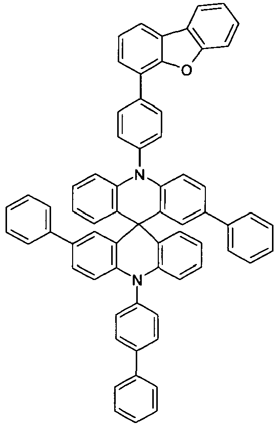 | |

### B) Device-Beispiele

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (z.B. Materialien) angepasst wird.

In den folgenden erfindungsgemäßen Beispielen E1 - E16 und in den Referenzbeispielen V1 - V4 werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben folgenden Schichtaufbau: Substrat / p-dotierte Lochinjektionsschicht (HIL) / Lochtransportschicht (HTL) / evtl. p-dotierte Lochtransportschicht / Elektronenblockierschicht (EBL) / Emissionsschicht (EML) / Elektronentransportschicht (ETL) / Elektroneninjektionsschicht (EIL) und abschließend eine Kathode. Die Kathode wird durch eine 100 nm dicke Aluminiumschicht gebildet. Die zur Herstellung der OLEDs benötigten Materialien sind in Tabelle 1 gezeigt, die verschiedenen Bauteilaufbauten in Tabelle 2.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1 :SEB(5%) bedeutet hierbei, dass das Material H1 in einem Volumenanteil von 95% und SEB in einem Volumenanteil von 5% in der Schicht vorliegt. Analog können auch die Elektronentransportschichten oder die Lochinjektionsschichten aus einer Mischung von zwei oder mehr Materialien bestehen.

Die OLEDs werden standardmäßig charakterisiert. Hierfür werden die Elektrolumineszenzspektren und die Stromeffizienz (in cd/A) gemessen, die Leistungseffizienz und die externe Quanteneffizienz (EQE, in Prozent) in Abhängigkeit der Leuchtdichte aus Strom-Spannungs-Leuchtdichte-Kennlinien (IUL-Kennlinien) unter Annahme einer lambertschen Abstrahlcharakteristik berechnet sowie die Lebensdauer bestimmt. Die Angabe EQE @ 10 mA/cm² bezeichnet die externe Quanteneffizienz bei einer Stromdichte von 10mA/cm². LD80 @ 60 mA/cm² ist die Lebensdauer, bis zu der die OLED bei einer Starthelligkeit bei konstantem Strom von 60 mA/cm² auf 80 % der Anfangsintensität abgefallen ist.

| ***Tabelle 1: Strukturen der verwendeten Materialien*** | | |
|---|---|---|
| 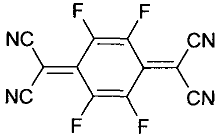 | 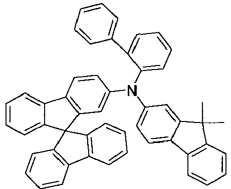 | 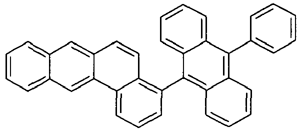 |
| F4TCNQ | HIM | H1 |
| 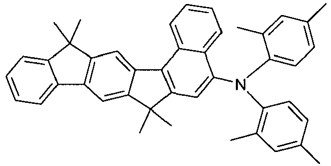 | 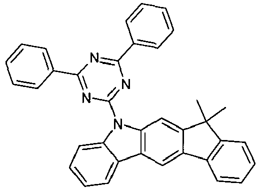 | 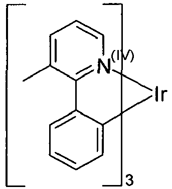 |
| SEB | H2 | TEG |
| 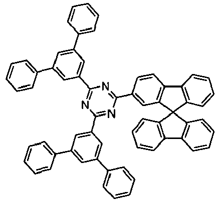 | 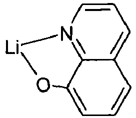 | 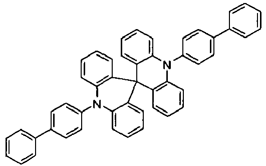 |
| ETM | LiQ | HTMV1 |
| 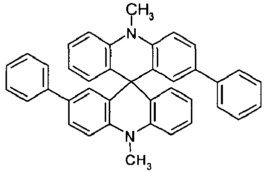 | 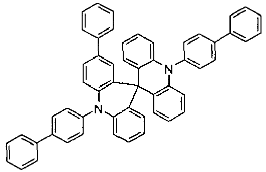 | 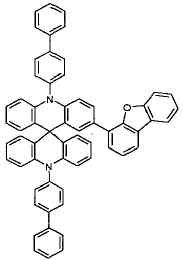 |
| HTMV2 | HTM1 | HTM2 |
| 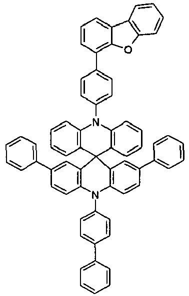 | 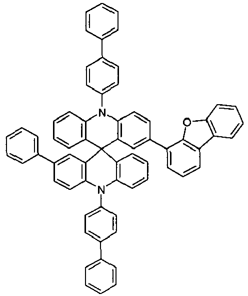 | 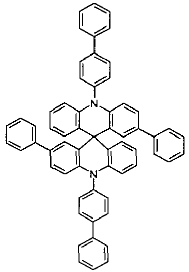 |
| HTM3 | HTM4 | HTM5 |
| 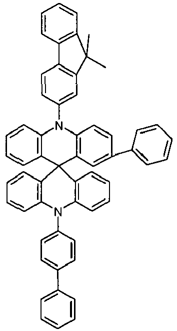 | 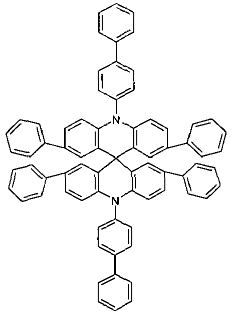 | 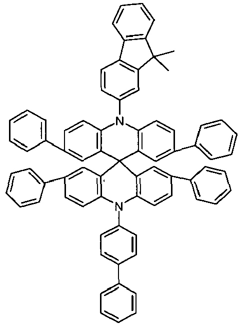 |
| HTM6 | HTM7 | HTM8 |

| ***Tabelle 2: Aufbau der OLEDs*** | | | | | | | |
|---|---|---|---|---|---|---|---|
| ***Exp.*** | ***HIL1*** | ***HTL*** | ***HIL2*** | ***EBL*** | ***EML*** | ***ETL*** | ***EIL*** |
| | *Dicke* / *nm* | *Dicke* / *nm* | *Dicke* / *nm* | *Dicke* / *nm* | *Dicke* / *nm* | *Dicke* / *nm* | *Dicke* / *nm* |
| *V1* | *HIM:F4TCNQ(5%) 20 nm* | *HIM 175 nm* | | *HTMV2 20 nm* | *H1:SEB(5%) 20 nm* | *ETM:LiQ(50%) 30 nm* | *LiQ 1 nm* |
| *E1* | *HIM:F4TCNQ(5%) 20 nm* | *HIM 175 nm* | | *HTM5 20 nm* | *H1:SEB(5%) 20 nm* | *ETM:LiQ(50%) 30 nm* | *LiQ 1 nm* |
| *V2* | *HIM:F4TCNQ(5%) 20 nm* | *HIM 220 nm* | *HTMV2:F4TCNQ(5%) 20 nm* | *HTMV2 20 nm* | *H2:TEG(10%) 30 nm* | *ETM:LiQ(50%) 40 nm* | *LiQ 1 nm* |
| *E2* | *HIM:F4TCNQ(5%) 20 nm* | *HIM 220 nm* | *HTM5:F4TCNQ(5%) 20 nm* | *HTM5 20 nm* | *H2:TEG(10%) 30 nm* | *ETM:LiQ(50%) 40 nm* | *LiQ 1 nm* |
| *V3* | *HIM:F4TCNQ(5%) 20 nm* | *HIM 175 nm* | | *HTMV1 20 nm* | *H1:SEB(5%) 20 nm* | *ETM:LiQ(50%) 30 nm* | *LiQ 1 nm* |
| *E3* | *HIM:F4TCNQ(5%) 20 nm* | *HIM 175 nm* | | *HTM1 20 nm* | *H1:SEB(5%) 20 nm* | *ETM:LiQ(50%) 30 nm* | *LiQ 1 nm* |
| *E4* | *HIM:F4TCNQ(5%) 20 nm* | *HIM 175 nm* | | *HTM2 20 nm* | *H1:SEB(5%) 20 nm* | *ETM:LiQ(50%) 30 nm* | *LiQ 1 nm* |
| *E5* | *HIM:F4TCNQ(5%) 20 nm* | *HIM 175 nm* | | *HTM3 20 nm* | *H1:SEB(5%) 20 nm* | *ETM:LiQ(50%) 30 nm* | *LiQ 1 nm* |
| *E6* | *HIM:F4TCNQ(5%) 20 nm* | *HIM 175 nm* | | *HTM4 20 nm* | *H1:SEB(5%) 20 nm* | *ETM:LiQ(50%) 30 nm* | *LiQ 1 nm* |
| *E7* | *HIM:F4TCNQ(5%) 20 nm* | *HIM 175 nm* | | *HTM6 20 nm* | *H1:SEB(5%) 20 nm* | *ETM:LiQ(50%) 30 nm* | *LiQ 1 nm* |
| *E8* | *HIM:F4TCNQ(5%) 20 nm* | *HIM 175 nm* | | *HTM7 20 nm* | *H1:SEB(5%) 20 nm* | *ETM:LiQ(50%) 30 nm* | *LiQ 1 nm* |
| *E9* | *HIM:F4TCNQ(5%) 20 nm* | *HIM 175 nm* | | *HTM8 20 nm* | *H1:SEB(5%) 20 nm* | *ETM:LiQ(50%) 30 nm* | *LiQ 1 nm* |
| *V4* | *HIM:F4TCNQ(5%) 20 nm* | *HIM 220 nm* | *HTMV1:F4TCNQ(5%) 20 nm* | *HTMV1 20 nm* | *H2:TEG(10%) 30 nm* | *ETM:LiQ(50%) 40 nm* | *LiQ 1 nm* |
| *E10* | *HIM:F4TCNQ(5%) 20 nm* | *HIM 220 nm* | *HTM1:F4TCNQ(5%) 20 nm* | *HTM1 20 nm* | *H2:TEG(10%) 30 nm* | *ETM:LiQ(50%) 40 nm* | *LiQ 1 nm* |
| *E11* | *HIM:F4TCNQ(5%) 20 nm* | *HIM 220 nm* | *HTM2:F4TCNQ(5%) 20 nm* | *HTM2 20 nm* | *H2:TEG(10%) 30 nm* | *ETM:LiQ(50%) 40 nm* | *LiQ 1 nm* |
| *E12* | *HIM:F4TCNQ(5%) 20 nm* | *HIM 220 nm* | *HTM3:F4TCNQ(5%) 20 nm* | *HTM3 20 nm* | *H2:TEG(10%) 30 nm* | *ETM:LiQ(50%) 40 nm* | *LiQ 1 nm* |
| *E13* | *HIM:F4TCNQ(5%) 20 nm* | *HIM 220 nm* | *HTM4:F4TCNQ(5%) 20 nm* | *HTM4 20 nm* | *H2:TEG(10%) 30 nm* | *ETM:LiQ(50%) 40 nm* | *LiQ 1 nm* |
| *E14* | *HIM:F4TCNQ(5%) 20 nm* | *HIM 220 nm* | *HTM6:F4TCNQ(5%) 20 nm* | *HTM6 20 nm* | *H2:TEG(10%) 30 nm* | *ETM:LiQ(50%) 40 nm* | *LiQ 1 nm* |
| *E15* | *HIM:F4TCNQ(5%) 20 nm* | *HIM 220 nm* | *HTM7:F4TCNQ(5%) 20 nm* | *HTM7 20 nm* | *H2:TEG(10%) 30 nm* | *ETM:LiQ(50%) 40 nm* | *LiQ 1 nm* |
| *E16* | *HIM:F4TCNQ(5%) 20 nm* | *HIM 220 nm* | *HTM8:F4TCNQ(5%) 20 nm* | *HTM8 20 nm* | *H2:TEG(10%) 30 nm* | *ETM:LiQ(50%) 40 nm* | *LiQ 1 nm* |

### Beispiel 1

Im Beispiel 1 wird eine erfindungsgemäße Substanz (HTM5) und der Stand der Technik (HTMV2) in einer OLED mit blau fluoreszierender emittierender Schicht verglichen. Die Verbindungen werden jeweils in lochtransportierenden Schichten der OLED eingesetzt.

Die externe Quanteneffizienz bei 10mA/cm² der erfindungsgemäßen Verbindung aus den Proben E1 ist mit 8,5% deutlich besser als die der Referenzprobe V1 mit lediglich 6,0%. Die Lebensdauer LD80 bei 60mA/cm² ist bei der erfindungsgemäßen Probe E1 mit 115h deutlich erhöht gegenüber dem Stand der Technik in V1 mit 38h.

Die beiden Substanzen werden ebenfalls in einem Triplett-Grün-Bauteil verglichen. Die Referenzprobe V2 zeigt mit 16,3% eine deutlich niedrigere Quanteneffizienz bei 2mA/cm² als die erfindungsgemäße Probe E2 mit 20,1%. Die Lebensdauer LD80 der erfindungsgemäßen Probe E2 liegt mit 223h deutlich über der Referenz-Lebensdauer von V2 mit 96h.

### Beispiel 2

Im Beispiel 2 werden weitere vier erfindungsgemäße Substanzen (HTM1, HTM2, HTM3 und HTM4) mit dem Stand der Technik (HTMV1) verglichen. In einem Singulett-Blau-Bauteil erzielt die erfindungsgemäße Probe E3 mit 9,8% bei 10mA/cm² eine höhere Quanteneffizienz als der Stand der Technik (V3) mit 8,5%. Die Lebensdauern (80%) der Bauteile mit erfindungsgemäßen Materialien E3-E6 liegen mit E3 (214h), E4 (180h), E5 (154h) und E6 (166h) deutlich über der Lebensdauer des Vergleichmaterials (V3) mit 66h.

In einem Triplett-Grün-Bauteil zeigt die Referenzprobe V4 (19,8 %) eine niedrigere Quanteneffizienz bei 2 mA/cm² als beispielsweise die erfindungsgemäße Probe E10 (20,4 %). Auch sind die Lebensdauern (80%) bei 20 mA/cm² der erfindungsgemäßen Proben E10 (199h), E11 (223h), E12 (205h) und E13 (214h) höher als beim Stand der Technik V4 mit lediglich 193h.

### Beispiel 3

Im Beispiel 3 werden weitere drei erfindungsgemäße Substanzen (HTM6, HTM7 und HTM8) mit dem Stand der Technik (HTMV1) verglichen.

In einem Singulett-Blau-Bauteil erzielen die erfindungsgemäßen Proben E8 mit 8,6% bei 10mA/cm² und E9 mit 9,1% eine höhere Quanteneffizienz als der Stand der Technik (V3) mit 8,5%. Die Lebensdauer (80%) des Bauteils mit erfindungsgemäßen Material E8 liegen mit 150h deutlich über der Lebensdauer des Vergleichbauteils V3 mit 66h.

In einem Triplett-Grün-Bauteil zeigt die Referenzprobe V4 (19,8 %) eine niedrigere Quanteneffizienz bei 2 mA/cm² als die erfindungsgemäßen Proben E14 (21,5 %), E15 (20,2 %) und E16 (20,6 %).

## Patentansprüche

1. Verbindung der Formel (I) 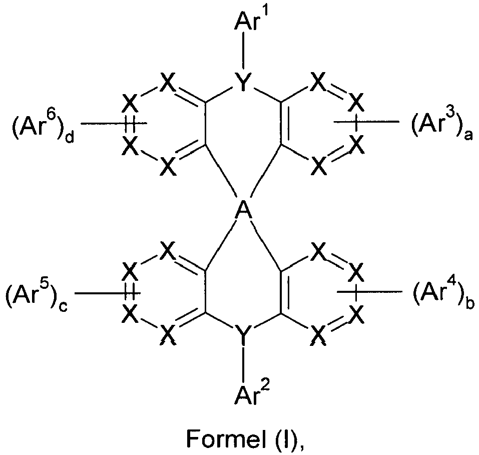 wobei für die auftretenden Symbole und Indices gilt:
A ist C oder Si;
Y ist bei jedem Auftreten gleich oder verschieden N oder P;
X ist gleich CR¹;
Ar¹, Ar² ist bei jedem Auftreten gleich oder verschieden ein aromatisches Ringsystem mit 6 bis 40 aromatischen Ringatomen, das mit einem oder mehreren Resten R² substituiert sein kann, oder ein heteroaromatisches Ringsystem mit 5 bis 40 aromatischen Ringatomen, das mit einem oder mehreren Resten R² substituiert sein kann;
Ar³, Ar⁴, Ar⁵, Ar⁶ ist bei jedem Auftreten gleich oder verschieden ein aromatisches Ringsystem mit 6 bis 40 aromatischen Ringatomen, das mit einem oder mehreren Resten R² substituiert sein kann, oder ein heteroaromatisches Ringsystem mit 5 bis 40 aromatischen Ringatomen, das mit einem oder mehreren Resten R² substituiert sein kann;
R¹, R² ist bei jedem Auftreten gleich oder verschieden gewählt aus H, D, F, C(=O)R³, CF₃, OCF₃, CN, Si(R³)₃, N(R³)₂, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ bzw. R² miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können;
R³ ist bei jedem Auftreten gleich oder verschieden gewählt aus H, D, F, C(=O)R⁴, CF₃, OCF₃, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
R⁴ ist bei jedem Auftreten gleich oder verschieden gewählt aus H, D, F, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁴ miteinander verknüpft sein können und einen Ring bilden können; und wobei die genannten Alkylgruppen, aromatischen Ringsysteme und heteroaromatischen Ringsysteme mit F oder CN substituiert sein können;
a, b, c, d ist bei jedem Auftreten gleich oder verschieden 0 oder 1;
wobei mindestens eine der beiden Gruppen Ar¹ und Ar² ein aromatisches Ringsystem mit 12 bis 40 aromatischen Ringatomen ist, das mit einem oder mehreren Resten R² substituiert sein kann, oder ein heteroaromatisches Ringsystem mit 12 bis 40 aromatischen Ringatomen ist, das mit einem oder mehreren Resten R² substituiert sein kann; und
wobei mindestens einer der Indices a, b, c und d gleich 1 ist.

2. Verbindung nach Anspruch 1, **dadurch gekennzeichnet, dass** sie keine Arylaminogruppe als Substituent umfasst.

3. Verbindung nach Anspruch 1 oder 2, **dadurch gekennzeichnet, dass** in Formel (I) genau 1, 2 oder 3 Indices gewählt aus den Indices a, b, c und d gleich 1 sind.

4. Verbindung nach einem oder mehreren der Ansprüche 1 bis 3, **dadurch gekennzeichnet, dass** A ein Kohlenstoffatom ist.

5. Verbindung nach einem oder mehreren der Ansprüche 1 bis 4, **dadurch gekennzeichnet, dass** Y ein Stickstoffatom ist.

6. Verbindung nach einem oder mehreren der Ansprüche 1 bis 5, **dadurch gekennzeichnet, dass** Ar¹ und Ar² bei jedem Auftreten gleich oder verschieden gewählt sind aus aromatischen Ringsystemen mit 6 bis 24 aromatischen Ringatomen, die mit einem oder mehreren Resten R² substituiert sein können, oder aus heteroaromatischen Ringsystemen mit 5 bis 24 aromatischen Ringatomen, die mit einem oder mehreren Resten R² substituiert sein können, wobei mindestens eine der beiden Gruppen Ar¹ und Ar² ein aromatisches Ringsystem mit 12 bis 24 aromatischen Ringatomen ist, das mit einem oder mehreren Resten R² substituiert sein kann, oder ein heteroaromatisches Ringsystem mit 12 bis 24 aromatischen Ringatomen ist, das mit einem oder mehreren Resten R² substituiert sein kann.

7. Verbindung nach einem oder mehreren der Ansprüche 1 bis 6, **dadurch gekennzeichnet, dass** beide der Gruppen Ar¹ und Ar² bei jedem Auftreten gleich oder verschieden gewählt sind aus aromatischen Ringsystemen mit 12 bis 24 aromatischen Ringatomen, die mit einem oder mehreren Resten R² substituiert sein können, und heteroaromatischen Ringsystemen mit 12 bis 24 aromatischen Ringatomen, die mit einem oder mehreren Resten R² substituiert sein können.

8. Verbindung nach einem oder mehreren der Ansprüche 1 bis 7, **dadurch gekennzeichnet, dass** die auftretenden Gruppen Ar³, Ar⁴, Ar⁵, Ar⁶ in der Verbindung der Formel (I) gleich gewählt sind.

9. Verbindung nach einem oder mehreren der Ansprüche 1 bis 8, **dadurch gekennzeichnet, dass** mindestens eine der Gruppen Ar¹, Ar², Ar³, Ar⁴, Ar⁵ und Ar⁶ mindestens eine Heteroarylgruppe mit 5 bis 20 aromatischen Ringatomen umfasst.

10. Verbindung nach einem oder mehreren der Ansprüche 1 bis 9, **dadurch gekennzeichnet, dass** die Verbindung der Formel (I) einer der Formeln I-1) bis (I-3) entspricht 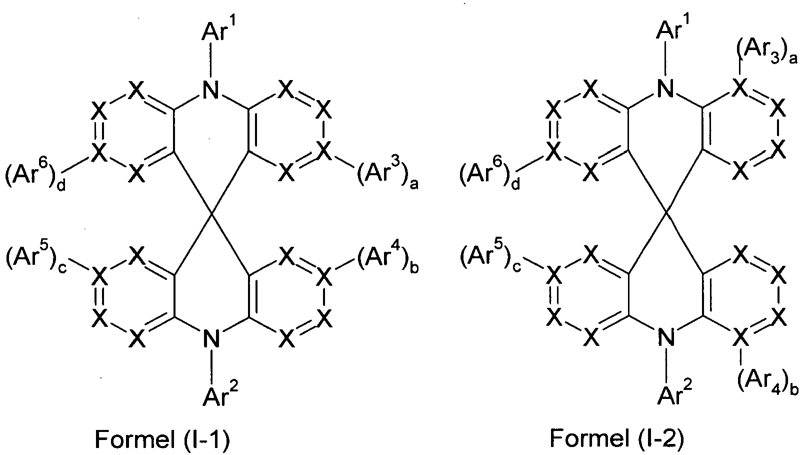 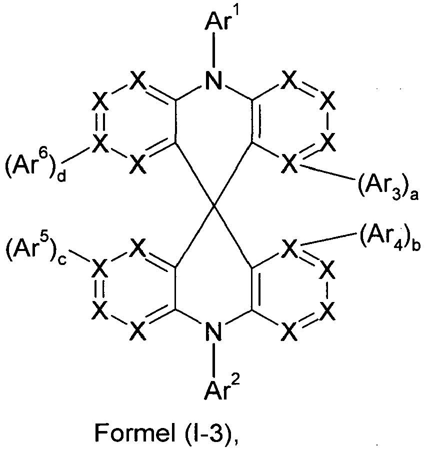 wobei die auftretenden Symbole und Indices definiert sind gemäß einem oder mehreren der Ansprüche 1 bis 9.

11. Verfahren zur Herstellung einer Verbindung nach einem oder mehreren der Ansprüche 1 bis 10, **dadurch gekennzeichnet, dass** es die folgenden Schritte umfasst:
1) Umsetzung eines halogensubstituierten Diphenylamin-Derivats mit einem Acridinon-Derivat in Gegenwart eines Metallorganyls;
2) Ringschlussreaktion der in 1) entstandenen Zwischenstufe zu einem Spiro-Bisacridin-Derivat;
3) Einführung einer Aryl- oder Heteroarylgruppe mittels Buchwald-Kupplung an einem freien Stickstoffatom des Spiro-Bisacridin-Derivats.

12. Oligomer, Polymer oder Dendrimer, enthaltend eine oder mehrere Verbindungen nach einem oder mehreren der Ansprüche 1 bis 10, wobei die Bindung(en) zum Polymer, Oligomer oder Dendrimer an beliebigen, in Formel (I) mit R¹ oder R² substituierten Positionen lokalisiert sein können.

13. Formulierung, enthaltend mindestens eine Verbindung nach einem oder mehreren der Ansprüche 1 bis 10, oder mindestens ein Polymer, Oligomer oder Dendrimer nach Anspruch 12, sowie mindestens ein Lösungsmittel.

14. Verwendung einer Verbindung nach einem oder mehreren der Ansprüche 1 bis 10 oder eines Oligomers, Polymers oder Dendrimers nach Anspruch 12 in einer elektronischen Vorrichtung.

15. Elektronische Vorrichtung, enthaltend mindestens eine Verbindung nach einem oder mehreren der Ansprüche 1 bis 10 oder mindestens ein Oligomer, Polymer oder Dendrimer nach Anspruch 12.

16. Elektronische Vorrichtung nach Anspruch 15, gewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs).

17. Organische Elektrolumineszenzvorrichtung nach Anspruch 16, **dadurch gekennzeichnet, dass** die mindestens eine Verbindung oder das mindestens eine Oligomer, Polymer oder Dendrimer in einer Schicht gewählt aus Lochtransportschichten und emittierenden Schichten vorliegt.

18. Organische Elektrolumineszenzvorrichtung nach Anspruch 17, **dadurch gekennzeichnet, dass** die mindestens eine Verbindung oder das mindestens eine Oligomer, Polymer oder Dendrimer in einer emittierenden Schicht zusammen mit einem oder mehreren phosphoreszierenden Emittern vorliegt.

19. Organische Elektrolumineszenzvorrichtung nach Anspruch 17, **dadurch gekennzeichnet, dass** die mindestens eine Verbindung oder das mindestens eine Oligomer, Polymer oder Dendrimer in einer Lochtransportschicht zusammen mit einem oder mehreren p-Dotanden vorliegt.

## Claims

1. Compound of the formula (I) 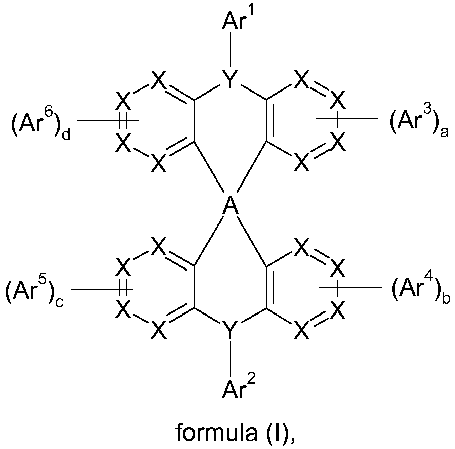 where the following applies to the symbols and indices occurring:
A is C or Si;
Y is on each occurrence, identically or differently, N or P;
X is equal to CR¹;
Ar¹, Ar² are on each occurrence, identically or differently, an aromatic ring system having 6 to 40 aromatic ring atoms, which may be substituted by one or more radicals R², or a heteroaromatic ring system having 5 to 40 aromatic ring atoms, which may be substituted by one or more radicals R²;
Ar³, Ar⁴, Ar⁵, Ar⁶ are on each occurrence, identically or differently, an aromatic ring system having 6 to 40 aromatic ring atoms, which may be substituted by one or more radicals R², or a heteroaromatic ring system having 5 to 40 aromatic ring atoms, which may be substituted by one or more radicals R²;
R¹, R² are selected on each occurrence, identically or differently, from H, D, F, C(=O)R³, CF₃, OCF₃, CN, Si(R³)₃, N(R³)₂, P(=O)(R³)₂, OR³, S(=O)R³, S(=O)₂R³, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where two or more radicals R¹ or R² may be linked to one another and may form a ring; where the said alkyl, alkoxy, alkenyl and alkynyl groups and the said aromatic ring systems and heteroaromatic ring systems may in each case be substituted by one or more radicals R³; and where one or more CH₂ groups in the said alkyl, alkoxy, alkenyl and alkynyl groups may be replaced by -R³C=CR³-, -C≡C-, Si(R³)₂, C=O, C=NR³, -C(=O)O-, -C(=O)NR³-, NR³, P(=O)(R³), -O-, -S-, SO or SO₂;
R³ is selected on each occurrence, identically or differently, from H, D, F, C(=O)R⁴, CF₃, OCF₃, CN, Si(R⁴)₃, N(R⁴)₂, P(=O)(R⁴)₂, OR⁴, S(=O)R⁴, S(=O)₂R⁴, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where two or more radicals R³ may be linked to one another and may form a ring; where the said alkyl, alkoxy, alkenyl and alkynyl groups and the said aromatic ring systems and heteroaromatic ring systems may in each case be substituted by one or more radicals R⁴; and where one or more CH₂ groups in the said alkyl, alkoxy, alkenyl and alkynyl groups may be replaced by -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO or SO₂;
R⁴ is selected on each occurrence, identically or differently, from H, D, F, CN, alkyl groups having 1 to 20 C atoms, aromatic ring systems having 6 to 40 aromatic ring atoms and heteroaromatic ring systems having 5 to 40 aromatic ring atoms; where two or more radicals R⁴ may be linked to one another and may form a ring; and where the said alkyl groups, aromatic ring systems and heteroaromatic ring systems may be substituted by F or CN;
a, b, c, d are on each occurrence, identically or differently, 0 or 1;
where at least one of the two groups Ar¹ and Ar² is an aromatic ring system having 12 to 40 aromatic ring atoms, which may be substituted by one or more radicals R², or a heteroaromatic ring system having 12 to 40 aromatic ring atoms, which may be substituted by one or more radicals R²; and
where at least one of the indices a, b, c and d is equal to 1.

2. Compound according to Claim 1, **characterised in that** it does not contain an arylamino group as substituent.

3. Compound according to Claim 1 or 2, **characterised in that** precisely 1, 2 or 3 indices selected from the indices a, b, c and d in formula (I) are equal to 1.

4. Compound according to one or more of Claims 1 to 3, **characterised in that** A is a carbon atom.

5. Compound according to one or more of Claims 1 to 4, **characterised in that** Y is a nitrogen atom.

6. Compound according to one or more of Claims 1 to 5, **characterised in that** Ar¹ and Ar² are selected on each occurrence, identically or differently, from aromatic ring systems having 6 to 24 aromatic ring atoms, which may be substituted by one or more radicals R², or from heteroaromatic ring systems having 5 to 24 aromatic ring atoms, which may be substituted by one or more radicals R², where at least one of the two groups Ar¹ and Ar² is an aromatic ring system having 12 to 24 aromatic ring atoms, which may be substituted by one or more radicals R², or a heteroaromatic ring system having 12 to 24 aromatic ring atoms, which may be substituted by one or more radicals R².

7. Compound according to one or more of Claims 1 to 6, **characterised in that** both of the groups Ar¹ and Ar² are selected on each occurrence, identically or differently, from aromatic ring systems having 12 to 24 aromatic ring atoms, which may be substituted by one or more radicals R², and heteroaromatic ring systems having 12 to 24 aromatic ring atoms, which may be substituted by one or more radicals R².

8. Compound according to one or more of Claims 1 to 7, **characterised in that** the groups Ar³, Ar⁴, Ar⁵, Ar⁶ occurring in the compound of the formula (I) are selected identically.

9. Compound according to one or more of Claims 1 to 8, **characterised in that** at least one of the groups Ar¹, Ar², Ar³, Ar⁴, Ar⁵ and Ar⁶ contains at least one heteroaryl group having 5 to 20 aromatic ring atoms.

10. Compound according to one or more of Claims 1 to 9, **characterised in that** the compound of the formula (I) conforms to one of the formulae (1-1) to (I-3) 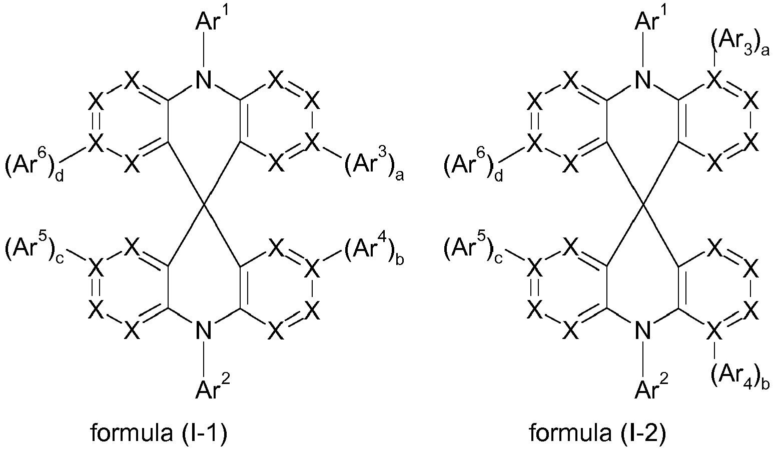 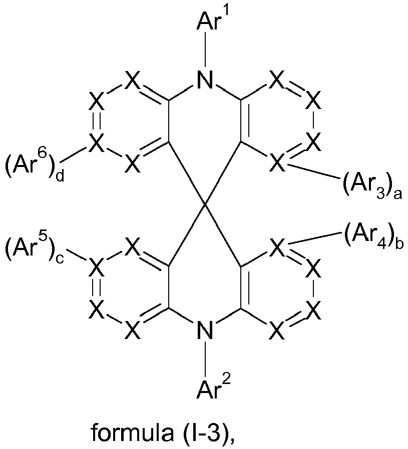 where the symbols and indices occurring are defined in accordance with one or more of Claims 1 to 9.

11. Process for the preparation of a compound according to one or more of Claims 1 to 10, **characterised in that** it comprises the following steps:
1) reaction of a halogen-substituted diphenylamine derivative with an acridinone derivative in the presence of an organometallic compound;
2) ring-closure reaction of the intermediate formed in 1) to give a spirobisacridine derivative;
3) introduction of an aryl or heteroaryl group on a free nitrogen atom of the spirobisacridine derivative by means of Buchwald coupling.

12. Oligomer, polymer or dendrimer containing one or more compounds according to one or more of Claims 1 to 10, where the bond(s) to the polymer, oligomer or dendrimer may be localised at any desired positions in formula (I) that are substituted by R¹ or R².

13. Formulation comprising at least one compound according to one or more of Claims 1 to 10, or at least one polymer, oligomer or dendrimer according to Claim 12, and at least one solvent.

14. Use of a compound according to one or more of Claims 1 to 10 or an oligomer, polymer or dendrimer according to Claim 12 in an electronic device.

15. Electronic device containing at least one compound according to one or more of Claims 1 to 10 or at least one oligomer, polymer or dendrimer according to Claim 12.

16. Electronic device according to Claim 15, selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs).

17. Organic electroluminescent device according to Claim 16, **characterised in that** the at least one compound or the at least one oligomer, polymer or dendrimer is present in a layer selected from hole-transport layers and emitting layers.

18. Organic electroluminescent device according to Claim 17, **characterised in that** the at least one compound or the at least one oligomer, polymer or dendrimer is present in an emitting layer together with one or more phosphorescent emitters.

19. Organic electroluminescent device according to Claim 17, **characterised in that** the at least one compound or the at least one oligomer, polymer or dendrimer is present in a hole-transport layer together with one or more p-dopants.

## Revendications

1. Composé de la formule (I) : 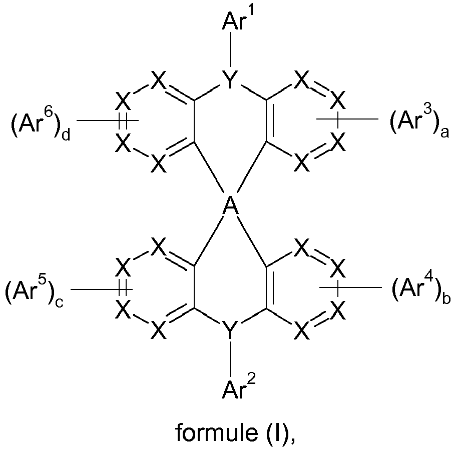 dans laquelle ce qui suit s'applique aux symboles et indices qui sont rencontrés :
A est C ou Si ;
Y est pour chaque occurrence, de manière identique ou différente, N ou P ;
X est égal à CR¹ ;
Ar¹, Ar² sont pour chaque occurrence, de manière identique ou différente, un système de cycle aromatique qui comporte 6 à 40 atomes de cycle aromatique, lequel peut être substitué par un radical ou par plusieurs radicaux R², ou un système de cycle hétéroaromatique qui comporte 5 à 40 atomes de cycle aromatique, lequel peut être substitué par un radical ou par plusieurs radicaux R²;
Ar³, Ar⁴, Ar⁵, Ar⁶ sont pour chaque occurrence, de manière identique ou différente, un système de cycle aromatique qui comporte 6 à 40 atomes de cycle aromatique, lequel R², ou un système de cycle hétéroaromatique qui comporte 5 à 40 atomes de cycle aromatique, lequel peut être substitué par un radical ou par plusieurs radicaux R² ;
R¹, R² sont sélectionnés pour chaque occurrence, de manière identique ou différente, parmi H, D, F, C(=O)R³, CF₃, OCF₃, CN, Si(R³)₃, N(R³)₂, P(=O)(R³)², OR³, S(=O)R³, S(=O)₂R³, des groupes alkyle ou alcoxy en chaîne droite qui comportent 1 à 20 atome(s) de C, des groupes alkyle ou alcoxy ramifiés ou cycliques qui comportent 3 à 20 atomes de C, des groupes alkényle ou alkynyle qui comportent 2 à 20 atomes de C, des systèmes de cycle aromatique qui comportent 6 à 40 atomes de cycle aromatique et des systèmes de cycle hétéroaromatique qui comportent 5 à 40 atomes de cycle aromatique ; où deux radicaux R¹ ou R² ou plus peuvent être liés l'un à l'autre ou les uns aux autres et peuvent former un cycle ; où lesdits groupes alkyle, alcoxy, alkényle et alkynyle et lesdits systèmes de cycle aromatique et hétéroaromatique peuvent dans chaque cas être substitués par un radical ou par plusieurs radicaux R³; et où un ou plusieurs groupe(s) CH₂ dans lesdits groupes alkyle, alcoxy, alkényle et alkynyle peut/ peuvent être remplacé(s) par -R³C=CR³-, -C=C-, Si(R³)₂, C=O, C=NR³, -C(=O)O-, -C(=O)NR³-, NR³, P(=O)(R³), -O-, -S-, SO ou SO₂ ;
R³ est sélectionné pour chaque occurrence, de manière identique ou différente, parmi H, D, F, C(=O)R⁴, CF₃, OCF₃, CN, Si(R⁴)₃, N(R⁴)₂, P(=O)(R⁴)₂, OR⁴, S(=O)R⁴, S(=O)₂R⁴, des groupes alkyle ou alcoxy en chaîne droite qui comportent 1 à 20 atome(s) de C, des groupes alkyle ou alcoxy ramifiés ou cycliques qui comportent 3 à 20 atomes de C, des groupes alkényle ou alkynyle qui comportent 2 à 20 atomes de C, des systèmes de cycle aromatique qui comportent 6 à 40 atomes de cycle aromatique et des systèmes de cycle hétéroaromatique qui comportent 5 à 40 atomes de cycle aromatique ; où deux radicaux R³ ou plus peuvent être liés l'un à l'autre ou les uns aux autres et peuvent former un cycle ; où lesdits groupes alkyle, alcoxy, alkényle et alkynyle et lesdits systèmes de cycle aromatique et hétéroaromatique peuvent dans chaque cas être substitués par un radical ou par plusieurs radicaux R⁴; et où un ou plusieurs groupe(s) CH₂ dans lesdits groupes alkyle, alcoxy, alkényle et alkynyle peut/ peuvent être remplacé(s) par -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO ou SO₂ ;
R⁴ est sélectionné pour chaque occurrence, de manière identique ou différente, parmi H, D, F, CN, des groupes alkyle qui comportent 1 à 20 atome(s) de C, des systèmes de cycle aromatique qui comportent 6 à 40 atomes de cycle [EDIT 1] tique et des systèmes de cycle hétéroaromatique qui comportent 5 à 40 atomes de cycle aromatique ; où deux radicaux R⁴ ou plus peuvent être liés l'un à l'autre ou les uns aux autres et peuvent former un cycle ; et où lesdits groupes alkyle, lesdits systèmes de cycle aromatique et lesdits systèmes de cycle hétéroaromatique peuvent être substitués par F ou par CN;
a, b, c, d sont pour chaque occurrence, de manière identique ou différente, 0 ou 1 ;
où au moins l'un des deux groupes Ar¹ et Ar² est un système de cycle aromatique qui comporte 12 à 40 atomes de cycle aromatique, lequel système de cycle peut être substitué par un radical ou par plusieurs radicaux R², ou un système de cycle hétéroaromatique qui comporte 12 à 40 atomes de cycle aromatique, lequel peut être substitué par un radical ou par plusieurs radicaux R²; et
où au moins l'un des indices a, b, c et d est égal à 1.

2. Composé selon la revendication 1, **caractérisé en ce qu'**il ne contient pas de groupe arylamino en tant que substituant.

3. Composé selon la revendication 1 ou 2, **caractérisé en ce que** précisément 1, 2 ou 3 indice(s) sélectionné(s) parmi les indices a, b, c et d dans la formule (I) est égal/sont égaux à 1.

4. Composé selon une ou plusieurs des revendications 1 à 3, **caractérisé en ce que** A est un atome de carbone.

5. Composé selon une ou plusieurs des revendications 1 à 4, **caractérisé en ce que** Y est un atome d'azote.

6. Composé selon une ou plusieurs des revendications 1 à 5, **caractérisé en ce que** Ar¹ et Ar² sont sélectionnés pour chaque occurrence, de manière identique ou différente, parmi des systèmes de cycle aromatique qui comportent 6 à 24 atomes de cycle aromatique, lesquels systèmes de cycle peuvent être substitués par un radical ou par plusieurs radicaux R², ou parmi des systèmes de cycle hétéroaromatique qui comportent 5 à 24 atomes de cycle aromatique, lesquels peuvent être substitués par un radical ou par plusieurs radicaux R², où au moins l'un des deux groupes Ar¹ et Ar² est un système de cycle aromatique qui comporte 12 à 24 atomes de cycle aromatique, lequel peut être substitué par un radical ou par plusieurs radicaux R², ou un système de cycle hétéroaromatique qui comporte 12 à 24 atomes de cycle aromatique, lequel peut être substitué par un radical ou par plusieurs radicaux R².

7. Composé selon une ou plusieurs des revendications 1 à 6, **caractérisé en ce que** les deux groupes Ar¹ et Ar² sont sélectionnés pour chaque occurrence, de manière identique ou différente, parmi des systèmes de cycle aromatique qui comportent 12 à 24 atomes de cycle aromatique, lesquels peuvent être substitués par un radical ou par plusieurs radicaux R², et des systèmes de cycle hétéroaromatique qui comportent 12 à 24 atomes de cycle aromatique, lesquels peuvent être substitués par un radical ou par plusieurs radicaux R².

8. Composé selon une ou plusieurs des revendications 1 à 7, **caractérisé en ce que** les groupes Ar³, Ar⁴, Ar⁵, Ar⁶ rencontrés dans le composé de la formule (I) sont sélectionnés de manière identique.

9. Composé selon une ou plusieurs des revendications 1 à 8, **caractérisé en ce qu'**au moins l'un des groupes Ar¹, Ar², Ar³, Ar⁴, Ar⁵ et Ar⁶ contient au moins un groupe hétéroaryle qui comporte 5 à 20 atomes de cycle aromatique.

10. Composé selon une ou plusieurs des revendications 1 à 9, **caractérisé en ce que** le composé de la formule (I) est conforme à l'une des formules (I-1) à (I-3) : 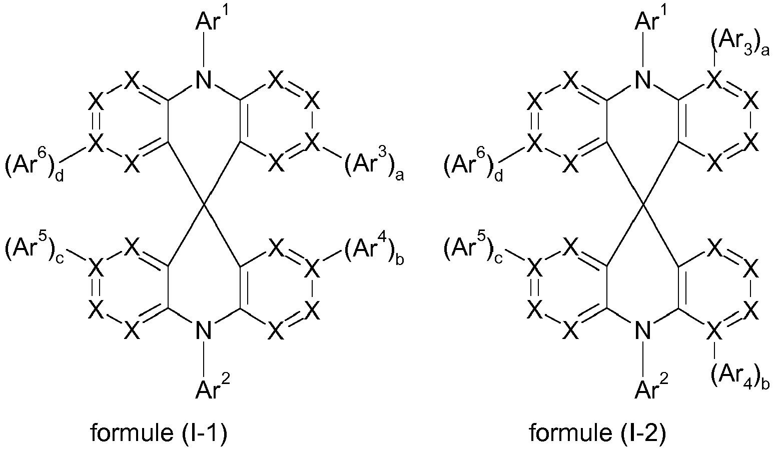 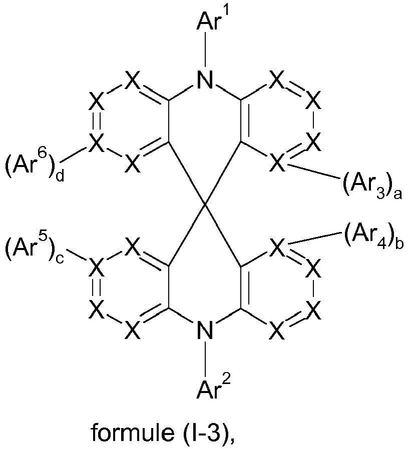 dans lesquelles les symboles et indices rencontrés sont définis conformément à une ou plusieurs des revendications 1 à 9.

11. Procédé pour la préparation d'un composé selon une ou plusieurs des revendications 1 à 10, **caractérisé en ce qu'**il comprend les étapes qui suivent :
1) la réaction d'un dérivé de diphénylamine substitué par halogène avec un dérivé d'acridinone en présence d'un composé organométallique ;
2) la réaction de fermeture de cycle/cyclisation du produit intermédiaire qui est formé en 1) de manière à obtenir un dérivé de spirobisacridine ; et
3) l'introduction d'un groupe aryle ou hétéroaryle sur un atome d'azote libre du dérivé de spirobisacridine au moyen d'un couplage de Buchwald.

12. Oligomère, polymère ou dendrimère contenant un ou plusieurs composé(s) selon une ou plusieurs des revendications 1 à 10, où la/les the liaison(s) sur le polymère, l'oligomère ou le dendrimère peut/ peuvent être localisée(s) au niveau de n'importe quelles positions souhaitées dans la formule (I) qui sont substituées par R¹ ou par R².

13. Formulation comprenant au moins un composé selon une ou plusieurs des revendications 1 à 10, ou au moins un polymère, un oligomère ou un dendrimère selon la revendication 12, et au moins un solvant.

14. Utilisation d'un composé selon une ou plusieurs des revendications 1 à 10 ou d'un oligomère, d'un polymère ou d'un dendrimère selon la revendication 12 dans un dispositif électronique.

15. Dispositif électronique contenant au moins un composé selon une ou plusieurs des revendications 1 à 10 ou au moins un oligomère, un polymère ou un dendrimère selon la revendication 12.

16. Dispositif électronique selon la revendication 15, sélectionné parmi le groupe qui est constitué par les circuits intégrés organiques (OIC), les transistors à effet de champ organiques (OFET), les transistors à film mince organiques (OTFT), les transistors à émission de lumière organiques (OLET), les cellules solaires organiques (OSC), les détecteurs optiques organiques, les photorécepteurs organiques , les dispositifs à extinction de champ organiques (OFQD), les cellules électrochimiques à émission de lumière organiques (OLEC), les diodes laser organiques (O-laser) et les dispositifs électroluminescents organiques (OLED).

17. Dispositif électroluminescent organique selon la revendication 16, **caractérisé en ce que** l'au moins un composé ou l'au moins un oligomère, polymère ou dendrimère est présent dans une couche qui est sélectionnée parmi les couches de transport de trous et les couches d'émission.

18. Dispositif électroluminescent organique selon la revendication 17, **caractérisé en ce que** l'au moins un composé ou l'au moins un oligomère, polymère ou dendrimère est présent dans une couche d'émission avec un ou plusieurs émetteur(s) phosphorescent(s).

19. Dispositif électroluminescent organique selon la revendication 17, **caractérisé en ce que** l'au moins un composé ou l'au moins un oligomère, polymère ou dendrimère est présent dans une couche de transport de trous en association avec un ou plusieurs dopant(s) de type p.
